# EUROPEAN PATENT SPECIFICATION

(11) **EP 1 642 580 B1**
(45) Date of publication and mention of the grant of the patent: **19.08.2009**
(21) Application number: 05021837.9
(22) Date of filing: 08.06.2000
(51) Int. Cl.: A61K 31/4745, A61K 31/437, C07D 471/02, C07D 471/04, A61K 31/47

(54) **Sulfonamide substituted imidazoquinolines**
Imidazoquinoline Sulfonamide
Sulfonamides d' imidazoquinoline

(30) Priority: 10.06.1999 US 138365 P; 07.06.2000 US 589216
(43) Date of publication of application: 05.04.2006
(62) Divisional of application: 00938211.0
(73) Proprietor: Coley Pharmaceutical Group, Inc., Wellesley, MA 02481 (US)
(72) Inventor: Crooks, Stephen L., Saint Paul, MN 55133-3427 (US); Lindstrom, Kyle J., Saint Paul, MN 55133-3427 (US); Merrill, Bryon A., Saint Paul, MN 55133-3427 (US); Rice, Michael J., Saint Paul, MN 55133-3427 (US)
(74) Representative: Vossius & Partner

(56) References cited:
- WO-A-99/29693
- PATENT ABSTRACTS OF JAPAN vol. 1999, no. 08, 30 June 1999 (1999-06-30) -& JP 11 080156 A (HOKURIKU SEIYAKU CO LTD), 26 March 1999 (1999-03-26)
- VOSSIUS & PARTNER PATENTANWÄLTE: "Comparative Tests" INTERNET ARTICLE, [Online] 8 April 2005 (2005-04-08), pages 1-7, XP002366599 Retrieved from the Internet: URL:http://ofi.epoline.org/view/GetDossier > [retrieved on 2006-02-08]

## Description

This invention relates to imidazoquinoline compounds that have sulfonamide or sulfamide substitution at the 1-position and to pharmaceutical compositions containing the compounds. A further aspect of this invention relates to the use of these compounds as immunomodulators, for inducing cytokine biosynthesis in animals and in the treatment of diseases including viral and neoplastic diseases.

The first reliable report on the 1*H-*imidazo[4,5-*c*]quinoline ring system, Backman et al., J. Org. Chem. 15, 1278-1284 (1950) describes the synthesis of 1-(6-methoxy-8-quinolinyl)-2-rnethyl-1*H*-imidazo[4,5-*c*]quinoline for possible use as an antimalarial agent. Subsequently, syntheses of various substituted 1*H*-imidazo[4,5-*c*] quinolines were reported. For example, Jain et al., J. Med. Chem. 11, pp. 87-92 (1968), synthesized the compound 1-[2-(4-pipeidyl)ethyl]-1*H*-imidazo[4,5-*c*]quinoline as a possible anticonvulsant and cardiovascular agent. Also, Baranov et al., Chem. Abs. 85, 94362 (1976), have reported several 2-oxoimidazo[4,5-*c*]quinolines, and Berenyi et al., J. Heterocyclic Chem. 18, 1537-1540 (1981), have reported certain 2-oxoimidazo[4,5-c]quinolines.

Certain 1*H*-imidazo[4,5-*c*]quinolin-4-amines and 1- and 2-substituted derivatives thereof were later found to be useful as antiviral agents, bronchodilators and immunomodulators. These are described in, *inter alia,* U.S. Patent Nos. 4,689,338; 4,698,348; 4,929,624; 5,037,986; 5,268,376; 5,346,905; and 5,389,640, all of which are incorporated herein by reference.

There continues to be interest in the imidazoquinoline ring system, as seen for example in WO 98/30562, EP 894 797 and WO 00/09506. EP 894 797 discloses amide substituted imidazoquinoline compounds that are disclosed to be useful as immune response modifying compounds, while WO 00/09506 discloses imidazoquinoline compounds that contain a sulfonamide substituent wherein the sulfonamide nitrogen is part of a saturated heterocyclic ring.

EP-A-0 894 797 relates to special imidazaquinoline amide derivatives and medicinal preparations containing the same which are said to have an eosinophilic infiltration inhibitory effect based on a potent interferon (a, y)-inducing activity and an exellent percutaneous absorbability and being efficacious in treating allergic inflammatory diseases such as atopic dermatitis, various tumors and viral diseases.

JP-A-11 080156 discloses 1-(substituted aryl) alkyl-1H-imidazopyridin-4-amine derivatives which are said to be useful for inducing the biosynthesis of interferon and as an antiviral agent and carcinostatic agent as well as for the treatment of diseases caused by viruses such as rheumatic arthritis, verruca, hepatitis B and hepatitis C, cancer and other neoplastic diseases.

Despite these efforts, however, there is a continuing need for compounds that have the ability to modulate the immune response, by induction of cytokine biosynthesis or other mechanisms.

We have found a new class of compounds that are useful in inducing cytokine biosynthesis in animals. Accordingly, this invention provides compounds of Formula I: 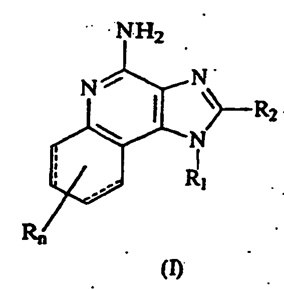 wherein R, R₁ and R₂ are as defined herein.

The compounds of Formula I are useful as immune response modifiers due to their ability to induce cytokine biosynthesis and otherwise modulate the immune reponse when .. administered to animals. This makes the compounds useful in the treatment of a variety of conditions such as viral diseases and tumors that are responsive to such changes in the immune response.

The invention further provides pharmaceutial compositions containing a therapeutically effective amount of a compound of Formula I. Furthermore, the present specification describes methods of inducing cytokine biosynthesis in an animal, treating a viral infection and/or treating a neoplastic disease in an animal by administering a effective amount of a compound of Formula I to the animal.

In addition, methods of synthesizing compounds of Formula I and intermediates useful in the synthesis of these compounds are described.

As mentioned earlier, the invention provides compounds of Formula I: 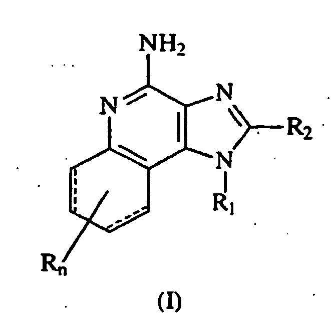 wherein
**R₁** is -alkyl-NR₃- SO₂-X-R₄ or -alkenyl-NR₃- SO₂ -X-R₄;
**X** is a bond or -NR₅-;
**R₄** is aryl, heteroaryl, heterocyclyl, alkyl or alkenyl, each of which may be unsubstituted or substituted by one or more substituents selected from the group consisting of:
-alkyl;
-alkenyl;
-aryl;
-heteroaryl;
-heterocyclyl;
-substituted aryl;
-substituted heteroaryl;
-substituted heterocyclyl;
-O-alkyl;
-O-(alkyl)₀₋₁-aryl;
-O-(alkyl)₀₋₁-substituted aryl;
-O-(alkyl)₀₋₁-heteroaryl;
-O-(alkyl)₀₋₁-substituted heteroaryl;
-O-(alkyl)₀₋₁-heterocyclyl;
-O-(alkyl)₀₋₁-substituted heterocyclyl;
-COOH;
-CO-O-alkyl;
-CO-alkyl;
-S(O)₀₋₂-alkyl;
-S(O)₀₋₂ -(alkyl)₀₋₁-aryl;
-S(O)₀₋₂-(alkyl)₀₋₁-substituted aryl;
-S(O)₀₋₂-(alkyl)₀₋₁-heteroaryl;
-S(O)₀₋₂-(alkyl)₀₋₁-substituted heteroaryl;
-S(O)₀₋₂-(alkyl)₀₋₁-heterocyclyl;
-S(O)₀₋₂-(alkyl)₀₋₁-substituted heterocyclyl;
-(alkyl)₀₋₁-NR₃R₃;
-(alkyl)₀₋₁-NR₃-CO-O-alkyl;
-(alkyl)₀₋₁-NR₃-CO-alkyl;
-(alkyl)₀₋₁-NR₃-CO-aryl;
-(alkyl)₀₋₁-NR₃-CO-substituted aryl;
-(alkyl)₀₋₁-NR₃-CO-heteroaryl;
-(alkyl)₀₋₁-NR₃-CO-substituted heteroaryl;
-N₃;
-halogen;
-haloalkyl;
-haloalkoxy;
-CO-haloalkoxy;
-NO₂;
-CN;
-OH;
-SH; and in the case of alkyl, alkenyl, or heterocyclyl, oxo;
**R₂** is selected from the group consisting of:
-hydrogen;
-alkyl;
-alkenyl;
-aryl;
-substituted aryl;
-heteroaryl;
-substituted heteroaryl;
-alkyl-O-alkyl;
-alkyl-O-alkenyl; and
-alkyl or alkenyl substituted by one or more substituents selected from the group consisting of:
   -OH;
   -halogen;
   -N(R)₂;
   -CO-N(R₃)₂;
   -CO-C₁₋₁₀alkyl;
   -CO-O-C₁₋₁₀alkyl;
   -N₃;
   -aryl;
   -substituted aryl;
   -heteroaryl;
   -substituted heteroaryl;
   -heterocyclyl;
   -substituted heterocyclyl;
   -CO-aryl;
   -CO-(substituted aryl);
   -CO-heteroaryl; and
   -CO-(substituted heteroaryl);
   each **R₃** is independently selected from the group consisting of hydrogen and C₁₋₁₀ alkyl;
   **R₅** is selected from the group consisting of hydrogen and C₁₋₁₀ alkyl, or **R₄** and **R₅** can combine to form a 3 to 7 membered heterocyclic or substituted heterocyclic ring;
   **n** is 0 to 4 and each **R** present is independently selected from the group consisting of C₁₋₁₀ alkyl, C₁₋₁₀ alkoxy, halogen and trifluoromethyl,
   or a pharmaceutically acceptable salt thereof.

### Preparation of the Compounds

Imidazoquinolines of the invention can be prepared according to Reaction Scheme I where R, R₁, R₂ and n are as defined above.

In step (1) of Reaction Scheme I a 4-chloro-3-nitroquinoline of Formula II is reacted with an amine of Formula R₁NH₂ where R₁ is as defined above to provide a 3-nitroquinolin-4-amine of Formula III. The reaction can be carried out by adding amine to a solution of a compound of Formula II in a suitable solvent such as chloroform or dichloromethane and optionally heating. Many quinolines of Formula II are known compounds (see for example, U.S. Patent 4,689,338 and references cited therein).

In step (2) of Reaction Scheme I a 3-nitroquinolin-4-amine of Formula III is reduced to provide a quinoline-3,4-diamine of Formula IV. Preferably, the reduction is carried out using a conventional heterogeneous hydrogenation catalyst such as platinum on carbon or palladium on carbon. The reaction can conveniently be carried out on a Parr apparatus in a suitable solvent such as isopropyl alcohol or toluene.

In step (3) of Reaction Scheme I a quinoline-3,4-diamine of Formula IV is reacted with a carboxylic acid or an equivalent thereof to provide a 1*H*-imidazo[4,5-*c*]quinoline of Formula V. Suitable equivalents to carboxylic acid include acid halides, orthoesters, and 1,1-dialkoxyalkyl alkanoates. The carboxylic acid or equivalent is selected such that it will provide the desired R₂ substituent in a compound of Formula V. For example, triethyl orthoformate will provide a compound where R₂ is hydrogen and triethyl orthoacetate will provide a compound where R₂ is methyl. The reaction can be run in the absence of solvent or in an inert solvent such as toluene. The reaction is run with sufficient heating to drive off any alcohol or water formed as a byproduct of the reaction.

In step (4) of Reaction Scheme I a 1*H*-imidazo[4,5-*c*]quinoline of Formula V is oxidized to provide a 1*H*-imidazo[4,5-*c*]quinoline-5N-oxide of Formula VI using a conventional oxidizing agent that is capable of forming N-oxides. Preferred reaction conditions involve reacting a solution of a compound of Formula V in chloroform with 3-chloroperoxybenzoic acid at ambient conditions.

In step (5) of Reaction Scheme I a 1*H*-imidazo[4,5-*c*]quinoline-5N-oxide of Formula VI is aminated to provide a 1*H*-imidazo[4,5-*c*]quinolin-4-amine of Formula VII which is a subgenus of Formula I. Step (5) involves (i) reacting a compound of Formula VI with an acylating agent and then (ii) reacting the product with an aminating agent. Part (i) of step (5) involves reacting an N-oxide of Formula VI with an acylating agent. Suitable acylating agents include alkyl- or arylsulfonyl chlorides (e.g., benezenesulfonyl chloride, methanesulfonyl chloride, p-toluenesulfonyl chloride). Arylsulfonyl chlorides are preferred. *Para*-toluenesulfonyl chloride is most preferred. Part (ii) of step (5) involves reacting the product of part (i) with an excess of an aminating agent. Suitable aminating agents include ammonia (e.g., in the form of ammonium hydroxide) and ammonium salts (e.g., ammonium carbonate, ammonium bicarbonate, ammonium phosphate). Ammonium hydroxide is preferred. The reaction is preferably carried out by dissolving the N-oxide of Formula VI in an inert solvent such as dichloromethane, adding the aminating agent to the solution, and then slowly adding the acylating agent. The product or a pharmaceutically acceptable salt thereof can be isolated using conventional methods.

Alternatively, step (5) may be carried out by (i) reacting an N-oxide of Formula VI with an isocyanate and then (ii) hydrolyzing the resulting product. Part (i) involves reacting the N-oxide with an isocyanate wherein the isocyanato group is bonded to a carbonyl group. Preferred isocyanates include trichloroacetyl isocyanate and aroyl isocyanates such as benzoyl isocyanate. The reaction of the isocyanate with the N-oxide is carried out under substantially anhydrous conditions by adding the isocyanate to a solution of the N-oxide in an inert solvent such as chloroform or dichloromethane. Part (ii) involves hydrolysis of the product from part (i). The hydrolysis can be carried out by conventional methods such as heating in the presence of water or a lower alkanol optionally in the presence of a catalyst such as an alkali metal hydroxide or lower alkoxide.

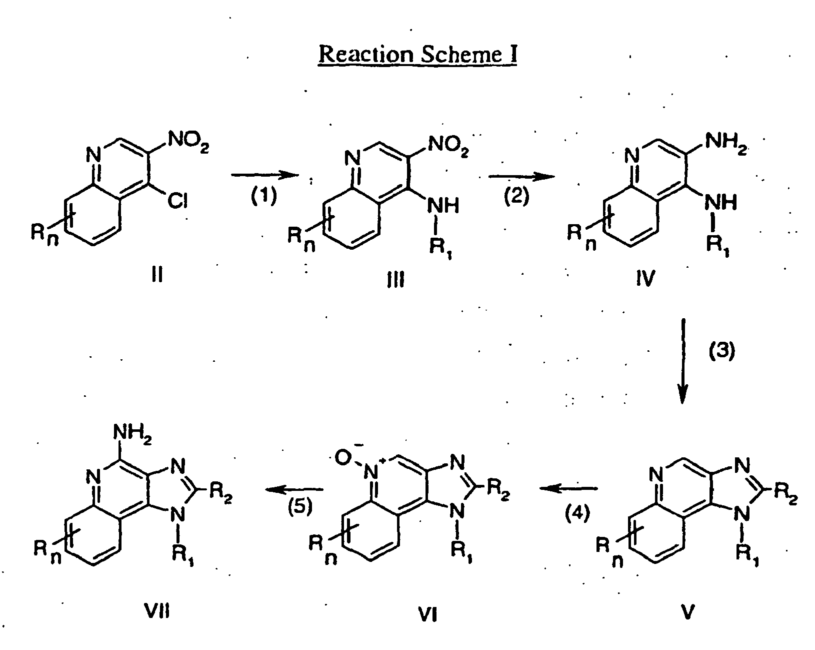

Compounds of the invention where the R₁ substituent contains a sulfonamide can also be prepared according to Reaction Scheme II where R, R₂, R₄ and n are as defined above and m is 1-20.

In Reaction Scheme II an aminoalkyl substituted 1*H*-imidazo[4,5-*c*]quinolin-4-amine of Formula VIII is reacted with a sulfonyl chloride of Formula IX to provide a compound of Formula X which is a subgenus of Formula I. The reaction can be run at ambient temperature in an inert solvent such as dichloromethane in the presence of a base such as pyridine or N,N-diisopropylethylamine. Many 1*H*-imidazo[4,5-*c*]quinolin-4-amines of Formula VIII are known compounds, see for example US Patent 6,069,149 (Namba); others can be readily prepared using known synthetic methods. Many sulfonyl chlorides of Formula IX are commercially available; others can be readily prepared using known synthetic methods. The product or a pharmaceutically acceptable salt thereof can be isolated using conventional methods.

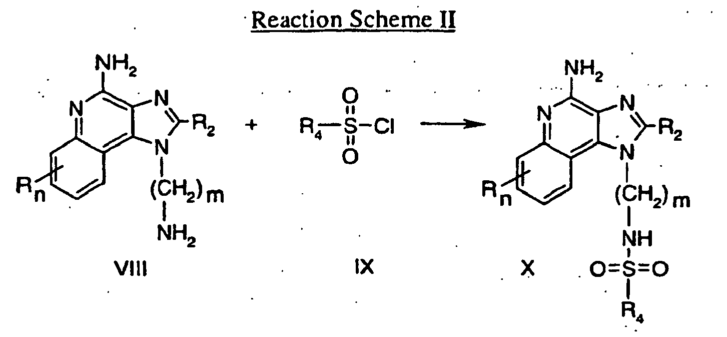

Compounds of the invention where the R₁ substituent contains a sulfonamide can also be prepared according to Reaction Scheme III where R, R₂, R₄ and n are as defined above and m is 1-20.

In Reaction Scheme III an aminoalkyl substituted 1*H*-imidazo[4,5-*c*]quinolin-4-amine of Formula VIII is reacted with a sulfonic anhydride of Formula XI to provide a compound of Formula X which is a subgenus of Formula I. The reaction can be run at ambient temperature in an inert solvent such as dichloromethane, in the presence of a base such as pyridine or N,N-diisopropylethylamine. Alternatively, the reaction can be run at ambient temperature in acetonitrile. Many sulfonic anhydrides of Formula XI are commercially available; others can be readily prepared using known synthetic methods. The product or a pharmaceutically acceptable salt thereof can be isolated using conventional methods.

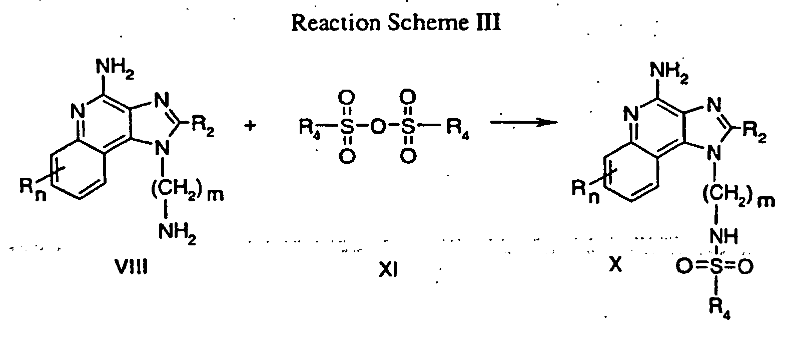

Tertiary sulfonamides of the invention can be prepared according to Reaction Scheme IV where R, R₂, R₃, R₄ and n are as defined above and m is 1-20.

In Reaction Scheme IV a 1*H*-imidazo[4,5-*c*]quinolinyl sulfonamide of Formula X is reacted with a halide of Formula XII to provide a compound of Formula XIII which is a subgenus of Formula I. The reaction can be carried out at ambient temperature by adding sodium hydride to a solution of a compound of Formula X in N,N-dimethylformamide and then adding the halide. Many halides of Formula XII are commercially available; others can be readily prepared using known synthetic methods. The product or a pharmaceutically acceptable salt thereof can be isolated using conventional methods.

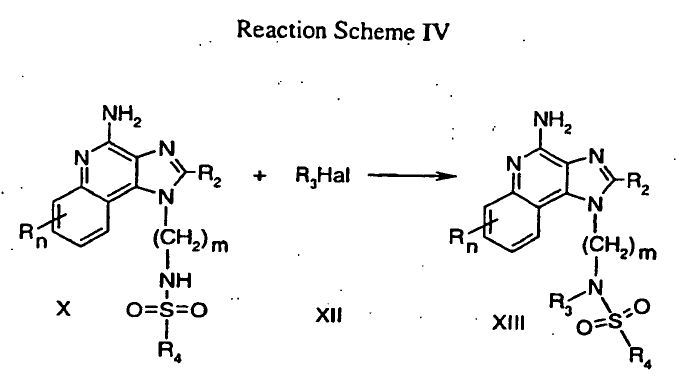

Compounds of the invention where R₁ contains a sulfamide group can be prepared according to Reaction Scheme V wherein R, R₂, R₄, R₅ and n are as defined above and m is 1-20.

In step (1) of Reaction Scheme V an aminoalkyl substituted 1*H*-imidazo[4,5-*c*]quinolin-4-amine of Formula VIII is reacted with sulfuryl chloride to generate in situ a sulfamoyl chloride of Formula XIV. The reaction can be carried out by adding a solution of sulfuryl chloride in dichloromethane to a solution of a compound of Formula VIII in dichloromethane in the presence of one equivalent of 4-(dimethylamino)pyridine. The reaction is preferably carried out at a reduced temperature (-78°C). Optionally, after the addition is complete the reaction mixture can be allowed to warm to ambient temperature.

In step (2) of Reaction Scheme V an amine of Formula R₅R₄NH is reacted with the sulfamoyl chloride of Formula XIV to provide a 1*H*-imidazo[4,5-*c*]quinolinyl sulfamide of Formula XV which is a subgenus of Formula I. The reaction can be carried out by adding a solution containing 2 equivalents of the amine and 2 equivalents of triethylamine in dichloromethane to the reaction mixture from step (1). The addition is preferably carried out at a reduced temperature (-78°C). After the addition is complete the reaction mixture can be allowed to warm to ambient temperature. The product or a pharmaceutically acceptable salt thereof can be isolated using conventional methods.

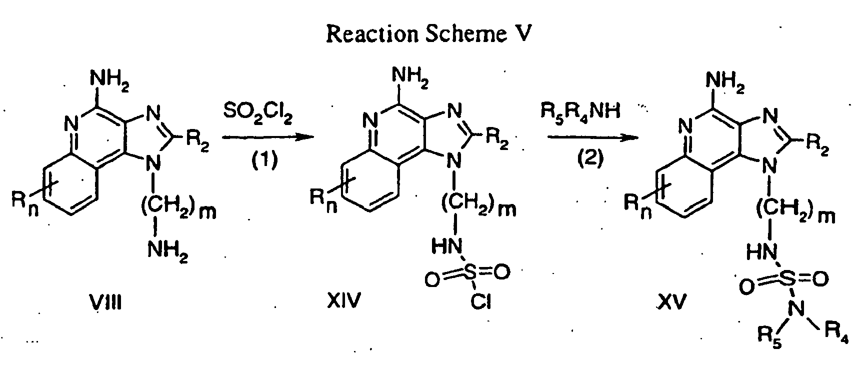

Tetrahydroimidazoquinolines of the invention can be prepared according to Reaction Scheme VI where R₂, R₃, R₄, and R₅ are as defined above and m is 1-20.

In step (1) of Reaction Scheme VI an aminoalkyl substituted 1*H*-imidazo[4,5-*c*]quinolin-4-amine of Formula XVI is reduced to provide an aminoalkyl substituted 6,7,8,9-tetrahydro-1*H*-imidazo[4,5-*c*]quinolin-4-amine of Formula XVII. Preferably the reduction is carried out by suspending or dissolving the compound of Formula XVI in trifluoroacetic acid, adding a catalytic amount of platinum (IV) oxide, and then subjecting the mixture to hydrogen pressure. The reaction can conveniently be carried out on a Parr apparatus. The product or a salt thereof can be isolated using conventional methods.

In step (2a) of Reaction Scheme VI an aminoalkyl substituted 6,7,8,9-tetrahydro-1*H*-imidazo[4,5-*c*]quinolin-4-amine of Formula XVII is reacted to provide a compounds of Formula XVIII which is a subgenus of Formula I. When R₃ is hydrogen, the reaction can be carried out in one step according to the methods described in Reaction Schemes II and III above using a tetrahydroimidazoquinoline of Formula XVII in place of the imidazoquinoline of Formula VIII. When R₃ is other than hydrogen, the reaction can be carried out in two steps with step one being carried out according to the methods of Reaction Schemes II and III and step two being carried out according to the method of Reaction IV using the tetrahydroimidazoquinoline analog of the imidazoquinoline. The product or a pharmaceutically acceptable salt thereof can be isolated using conventional methods.

In step (2b) of Reaction Scheme VI an aminoalkyl substituted 6,7,8,9-tetrahydro-1*H*-imidazo[4,5-*c*]quinolin-4-amine of Formula XVII is reacted to provide a compound of Formula XIX which is a subgenus of Formula I. The reaction can be carried out according to the method described in Reaction Scheme V using a tetrahydroimidazoquinoline of Formula XVII in place of the imidazoquinoline of Formula VIII. The product or a pharmaceutically acceptable salt thereof can be isolated using conventional methods.

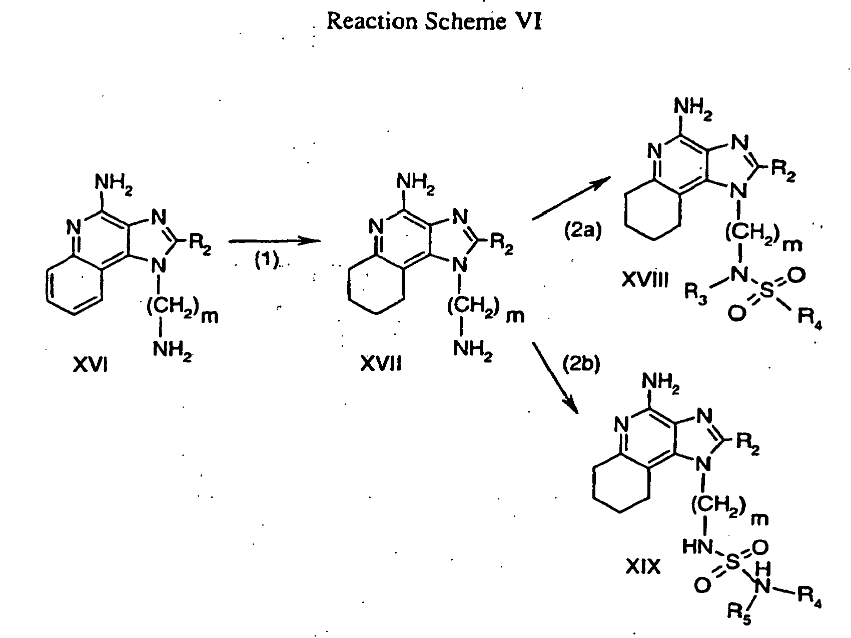

Tetrahydroimidazoquinolines of the invention can also be prepared according to Reaction Scheme VII where R, R₂, R₃, R₄, R₅ and n are as defined above and m is 1-20.

In step (1) of Reaction Scheme VII a 6,7,8,9-tetrahydro-1*H*-imidazo[4,5-*c*]quinolinyl *tert-*butylcarbamate of Formula XX is hydrolyzed to provide an aminoalkyl substituted 6,7,8,9-tetrahydro-1*H*-imidazo[4,5-*c*]quinolin-4-amine of Formula XXI. The reaction can be carried out dissolving the compound of Formula XX in a mixture of trifluoroacetic acid and acetonitrile and stirring at ambient temperature. Alternatively, the compound of Formula XX can be combined with dilute hydrochloric acid and heated on a steam bath. Tetrahydro-1*H*-imidazo[4,5-*c*]quinolinyl *tert*-butylcarbamates of Formula XX can be prepared using the synthetic route disclosed in U.S. Patent 5,352,784 (Nikolaides). The product or a salt thereof can be isolated using conventional methods.

Steps (2a) and (2b) can be carried out in the same manner as in Reaction Scheme VI.

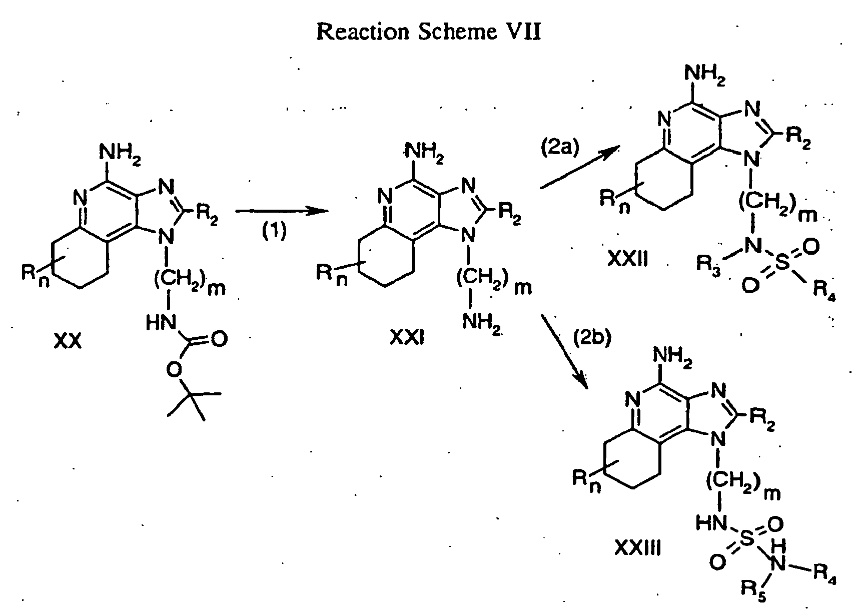

Some compounds of Formula I can be readily prepared from other compounds of Formula I. For example, compounds wherein the R₄ substituent contains a chloroalkyl group can be reacted with an amine to provide an R₄ substituent substituted by a secondary or teriary amino group; compounds wherein the R₄ substituent contains a nitro group can be reduced to provide a compound wherein the R₄ substituent contains a primary amine.

As used herein, the terms "alkyl", "alkenyl", "alkynyl" and the prefix."-alk" are inclusive of both straight chain and branched chain groups and of cyclic groups, i.e. cycloalkyl and cycloalkenyl. Unless otherwise specified, these groups contain from 1 to 20 carbon atoms, with alkenyl and alkynyl groups containing from 2 to 20 carbon atoms. Preferred groups have a total of up to 10 carbon atoms. Cyclic groups can be monocyclic or polycyclic and preferably have from 3 to 10 ring carbon atoms. Exemplary cyclic groups include cyclopropyl, cyclopentyl, cyclohexyl and adamantyl.

The term "haloalkyl" is inclusive of groups that are substituted by one or more halogen atoms, including groups wherein all of the available hydrogen atoms are replaced by halogen atoms. This is also true of groups that include the prefix "haloalk-". Examples of suitable haloalkyl groups are chloromethyl, trifluoromethyl, and the like.

The term "aryl" as used herein includes carbocyclic aromatic rings or ring systems. Examples of aryl groups include phenyl, naphthyl, biphenyl, fluorenyl and indenyl. The term "heteroaryl" includes aromatic rings or ring systems that contain at least one ring hetero atom (e.g., O, S, N). Suitable heteroaryl groups include furyl, thienyl, pyridyl, quinolinyl, tetrazolyl, imidazo, pyrazolo, thiazolo, oxazolo, and the like.

"Heterocyclyl" includes non-aromatic rings or ring systems that contain at least one ring hetero atom (e.g., O, S, N). Exemplary heterocyclic groups include pyrrolidinyl, tetrahydrofuranyl, morpholinyl, thiomorpholinyl, piperidinyl, piperazinyl, thiazolidinyl, imidazolidinyl, and the like.

Unless otherwise specified, the terms "substituted cycloalkyl", "substituted aryl", "substituted heteroaryl" and "substituted heterocyclyl" indicate that the rings or ring systems in question are further substituted by one or more substituents independently selected from the group consisting of alkyl, alkoxy, alkylthio, hydroxy, halogen, haloalkyl, haloalkylcarbonyl, haloalkoxy (e.g., trifluoromethoxy), nitro, alkylcarbonyl, alkenylcarbonyl, arylcarbonyl, heteroarylcarbonyl, aryl, arylalkyl, heteroaryl, heteroarylalkyl, heterocyclyl, heterocycloalkyl, nitrile, alkoxycarbonyl, alkanoyloxy, alkanoylthio, and in the case of cycloalkyl and heterocyclyl, oxo.

In structural formulas representing compounds of the invention certain bonds are represented by dashed lines. These lines mean that the bonds represented by the dashed line can be present or absent. Accordingly, compounds of Formula I can be either imidazoquinoline compounds or tetrahydroimidazoquinoline compounds.

The invention is inclusive of the compounds described herein in any of their pharmaceutically acceptable forms, including isomers such as diastereomers and enantiomers, salts, solvates, polymorphs, and the like.

### Pharmaceutical Compositions and Biological Activity

Pharmaceutical compositions of the invention contain a therapeutically effective amount of a compound of Formula I in combination with a pharmaceutically acceptable carrier.

As used herein, the term "a therapeutically effective amount" means an amount of the compound sufficient to induce a therapeutic effect, such as cytokine induction, antitumor activity and/or antiviral activity. Although the exact amount of active compound used in a pharmaceutical composition of the invention will vary according to factors known to those of skill in the art, such as the physical and chemical nature of the compound as well as the nature of the carrier and the intended dosing regimen, it is anticipated that the compositions of the invention will contain sufficient active ingredient to provide a dose of about 100ng/kg to about 50mg/kg, preferably about 10µg/kg to about 5mg/kg of the compound to the subject. Any of the conventional dosage forms may be used, such as tablets, lozenges, parenteral formulations, syrups, creams, ointments, aerosol formulations, transdermal patches, transmucosal patches and the like.

The compounds of the invention have been shown to induce the production of certain cytokines in experiments performed according to the tests set forth below. These results indicate that the compounds are useful as immune response modifiers that can modulate the immune response in a number of different ways, rendering them useful in the treatment of a variety of disorders.

Cytokines that may be induced by the administration of compounds according to the invention generally include interferon-α (IFN-α) and tumor necrosis factor-α (TNF-α) as well as certain interleukins (IL). Cytokines whose biosynthesis may be induced by compounds of the invention include IFN-α TNF-α, IL-1, 6, 10 and 12, and a variety of other cytokines. Among other effects, cytokines inhibit virus production and tumor cell growth, making the compounds useful in the treatment of viral diseases and tumors.

In addition to the ability to induce the production of cytokines, the compounds of the invention affect other aspects of the innate immune response. For example, natural killer cell activity may be stimulated, an effect that may be due to cytokine induction. The compounds may also activate macrophages, which in turn stimulates secretion of nitric oxide and the production of additional cytokines. Further, the compounds may cause proliferation and differentiation of B-lymphocytes.

Compounds of the invention also have an effect on the acquired immune response. For example, although there is not believed to be any direct effect on T cells or direct induction of T cell cytokines, the production of the T helper type 1 (Th1) cytokine IFN-γ is induced indirectly and the production of the T helper type 2 (Th2) cytokines IL-4, IL-5 and IL-13 are inhibited upon administration of the compounds. This activity means that the compounds are useful in the treatment of diseases where upregulation of the Th1 response and/or downregulation of the Th2 response is desired. In view of the ability of compounds of Formula Ia to inhibit the Th2 immune response, the compounds are expected to be useful in the treatment of atopic diseases, e.g., atopic dermatitis, asthma, allergy, and allergic rhinitis; and systemic lupus erythematosis; as a vaccine adjuvant for cell mediated immunity; and possibly as a treatment for recurrent fungal diseases and chlamydia.

The immune response modifying effects of the compounds make them useful in the treatment of a wide variety of conditions. Because of their ability to induce the production of cytokines such as IFN-α and/orTNF-α, the compounds are particularly useful in the treatment of viral diseases and tumors. This immunomodulating activity suggests that compounds of the invention are useful in treating diseases such as, but not limited to, viral diseases including genital warts; common warts; plantar warts; Hepatitis B; Hepatitis C; Herpes Simplex Virus Type I and Type II; molluscum contagiosum; HIV; CMV; VZV; intraepithelial neoplasias such as cervical intraepithelial neoplasia; human papillomavirus (HPV) and associated neoplasias: fungal diseases, e.g. candida, aspergillus, and cryptococcal meningitis; neoplastic diseases, e.g., basal cell carcinoma, hairy cell leukemia, Kaposi's sarcoma, renal cell carcinoma, squamous cell carcinoma, myelogenous leukemia, multiple myeloma, melanoma, non-Hodgkin's lymphoma, cutaneous T-cell lymphoma, and other cancers; parasitic diseases, e.g. pneumocystis carnii, cryptosporidiosis, histoplasmosis, toxoplasmosis, trypanosome infection, leishmaniasis; and bacterial infections, e.g., tuberculosis, mycobacterium avium. Additional diseases or conditions that can be treated using the compounds of the invention include eczema; eosinophilia; essential thrombocythaemia; leprosy; multiple sclerosis; Ommen's syndrome; discoid lupus; Bowen's disease; Bowenoid papulosis; and to enhance or stimulate the healing of wounds, including chronic wounds.

Accordingly, the present specification describes a use of a compound of Formula I for the manufacture of a medicament for inducing cytokine biosynthesis in an animal. An amount of a compound effective to induce cytokine biosynthesis is an amount sufficient to cause one or more cell types, such as monocytes, macrophages, dendritic cells and B-cells to produce an amount of one or more cytokines such as, for example, IFN-α, TNF-α, IL-1,6,10 and 12 that is increased over the background level of such cytokines. The precise amount will vary according to factors known in the art but is expected to be a dose of about 100ng/kg to about 50 mg/kg, preferably about 10µ g/kg to about 5mg/kg. The present specification also describes a use of a compound of Formula I for the manufacture of a medicament for treating a viral infection in an animal and for treating a neoplastic disease in an animal. An amount effective to treat or inhibit a viral infection is an amount that will cause a reduction in one or more of the manifestations of viral infection, such as viral lesions, viral load, rate of virus production, and mortality as compared to untreated control animals. The precise amount will vary according to factors known in the art but is expected to be a dose of 100ng/kg to about 50mg/kg, preferably about 10µg/kg to about 5mg/kg. An amount of a compound effective to treat a neoplastic condition is an amount that will cause a reduction in tumor size or in the number of tumor foci. Again, the precise amount will vary according to factors known in the art but is expected to be a dose of about 100ng/kg to about 50mg/kg, preferably about 10µg/kg to about 5mg/kg.

The invention is further described by the following examples, which are provided for illustration only and are not intended to be limiting in any way.

### Example 1

### N¹-[4-(4-Amino-2-butyl-1H-imidazo[4,5-c]quinolin-1-yl)butyl]-5-(dimethylamino)-1-naphthalenesulfonamide

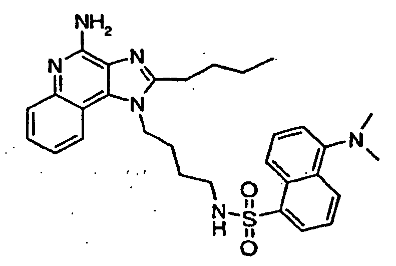

5-Dimethylamino-l-naphthalenesulfonyl chloride (1.82 g, 6.74 mmol) was added to a mixture of N,N-diisopropylethylamine (1.23 mL, 7.06 mmol), dichloromethane (15 mL) and 1-(4-aminobutyl)-2-butyl-1*H*-imidazo[4,5-c]quinolin-4-amine (2.0 g, 6.42 mmol). The reaction mixture was allowed to stir at ambient temperature overnight. Methanol was added to the reaction mixture until a clear solution was obtained. Silica gel was added to the reaction mixture and then the solvents were removed. The silica gel was placed in a column and then eluted with chloroform in a stepwise gradient to 9:1 chloroform:methanol. The resulting product was recrystallized from N,N-dimethylformamide and deionized water to provide 2.5 g of N¹-[4-(4-amino-2-butyl-1*H-*imidazo[4,5-*c*]quinolin-1-yl)butyl]-5-(dimethylamino)-1-naphthalenesulfonamide as a yellow crystalline solid, m.p. 223 -224°C. Analysis: Calculated for C₃₀H₃₆N₆O₂S: %C, 66.15; %H, 6.66; %N, 15.43; Found: %C, 66.36; %H, 6.34; %N, 15.23.

### Example 2

### N¹-[4-(4-Amino- 1H-imidazo[4,5-c]quinolin-1-yl)butyl]-5-(dimethylamino)-1-naphthalenesulfonamide

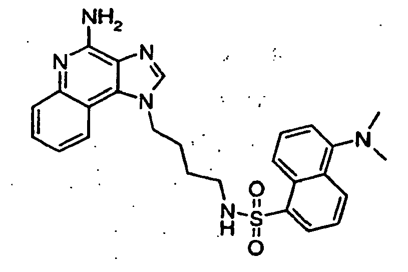

A suspension of 1-(4-aminobutyl)-1*H*-imidazo[4,5-c]quinolin-4-amine (0.5 g, 2.0 mmol) in pyridine (250 mL) was warmed to 60°C to dissolve the amine. The solution was allowed to cool to about 30°C and then 5-dimethylamino-1-naphthalenesulfonyl chloride (0.5 g, 1.8 mmol) was slowly added. After 1, hour 0.3 g of 5-dimethylamino-1-naphthalenesulfonyl chloride was added. The reaction mixture was warmed to 60°C and maintained at that temperature overnight. The reaction mixture was concentrated under vacuum. The residue was recrystallized from propyl acetate to provide N¹-[4-(4-amino-1*H*-imidazo[4,5-*c*]quinolin-1-yl)butyl]-5-(dimethylamino)-1-naphthalenesulfonamide as a solid, m.p. 200-201°C.

### Example 3

### N²[4-(4-Amino-2-butyl-1H-imidazo[4,5-c]quinolin-1-yl)butyl]-2-thiophenesulfonamide

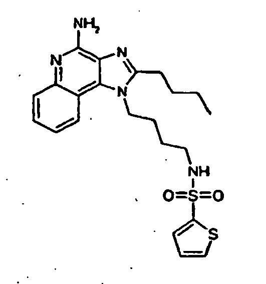

2-Thiophenesulfonyl chloride (0.3 g in 10 ml dichloromethane, 1.6 mmol) was added dropwise to a stirring solution of 1-(4-aminobutyl)-2-butyl-1*H*-imidazo[4,5-c]quinoline-4-amine (0.5 g, 1.6 mmol), dichloromethane (40 ml), and pyridine (0.8 ml). The reaction was maintained at room temperature for a few hours and then an additional portion of 2-thiophenesulfonyl chloride (0.1 g, 0.6 mmol) was added. The reaction was maintained overnight and then concentrated *in vacuo.* The resulting residue was purified by flash column chromatography (silica gel, 9:1 dichloromethane\methanol) and the fractions containing product were washed with saturated aqueous sodium bicarbonate. The organic layer was dried (MgSO₄), filtered, and concentrated to provide 0.2 g of N²-[4-(4-amino-2-butyl-1*H*-imidazo[4,5-c]quinolin-1-yl)butyl]-2-thiophenesulfonamide as an off white powder, m.p. 137.5-141.5 °C. ¹H NMR (300 MHz, DMSO-d₆) δ 8.00 (d, J=8.0 Hz, 1H), 7.89 (dd, J=5.0, 1.3 Hz, 1H), 7.83 (broad s, 1H), 7.61 (dd, J=8.3, 1.1 Hz, 1H), 7.54 (dd, J=3.7, 1.3 Hz, 1H), 7.42 (t, J=7.2 Hz, 1H), 7.25 (m, 1H), 7.15 (m, 1H), 6.44 (broad s, 2H), 4.47 (t, J=7.4 Hz, 2H), 2.87 (m, 4H), 1.80 (m, 4H), 1.58-1.38 (m, 4H), 0.96 (t, J=7.4 Hz, 3H); IR (KBr) 3467, 3361, 3167, 3091, 2957, 2933, 2870, 1644, 1617, 1585, 1533, 1478, 1405, 1336, 1154, 1095, 1014, 854, 761, 733 cm⁻¹ ; MS (EI) m/e 457.1606 (457.1606 calcd for C₂₂H₂₇N₅O₂S₂); Anal calcd for C₂₂H₂₇N₅O₂S₂: C, 57.74; H, 5.95; N, 15.30. Found: C, 57.50; H, 5.98; N, 15.15.

### Example 4

### N-[4-(4-Amino-2-butyl-1H-imidazo[4,5-c]quinolin-1-yl)butyl] phenylmethanesulfonamide .

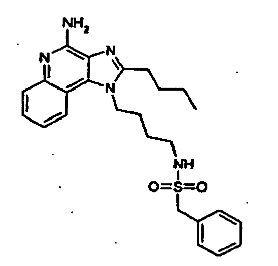

α-Toluenesulfonyl chloride (0.5 g in 10 ml dichloromethane, 2.7 mmol) was added dropwise to a stirring solution of 1-(4-aminobutyl)-2-butyl-1*H*-imidazo[4,5-c]quinoline-4-amine (0.75 g, 2.4 mmol), dichloromethane (115 ml), and pyridine (1 ml). The reaction was maintained at room temperature for 4 hours and then concentrated *in vacuo.* The residue was purified by flash column chromatography (silica gel, 9:1 dichloromethane\methanol, Rf 0.16). The fractions containing product were combined and washed with saturated aqueous bicarbonate. The organic layer was dried (MgSO₄), filtered, and concentrated. A final recrystallization from dichloromethane\diethyl ether provided 0.65 g of N-[4-(4-amino-2-butyl-1*H*-imidazo[4,5-c]quinolin-1-yl)butyl]phenylmethanesulfonamide as a white crystalline solid, m.p. 197.0-199.5 °C. ¹H NMR (300 MHz, DMSO-d₆) δ 8.02 (d, J=7.6 Hz, 1H), 7.62 (dd, J=8.3, 1.1 Hz, 1H), 7.42 (dt, J=7.5, 1.1 Hz, 1H), 7.35-7.23 (m, 7H), 7.12 (t, J=5.4 Hz, 1H), 6.46 (broad s, 2H), 4.49 (t, J=7.5 Hz, 2H), 4.29 (s, 2H), 2.91 (m, 4H), 1.83-1.42 (m, 8H), 0.96 (t, J=7.4 Hz, 3H); IR (KBr) 3460, 3293, 3226, 3158, 2955, 2931, 2867, 1632, 1586, 1534, 1482, 1437, 1389, 1331, 1152, 1094,752, 700 cm⁻¹; MS (EI) m/e 465.2204 (465.2198 calcd for C₂₅H₃₁N₅O₂S); Anal calcd for C₂₅H₃₁NSO₂S: C, 64.49; H, 6.71; N, 15.04. Found: C, 64:15; H, 6.71; N, 15.00.

### Example 5

### N¹-[4-(4-Amino-2-butyl-1H-imidazo[4,5-c]quinolin-1-yl)butyl]-1-benzenesulfonamide

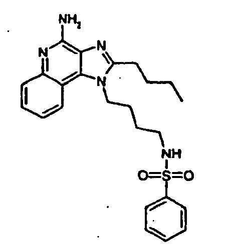

Benzenesulfonyl chloride (0.45 ml in 10 ml dichloromethane, 3.5 mmol) was added dropwise to a stirring solution of 1-(4-aminobutyl)-2-butyl-1*H*-imidazo[4,5-c]quinoline-4-amine (1.0 g, 3.2 mmol), dichloromethane (140 ml), and pyridine (0.8 ml). The reaction was maintained at room temperature for four hours and then concentrated *in vacuo.* The residue was purified by flash column chromatography (silica gel, 9:1 dichloromethane\methanol, R_{f} 0.28) followed by recrystallization from dichloromethane\diethyl ether to provide 1.14 g of N¹-[4-(4-amino-2-butyl-1*H-*imidazo[4,5-c]quinolin-1-yl)butyl]-1-benzenesulfonamide as a white powder, m.p. 75.5-79.0 °C. ¹H NMR (500 MHz, DMSO-d₆) δ 7.99 (d, J=7.7 Hz, 1H), 7.76 (d, J=7.2, 2H), 7.63-7.53 (m, 5H), 7.42 (m, 1H), 7.25 (m, 1H), 6.43 (broad s, 2H), 4.45 (t, J=7.6 Hz, 2H), 2.87 (t, J=7.7 Hz, 2H), 2.78 (m, 2H), 1.79 (m, 4H), 1.55-1.40 (m, 4H), 0.95 (t, J=7.4 Hz, 3H); MS (EI) m/e 451.2036 (451.2042 calcd for C₂₄H₂₉N₅O₂S); Anal calcd for C₂₄H₂₉N₅C₂S: C, 63.83; H, 6.47; N, 15.51. Found: C, 63.89; H, 6.42; N, 15.30.

### Example 6

### N-[4-(4-Amino-2-butyl-1H-imidazo[4,5-c]quinolin-7-yl)butyl] methanesulfonamide

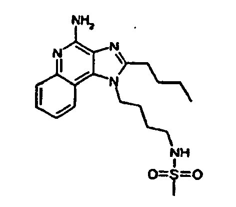

Methanesulfonic anhydride (0.6 g, 3.4 mmol) was added dropwise to a stirring solution of 1-(4-aminobutyl)-2-butyl-1*H*-imidazo[4,5-c]quinoline-4-amine (1.0 g, 3.2 mmol) and acetonitrile (200 ml). A precipitate formed within a few minutes. The solvent was removed *in vacuo* and the residue was partitioned between dichloromethane and saturated aqueous sodium bicarbonate. The fractions were separated and the organic fraction was dried (MgSO₄) filtered and concentrated to yield the crude product as a white solid. Recrystallization from methyl acetate provided N-[4-(4-amino-2-butyl-1*H-*imidazo[4,5-c]quinolin-1-yl)butyl]methanesulfonamide as a white crystalline solid, m.p. 195.1-196.0°C. ¹H NMR (300 MHz, DMSO-d₆) δ 8.04 (d, J=7.4 Hz, 1H), 7.61 (dd, J=8.3, 1.2 Hz, 1H), 7.50 (dt, J=7.5, 1.1 Hz, 1H), 7.26 (dt, J=7.5, 1.2 Hz, 1H), 6.99 (t, J=5.7 Hz, 1H), 6.44 (broad s, 2H), 4.52 (t, J=7.5 Hz, 2H), 3.02-2.86 (m, 7H), 1.82 (m, 4H), 1.62 (m, 2H), 1.46 (q, J=7.4 Hz, 2H), 0.96 (t, J=7.4 Hz, 3H); IR (KBr) 3348, 3299, 3152, 2952, 2931, 2869, 1642, 1584, 1530, 1480, 1323, 1155, 1142, 1094, 982, 765 cm⁻¹ ; MS (EI) m/e 389.1889 (389.1885 calcd for C₁₉H₂₇N₅O₂S); Anal calcd for C₁₉H₂₇N₅O₂S: C, 58.59; H, 6.99; N, 17.98. Found: C, 58.26; H, 6.64; N, 17.69

### Example 7

### N¹-[4-(4-Amino-2-butyl-1H-imidazo[4,5-c]quinolin-1-yl)butyl]-3-nitro- -benzenesulfonamide Hydrochloride

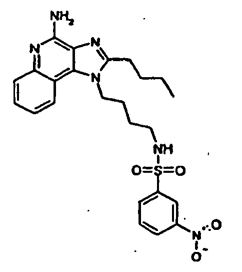

According to the general method of Example 5, 3-nitrobenzenesulfonyl chloride and 1-(4-aminobutyl)-2-butyl-1*H*-imidazo[4,5-c]quinoline-4-amine were combined. N¹-[4-(4-Amino-2-butyl-1*H*-imidazo[4,5-c]quinolin-1-yl)butyl]-3-nitro-1-benzenesulfonamide was isolated as the hydrochloride salt (white solid), m.p. 176.0-178.2 °C. ¹H NMR (300 MHz, DMSO-d₆) δ 8.70 (very broad s, 2H), 8.49-8.42 (m, 2H), 8.21-8.17 (m, 2H), 8.06 (t, J=5.7 Hz, 1H), 7.88-7.81 (m, 2H), 7.71 (t, J=7.7 Hz, 1H), 7.57 (t, J=7.7 Hz, 1H), 4.56 (t, J=7.3 Hz, 2H), 2.94 (t, J=7.7 Hz, 2H), 2.86 (m, 2H), 1.81 (m, 4H), 1.60-1.42 (m, 4H), 0.96 (t, J=7.3 Hz, 3H); IR (KBr) 3096, 2954, 2869, 2771, 1671, 1607, 1528, 1351, 1335, 1163, 1128, 1083, 879, 758, 735, 672, 661 cm⁻¹; MS (EI) m/e 496.1897 (496.1893 calcd for C₂₄H₂₈N₆O₄S). Anal calcd for C₂₄H₂₈N₆O₄S*HCl*H₂O: C, 52.31; H, 5.67; N, 15.25. Found: C, 52.26; H, 5.46; N, 15.09.

### Example 8

### N¹-[4-(4-Amino-2-butyl-1H-imidazo[4,5-c]quinolin-1-yl)butyl]-3-amino- 1-benzenesulfonamide Hydrochloride

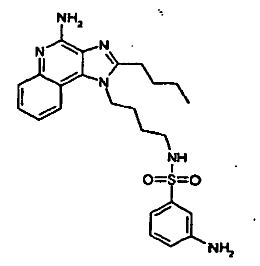

A solution of N¹-[4-(4-amino-2-butyl-1*H*-imidazo[4,5-c]quinolin-1-yl)butyl]-3-nitro-1-benzenesulfonamide hydrochloride (0.4 g) in methanol (250 ml) was charged with a catalytic amount of 10% palladium on carbon (0.085 g). The reaction was placed under an atmosphere of hydrogen (50 psi; 3.44 X 10⁵ Pa) and shaken on a Parr apparatus for 2 hours. The reaction mixture was filtered and the solvent removed in *vacuo.* The solid product was recrystallized from 2-propanol to provide 0.18 g of N¹-[4-(4-amino-2-butyl-1*H*-imidazo[4,5-c]quinolin-1-yl)butyl]-3-amino-1-benzenesulfonamide hydrochloride as an off white crystalline solid, m.p. 110.2 °C (decomposition). ¹H NMR (300 MHz, DMSO-d₆) δ 8.70 (very broad s, 2H), 8.22 (d, J=8.2 Hz, 1H), 7.83 (d, J=7.8 Hz, 1H), 7.72 (t, J=7.6 Hz, 1H), 7.59 (t, J=7.7 Hz, 1H), 7.43 (t, J=5.9 Hz, 1H), 7.15 (t, J=7.9 Hz, 1H), 6.95 (t, J=1.9 Hz, 1H), 6.84 (d, J=7.7 Hz, 1H), 6.73 (dd, J=8.0, 1.5 Hz, 1H), 5.63 (broad s, 2H), 4.56 (t, J=7.5 Hz, 2H), 2.96 (t, J=7.7 Hz, 2H), 2.77 (q, J=6.3 Hz, 2H), 1.83 (m, 4H), 1.60-1.40 (m, 4H), 0.97 (t, J=7.3 Hz, 3H); IR (KBr) 3313, 3135, 2957, 2870, 2782, 1671, 1599, 1485, 1454, 1313, 1155, 1084, 754, 686 cm⁻¹ ; MS (EI) m/e 466.2150 (466.2151 calcd for C₂₄H₃₀N₆O₂S). Anal calcd for C₂₄H₃₀N₆O₂S*HCl*0.25H₂O: C, 56.79; H, 6.26; N, 16.56; Cl, 6.98. Found: C, 56.87; H, 6.22; N, 16.19; Cl, 7.22.

### Example 9

### N¹-[4-(4-Amino-2-butyl-1H-imidazo[4,5-c]quinolin-1-yl)butyl]-4-nitro-1-benzenesulfonamide Hydrochloride

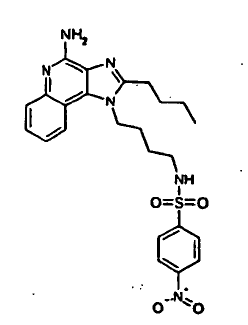

According to the general method of Example 5, 4-nitrobenzenesulfonyl chloride and 1-(4-aminobutyl)-2-butyl-1*H*-imidazo[4,5-c]quinoline-4-amine were combined. N¹-[4-(4-Amino-2-butyl-1*H*-imidazo[4,5-c]quinolin-1-yl)butyl]-4-nitro-1-benzenesulfonamide was isolated as the hydrochloride salt (white solid), m.p. 96.0 °C (decomposition). ¹H NMR (300 MHz, DMSO-d₆) δ 8.70 (very broad s, 2H), 8.38-8.34 (m, 2H), 8.19 (d, J=8.2 Hz, 1H), 8.09 (t, J=5.6 Hz, 1H), 8.03-7.99 (m, 2H), 7.80 (d, J=7.4 Hz, 1H), 7.68 (t, J=7.4 Hz, 1H), 7.54 (t, J=7.2 Hz, 1H), 4.55 (t, J=7.4 Hz, 2H), 2.94 (t, J=7.7 Hz, 2H), 2.86 (q, J=6.2 Hz, 2H), 1.80 (m, 4H), 1.58 (m, 2H), 1.45 (q, J=7.5 Hz, 2H), 0.96 (t, J=7.3 Hz, 3H); IR (KBr) 3283, 3100, 2957, 2870, 2782, 1670, 1606, 1528, 1347, 1311, 1162, 1092, 854, 746, 737, 686 cm⁻¹; MS (EI) m/e 496.1902 (496.1893 calcd for C₂₄H₂₈N₆O₄S). Anal calcd for C₂₄H₂₈N₆O₄S*HCl*0.85H₂O: C, 52.57; H, 5.64; N, 15.33. Found: C, 52.57; H, 5.46; N, 15.33.

### Example 10

### N¹-[4-(4-Amino-2-butyl-1H-imidazo[4,5-c]quinolin-1-yl)butyl]-4-amino-1-benzenesulfonamide Hydrochloride

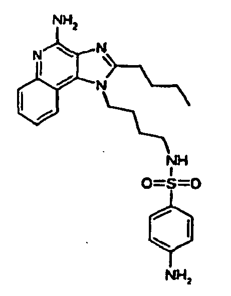

A solution of N¹-[4-(4-amino-2-butyl-1*H-*imidazo[4.5-c]quinolin-1-yl)butyl]-4-nitro-1-benzenesulfonamide hydrochloride (0.38 g) in methanol (250 ml) was charged with a catalytic amount of 10% palladium on carbon (0.085 g). The reaction was placed under an atmosphere of hydrogen (50 psi; 3.44 X 10⁵ Pa)) and shaken on a Parr apparatus for 2 hours. The reaction mixture was filtered and the solvent removed *in vacuo.* The solid product was recrystallized from 2-propanol to provide 0.34 g of N¹-[4-(4-amino-2-butyl-1*H*-imidazo[4,5-c]quinolin-1-yl)butyl]-4-amino-1-benzenesulfonamide hydrochloride as an off white powder, m.p. 203.1-205.0 °C. ¹H NMR (300 MHz, DMSO-d₆) δ 8.65 (very broad s, 2H), 8.2 1 (d, J=8.0 Hz, 1H), 7.82 (m, 1H), 7.71 (t, J=7.7 Hz, 7.58 (t, J=7.7 Hz, 1H), 7.38 (d, J=8.7 Hz, 2H), 7.13 (t, J=5.9 Hz, 1H), 6.60 (d, J=8.7 Hz, 2H), 5.92 (broad s, 2H), 4.55 (t, J=7.6 Hz, 2H), 2.96 (t, J=7.6 Hz, 2H), 2.70 (q, J=6.4 Hz, 2H), 1.81 (m, 4H), 1.58-1.43 (m, 4H), 0.96 (t, J=7.4 Hz, 3H); IR (KBr) 3430, 3316, 3215, 3046, 2955, 2868, 2679, 1671, 1594, 1334, 1157, 1091, 851, 776, 759 cm⁻¹; MS (EI) m/e 466.2145 (466.2151 calcd for C₂₄H₃₀N₆O₂S). Anal calcd for C₂₄H₃₀N₆O₂S*HCl: C, 57.30; H, 6.21; N, 16.71. Found: C, 57.36; H, 6.31; N, 16.21.

### Example 11

### N⁵-[4-(4-Amino-2-butyl-1H-imidazo[4,5-c]quinolin-1-yl)butyl]-5-isoquinolinesulfonamide

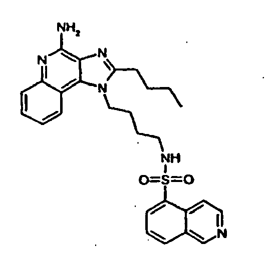

A suspension of isoquinoline-5-sulfonyl chloride hydrochloride (0.83 g in 50 ml of pyridine, 3.1 mmol) was added dropwise to a stirring solution of 1-(4-aminobutyl)-2-butyl-1*H*-imidazo[4,5-c]quinoline-4-amine (1.0 g, 3.2 mmol) and dichloromethane (175 ml). The solution turned a bright yellow color and was maintained at room temperature for 4 hours. An additional 0.18 g of isoquinoline-5-sulfonyl chloride hydrochloride was added and the reaction was maintained an additional 60 hours. The yellow solution was concentrated *in vacuo,* dissolved in dichloromethane, and washed sequentially with saturated aqueous sodium bicarbonate and water. The organic fraction was dried (MgSO₄), filtered, and concentrated *in vacuo.* The residue was purified by flash column chromatography (silica gel, 9:1 dichloromethane\methanol) to provide 0.7 g of N⁵-[4-(4-amino-2-butyl-1*H*-imidazo[4,5-c]quinolin-1-yl)butyl]-5-isoquinolinesulfonamide as a white crystalline solid, m.p. 96.0 °C (decomposition). ¹H NMR (300 MHz, DMSO-d₆) δ 9.44 (d, J=0.7 Hz, 1H), 8.64(d, J=6.1 Hz, 1H), 8.41-8.35 (m, 2H), 8.30 (dd, J=7.4, 1.2 Hz, 1H), 8.11 (t, J=5.6 Hz, 1H), 7.92 (d, J=7.6 Hz, 1H), 7.75 (t, J=7.7 Hz, 1H), 7.61 (dd, J=8.3, 1.2 Hz, 1H), 7.41 (dt, J=7.7, 1.2 Hz, 1H), 7.22 (dt, J=7.6, 1.2 Hz, 1H), 6.47 (broad s, 2H), 4.38 (t, J=7.5 Hz, 2H), 2.86-2.74 (m, 4H), 1.78-1.63 (m, 4H), 1.50-1.34 (m, 4H), 0.94 (t, J=7.4 Hz, 3H); MS (EI), m/e 502.2151 (502.2151 calcd for C₂₇H₃₀N₆O₂S). Anal calcd for C₂₇H₃₀N₆OS: C, 64.52; H, 6.02; N, 16.72. Found: C, 64.03; H, 6.03; N, 16.55.

### Example 12

### N-[4-(4-Amino-2-(4-methoxybenzyl)-1H-imidazo[4,5-c]quinolin-1-yl]butyl] methanesulfonamide

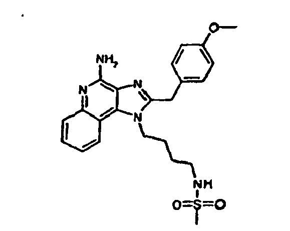

Methanesulfonic anhydride (0.19 g, 1.1 mmol) was added to a stirring solution of 1-(4-aminobutyl)-2-(4-methoxybenzyl)-1*H*-imidazo[4,5-c]quinolin-4-amine (0.4 g, 1.07 mmol), dichloromethane (75 ml) and acetonitrile (100 ml). The reaction was maintained at room temperature for 60 hours. The solvent was removed *in vacuo* and the residue was purified by flash column chromatography (silica gel, 9:1 dichloromethanelmethanol). The fractions containing product were combined, washed with saturated aqueous sodium bicarbonate, dried (MgSO₄), filtered, and concentrated to provide 0.3 g of N-[4-(4-amino-2-(4-methoxybenzyl)-1*H*-imidazo[4,5-c]quinolin-1-yl]butyl]methanesulfonamide as a white solid, m.p. 78.1-79.5 °C. ¹H NMR (300 MHz, DMSO-d₆) δ 7.99 (d, J=7.6 Hz, 1H), 7.62 (dd, J=8.3, 1.2 Hz, 1H), 7.42 (m, 1H), 7.27-7.21 (m, 3H), 6.98 (t, J=5.7 Hz, 1H), 6.89 (d, J=8.7 Hz, 2H), 6.58 (broad s, 2H), 4.45 (broad s, 2H), 4.33 (s, 2H), 3.72 (s, 3H), 2.87 (m, 5H), 1.55 (broad s, 2H); MS (CI) m/e 454 (M+H).

### Example 13

### N¹-[4-(4-Amino-1H-imidazo[4,5-c]quinolin-1-y)butyl]-1-butanesulfonamide

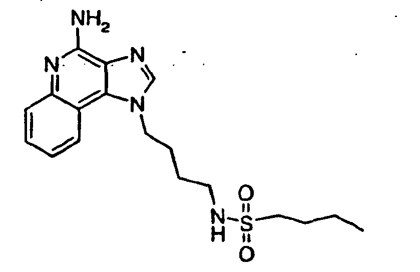

A solution of 1-(4-aminobutyl)-1*H*-imidazo[4,5-c]quinolin-4-amine (9.3 mg, 36 µmol) in 10 mL of dichloromethane in a screw-capped test tube was cooled down to -5°C. Butanesulfonyl chloride (45 µmol) was added as a 0.3 M solution in dichloromethane, with argon bubbling through the mixture during addition and for an additional 15 seconds. The mixture was allowed to stand at -5°C overnight. Aminomethyl polystyrene resin (ca. 90 mg, 0.62 meq/g, 100-200 mesh, Bachem) was added and the mixture was warmed to reflux and shaken at about 600 rpm for 3 hours. The mixture was filtered through a Poly-Prep column (Bio-Rad #731-1550) to remove resin. Solvent was removed *in vacuo* and the residue was purified by semi-preparative hplc on a Gilson system (Rainin Microsorb C18 column , 21.4 x 250 mm, 8 micron particle size, 60A pore, 10 mL/min., gradient elution from 2-95% B in 25 min., hold at 95% B for 5 min., where A=0.1 % trifluoroacetic acid/water and B=0.1 % trifluoroacetic acid/acetonitrile, peak detection at 254 nm for triggering fraction collection). The semi-prep hplc fractions were analyzed by LC-APCI/MS and the appropriate fractions were combined and lyophilized. The solid was dissolved in ca. 3 mL of 2:1 dichloromethane-methanol and shaken with ca. 80 mg (300 µmol) of diisopropylaminomethyl-polystyrene resin (Argonaut PS-DIEA, 3.86 mmol/g) for -2 h to liberate the free amine, and then filtered and dried *in vacuo* to give the product as a solid. MS (APCI) m/e 376.16 (M+H).

### Example 14

### N¹-{4-[4-Amino-2-(2-methoxyethyl)-6,7,8,9-tetrahydro-1H-imidazo[4,5-c]quinolin-1-yl]butyl}-4-fluoro-1-benzenesulfonamide

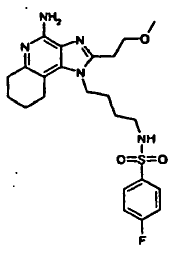

According to the general method of Example 5, 1-(4-aminobutyl)-2-(2-methoxyethyl)-6,7,8,9-tetrahydro-1*H*-imidazo[4,5-c]quinolin-4-amine and 4-fluorobenzenesulfonyl chloride were combined. Recrystallization from 4:1 n-propyl acetate\methanol provided N¹-{4-[4-amino-2-(2-methoxyethyl)-6,7,8,9-tetrahydro-1*H-*imidazo[4,5-c]quinolin-1-yl]butyl}-4-fluoro-1-benzenesulfonamide as a white crystalline solid, m.p. 191.0-193.0 °C. ¹H NMR (300 MHz, DMSO-d₆) δ 7.86-7.81 (m, 2H), 7.67 (broad s, 1H), 7.45-7.39 (m, 2H), 5.65 (broad s, 2H), 4.15 (m, 2H), 3.76 (t, J=6.7 Hz, 2H), 3.27 (s, 3H), 3.00 (t, J=6.7 Hz, 2H), 2.90 (broad s, 2H), 2.78 (m, 2H), 2.65 (broad s, 2H), 1.75 (broad s, 4H), 1.61 (m, 2H), 1.43 (m, 2H); MS (CI) m/e 476 (M+H). Analysis: Calculated for C₂₃H₃₀FN₅O₃S: %C, 58.09; %H, 6.36; %N, 14.73; Found: %C, 58.37; %H, 6.35; %N, 14.60.

### Example 15

### N-[4-(4-Amino-2-phenyl-1H-imidazo[4,5-c]quinolin-1-yl)butyl]-4-fluoro-1-benzenesulfonamide

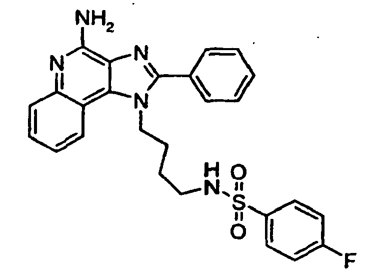

### Part A

A solution of benzoyl chloride (5.3 g, 37.7 mmol) in dichloromethane (100 mL) was slowly added to a solution of *tert*-butyl N-[4-[(3-aminoquinolin-4-yl)amino]butyl}carbamate (12.5 g, 37.7 mmol) in dichloromethane (250 mL) at ambient temperature. The reaction mixture was maintained at ambient temperature overnight. The resulting precipitate was isolated by filtration and dried to provide 11.0 g of *tert*-butyl N-(4-{[3-(benzoylamino)quinolin-4-yl]amino} butyl)carbamate hydrochloride as a white solid.

### Part B

Triethylamine (7.26 g, 71.7 mmol) was added to a solution of the material from Part A in ethanol (200 mL) and heated at reflux for 2 days. The reaction mixture was concentrated to provide an orange syrup. HPLC mass spec analysis showed that the syrup contained the desired product and starting material. The syrup was taken up in dichloromethane (100 mL) and then cooled in an ice bath. Triethylamine (5 mL) and benzoyl chloride (1.9 mL) were added. The reaction mixture was maintained at ambient temperature for 2 days at which time analysis by HPLC indicated that the reaction was not complete. The reaction mixture was concentrated under vacuum. The residue was taken up in isopropyl alcohol (150 mL). Triethylamine (5 mL) was added and the reaction mixture was heated at reflux overnight. The reaction mixture was concentrated under vacuum. The residue was purified by flash chromatography (silica gel: eluting with 10% methanol in dichloromethane). The fractions containing product were combined and concentrated under vacuum. The residue was recrystallized from acetonitrile to provide 6.7 g of *tert*-butyl N-[4-(2-phenyl-1*H*-imidazo[4,5-*c*]quinolin-1-yl)butyl]carbamate as a solid, m.p. 158-159°C.

### Part C

3-Chloroperoxybenzoic acid (1.05 eq of 65%) was slowly added in small portions to a solution of *tert*-butyl N-[4-(2-phenyl-1*H*-imidazo[4,5-*c*]quinolin-1-yl)butyl]carbamate (6.56 g, 15:75 mmol) in dichloromethane (120 mL). After 3 hours the reaction was quenched with 1% aqueous sodium bicarbonate (200 mL). The layers were separated. The aqueous layer was extracted with dichloromethane (2 X 50 mL). The organic fractions were combined, dried over magnesium sulfate and then concentrated under vacuum to provide a pale orange syrup. The syrup was triturated with diethyl ether to provide 6.8 g of 1-[4-(*tert*-butylcarbamyl)butyl]-2-phenyl-1*H*-imidazo[4,5-*c*]quinoline-5N-oxide as a pale tan solid, m.p. 178-181°C.

### Part D

A solution of 1-[4-(*tert*-butylcarbamyl)butyl]-2-phenyl-1*H*-imidazo[4,5-*c*]quinoline-5N-oxide (6.8 g, 15.75 mmol) in dichloromethane (100 mL) was chilled in an ice bath. Concentrated ammonium hydroxide (30 mL) was added. Tosyl chloride (3.0 g, 15.75 mmol) was added in small portions over a period of 30 minutes. The reaction mixture was allowed to warm to ambient temperature overnight. The reaction was quenched with water (350 mL). The layers were separated. The aqueous layer was extracted with dichloromethane. The organic fractions were combined, dried over magnesium sulfate and then concentrated under vacuum to provide a tan solid. This material was purified by flash chromatography (silica gel eluting with 10% methanol in dichloromethane) to provide 4.8 g of product. The bulk of the material was carried on to the next step. A small portion was recrystallized from toluene to provide *tert*-butyl N-[4-(4-amino-2-pheny]-1*H*-imidazo[4,5-*c*]quinolin-1-yl)butyl]carbamate as a solid, m.p. 182-183°C. Analysis: Calculated for C₂₅H₂₉N₅O₂: %C, 69.58; %H, 6.77; %N, 16.22; Found: %C, 69.86; %H, 6.95; %N, 15.80.

### Part E

The material from Part D was dissolved in methanol (15 mL) and 1 N hydrochloric acid (100 mL) and then heated at reflux for 2 hours. The reaction mixture was concentrated under vacuum to a volume of about 50 mL. Addition of concentrated ammonium hydroxide to pH 12 did not produce a precipitate. The pH was adjusted to 7 with 1N hydrochloric acid. The mixture was extracted with dichloromethane and then with ethyl acetate. The aqueous layer was concentrated to dryness. The residue was dissolved in water (50 mL) and then extracted continuously with refluxing chloroform for 36 hours. The chloroform extract was concentrated under vacuum to provide a light tan solid. This material was recrystallized from acetonitrile to provide 2.5 g of 1-(4-aminobutyl)-2-phenyl-1*H*-imidazo[4,5-*c*]quinolin-4-amine as an off white solid, m.p. 175-177°C. Analysis: Calculated for C₂₀H₂₁N₅: %C, 72.48; %H, 6.39; %N, 21.13; Found: %C, 72.72; %H, 632; %N, 20.71.

### Part F

1-(4-Aminobutyl)-2-phenyl-1*H*-imidazo[4,5-*c*]quinolin-4-amine (0.331 g, 1.0 mmol) was dissolved in anhydrous acetonitrile (35 mL) and the solution was cooled to 4°C. A solution of 4-fluorobenzenesulfonyl chloride (0.194 g, 1.0 mmol) in anhydrous dichloromethane (10 mL) was slowly added. The reaction was allowed to slowly warm to ambient temperature over the weekend. The reaction was quenched by the addition of aqueous saturated sodium bicarbonate solution. The layers were separated and the organic layer was concentrated to provide a pale yellow solid. This material was recrystallized from isopropyl alcohol and then further purified by flash chromatography (silica gel eluting with 10% methanol in dichloromethane). The pure fractions were combined and concentrated. The residue was recrystallized from isopropyl alcohol to provide 0.2 g of N-[4-(4-amino-2-phenyl-1*H*-imidazo[4,5-*c*]quinolin-1-yl)butyl]-4-fluoro-1-benzenesulfonamide as a pale yellow solid, m.p. 214-216°C. Analysis: Calculated for C₂₆H₂₄FN₅O₂)S: %C, 63.79; %H, 4.94; %N, 14.30; Found: %C, 63.19; %H, 4.85; %N, 13.90. Mass spec M+1 = 490.2

### Example 16

### N-[4-(4-Amino-2-phenyl-1H-imidazo[4,5-c]quinolin-1-yl)butyl] methanesulfonamide

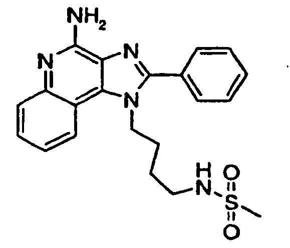

Using the general method of Example 15 Part F, 1-(4-aminobutyl)-2-phenyl-1*H-*imidazo[4,5-*c*]quinolin-4-amine (0.331 g, 1.0 mmol) was reacted with Methanesulfonic anhydride to provide 0.14 g of N-[4-(4-Amino-2-phenyl-1*H*-imidazo[4,5-*c*]quinolin-1-yl)butyl]methanesulfonamide as a white solid, m.p. 234-235°C. Mass spec M+1 = 410.2.

### Examples 17 - 33

The compounds shown in the Table below were prepared using the synthetic method described in Reaction Scheme II above.

1-(2-Aminoethyl)-2-butyl-1*H*-imidazo[4,5-*c*]quinolin-4-amine (25 mg) was placed in a 2 dram (7.4 mL) vial. Diisopropylethylamine (11 µL, 1.2 eq), dichloromethane (1 mL) and the sulfonyl chloride (1.1 eq) were added in order. The vial was placed on a shaker for about 2 hours and then on a sonicator for about 0.5 hours. The reaction mixture was allowed to stand at ambient temperature overnight and analyzed by LC/MS to confirm the formation of the desired product. The solvent was removed and the residue was purified by semi-preparative HPLC (Capcell Pak C18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfonamide.

| Example # | Structure of the Free Base | Observed Mass |
|---|---|---|
| 17 | 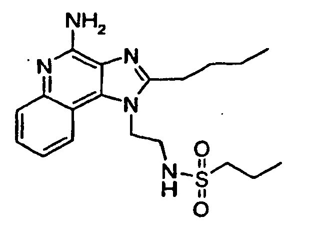 | 390.2 |
| 18 | 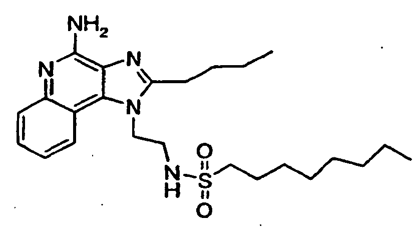 | 460.2 |
| 19 | 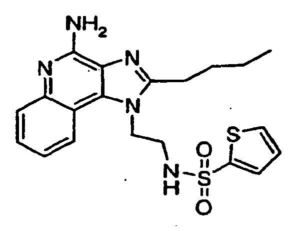 | 430.1 |
| 20 | 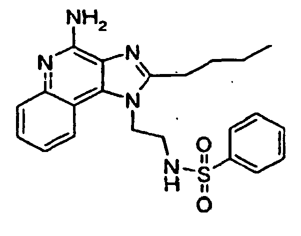 | 424.1 |
| 21 | 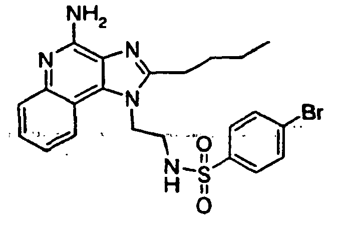 | 504.0 |
| 22 | 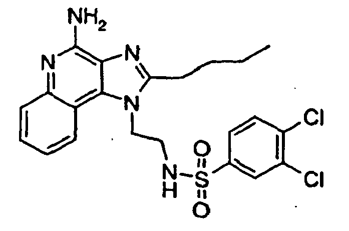 | 492.0 |
| 23 | 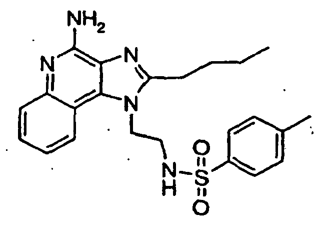 | 438.1 |
| 24 | 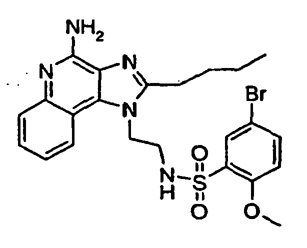 | 534.0 |
| 25 | 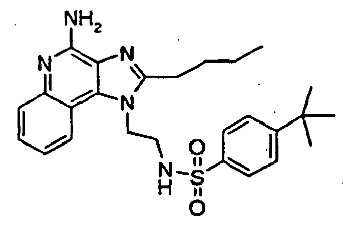 | 480.2 |
| 26 | 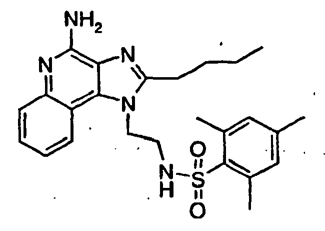 | 466.2 |
| 27 | 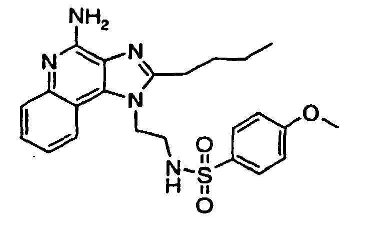 | 454.1 |
| 28 | 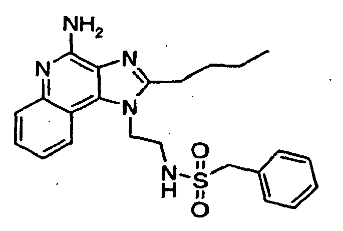 | 438.1 |
| 29 | 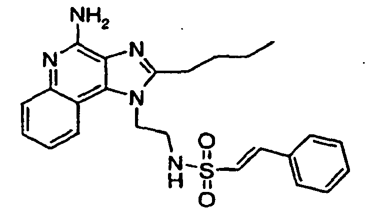 | 450.1 |
| 30 | 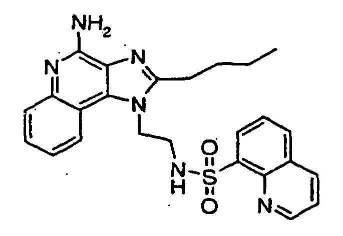 | 475.1 |
| 31 | 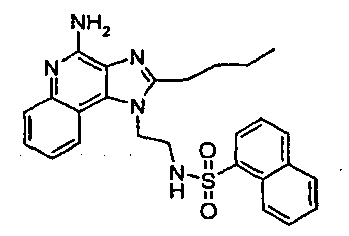 | 474.2 |
| 32 | 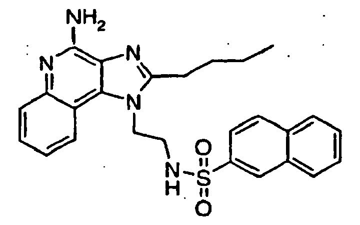 | 474.1 |
| 33 | 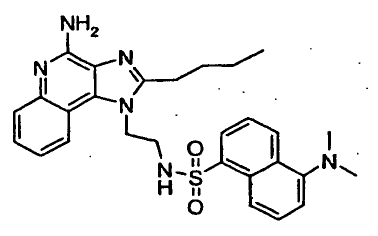 | 517.2 |

### Example 34

### N-[2-(4-Amino-2-butyl-1H-imidazo[4,5-c]quinolin-1-yl)ethyl] methanesulfonamide Trifluoroacetate

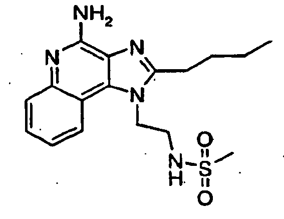

This compound was prepared using the method of Examples 17-33 above except that 1.1 eq of methanesulfonic anhydride was used in place of the sulfonyl chloride.
(Observed Mass = 362.2)

### Example 35

### N-[2-(4-Amino-2-butyl-1H-imidazo[4,5-c]quinolin-1-yl)ethyl] trifluoromethanesulfonamide Trifluoroacetate

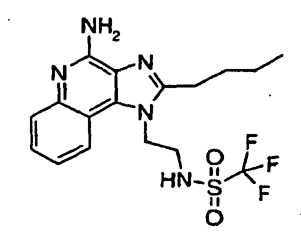

This compound was prepared using the method of Examples 17 - 33 above except that 1.1 eq of trifluoromethanesulfonic anhydride was used in place of the sulfonyl chloride. (Observed Mass =416.1)

### Examples 36 - 48

The compounds shown in the Table below were prepared using the synthetic method described in Reaction Scheme II above.

1-(4-Aminobutyl)-2-butyl-1*H*-imidazo[4,5-*c*]quinolin-4-amine (25 mg) was placed in a 2 dram (7.4 mL) vial. Diisopropylethylamine (14 µL, 1.0 eq), dichloromethane (1 mL) and the sulfonyl chloride (1.0 eq) were added in order. The vial was placed on a shaker for about 30 minutes at which time almost everything was in solution. Some time later a precipitate formed. A small amount of methanol was added and the precipitate dissolved. The reaction mixture was left on the shaker for an additional hour and then it was put on a sonicator for about 0.5 hours. The reaction mixture was analyzed by LC/MS to confirm the formation of the desired product. The solvent was removed and the residue was purified by semi-preparative HPLC (Capcell Pak C18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfonamide.

| Example # | Structure of Free Base | Observed Mass |
|---|---|---|
| 36 | 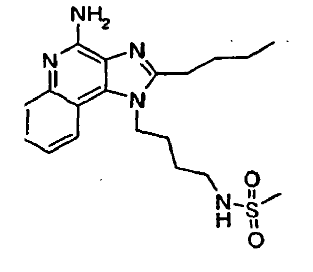 | 390.1 |
| 37 | 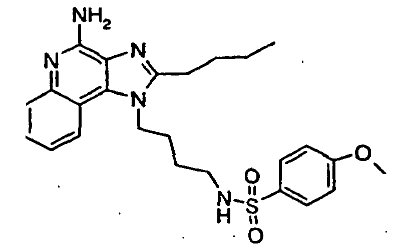 | 482.1 |
| 38 | 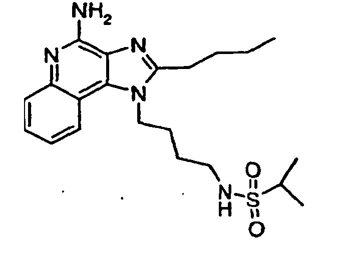 | 418.1 |
| 39 | 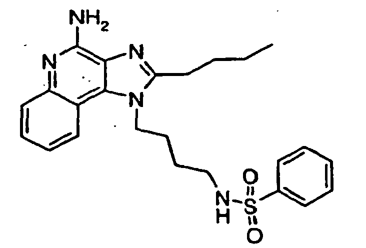 | 452.1 |
| 40 | 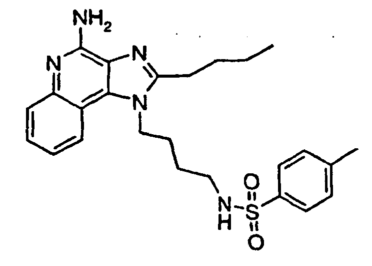 | 466.1 |

### Examples 41 - 52

The compounds shown in the Table below were prepared using the synthetic method described in Reaction Scheme II above.

1-(4-Aminobutyl)-2-butyl-1*H*-imidazo[4,5-*c*]quinolin-4-amine (25 mg) was placed in a 2 dram (7.4 mL) vial. Diisopropylethylamine (14 µL, 1.0 eq), dichloromethane (1 mL) and the sulfonyl chloride (1.0 eq) were added in order. The vial was placed on a sonicator at ambient temperature for about 60 minutes. The reaction mixture was analyzed by LC/MS to confirm the formation of the desired product. The solvent was removed and the residue was purified by semi-preparative HPLC (Capcell Pak C18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfonamide.

| Example # | Structure of Free Base | Observed Mass |
|---|---|---|
| 41 | 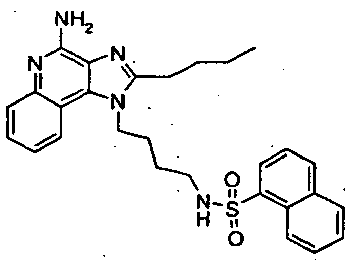 | 502.1 |
| 42 | 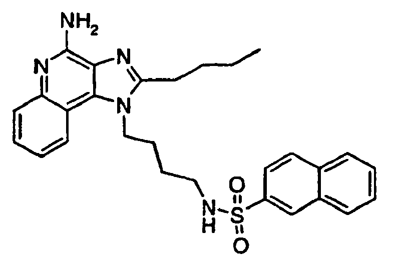 | 502.1 |
| 43 | 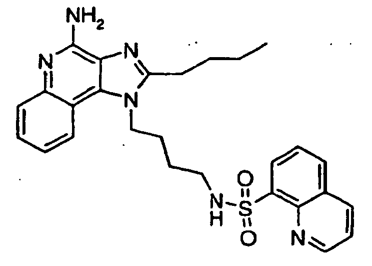 | 503.2 |
| 44 | 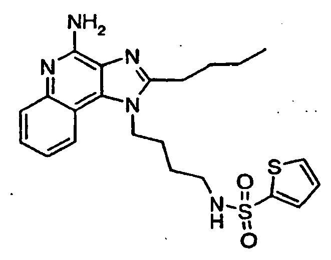 | 458.1 |
| 45 | 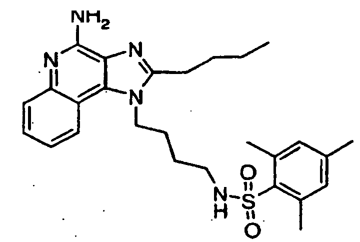 | 494.2 |
| 46 | 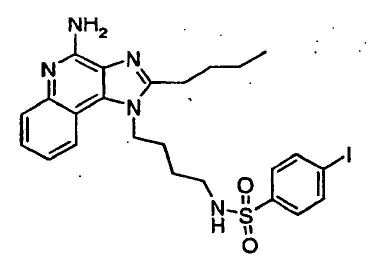 | 578.2 |
| 47 | 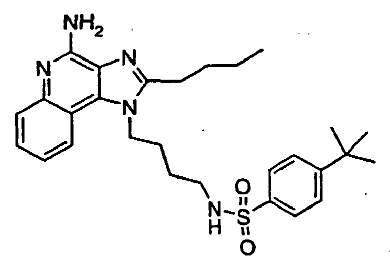 | 508.3 |
| 48 | 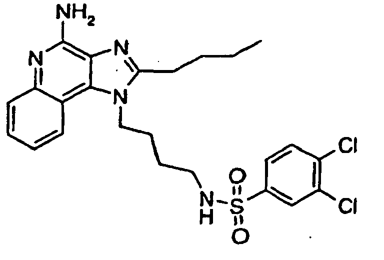 | 520.1 |
| 49 | 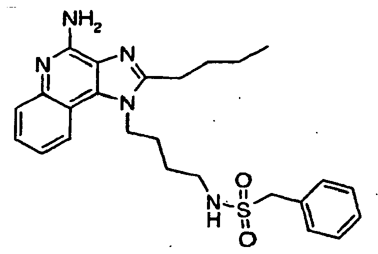 | 466.2 |
| 50 | 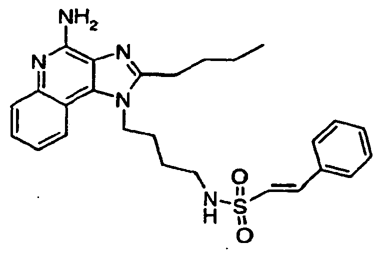 | 478.2 |
| 51 | 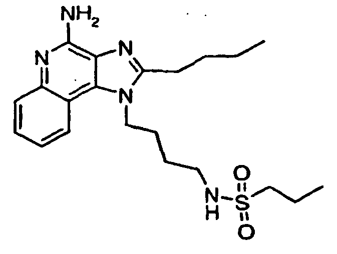 | 418.2 |
| 52 | 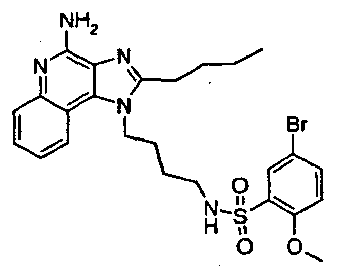 | 560.1 |

### Example 53

### N-[4-(4-Amino-2-butyl-1H-imidazo[4,5-c]quinolin-1-yl)butyl] trifluoromethanesulfonamide Trifluoroacetate

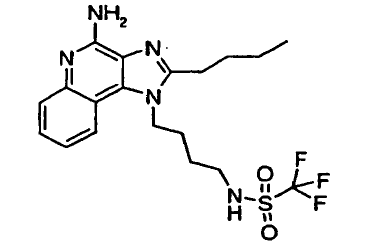

This compound was prepared using the method of Examples 41-52 above except that 1.0 eq of trifluoromethanesulfonic anhydride was used in place of the sulfonyl chloride. (Observed Mass = 444.1)

### Examples 54 - 71

The compounds shown in the Table below were prepared using the synthetic method described in Reaction Scheme IV above.

### Part A

A catalytic amount of platinum (IV) oxide was added to a solution of 1-(4-aminobutyl)-1*H*-imidazo[4,5-*c*]quinolin-4-amine (2.75 g, 10.8 mmol) in trifluoroacetic acid (150 mL). The reaction mixture was placed under a hydrogen atmosphere at 50 psi (3.44 X 10⁵ Pa). After 1 week analysis by mass spectroscopy indicated the presence of both starting material and the tetrahydro product. Fresh catalyst was added to the reaction mixture and hydrogenation was continued at 50 psi (3.44 X 10⁵ Pa). After 2 weeks the reaction mixture was filtered to remove the catalyst. The filtrate was concentrated under vacuum. The residue was dissolved in 1N hydrochloric acid (120 mL) and the solution was stirred at ambient temperature for 1 hour. The solution was made basic (pH 10) by the addition of 50% sodium hydroxide and then extracted with dichloromethane (5 X 100 mL). The extracts were combined and concentrated under vacuum to provide 2.08 g of 1-(4-aminobutyl)-6,7,8,9-tetrahydro-1*H*-imidazo[4,5-*c*]quinolin-4-amine as a white solid.

### Part B

1-(4-Aminobutyl)-6,7,8,9-tetrahydro-1*H*-imidazo[4,5-*c*]quinolin-4-amine (25 mg) was placed in a 2 dram (7.4 mL) vial. Diisopropylethylamine (11 µL, 1.2 eq), dichloromethane (1 mL) and the sulfonyl chloride (1.1 eq) were added in order. The vial was placed on a shaker for about 6 hours. The reaction mixture was allowed to stand at ambient temperature overnight and was analyzed by LC/MS to confirm the formation of the desired product. The solvent was removed and the residue was purified by semi-preparative HPLC (Capcell Pak C18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfonamide.

| Example # | Structure of Free Base | Observed Mass |
|---|---|---|
| 54 | 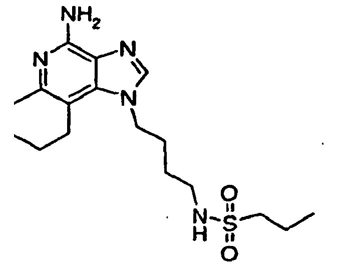 | 366.2 |
| 55 | 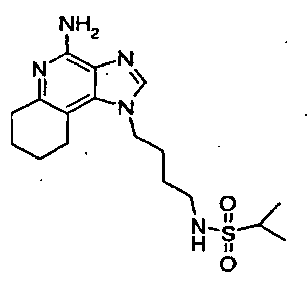 | 366.1 |
| 56 | 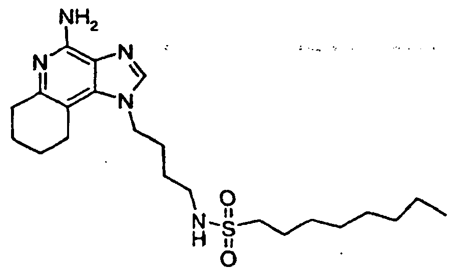 | 436.2 |
| 57 | 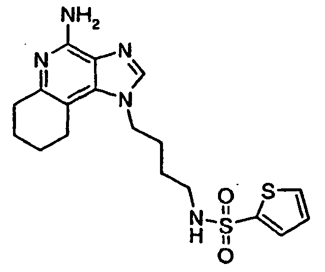 | 406.1 |
| 58 | 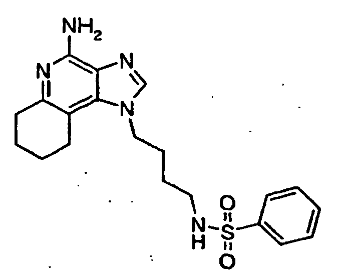 | 400.1 |
| 59 | 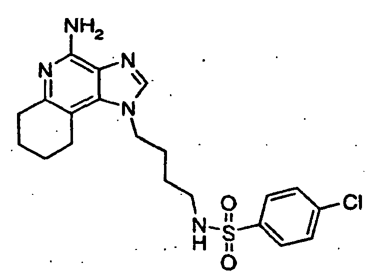 | 434.0 |
| 60 | 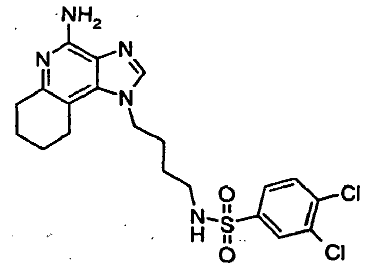 | 468.0 |
| 61 | 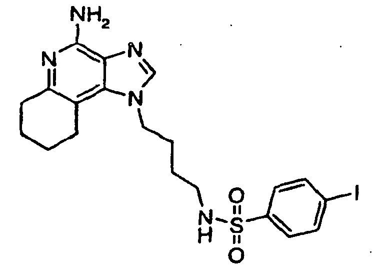 | 526.0 |
| 62 | 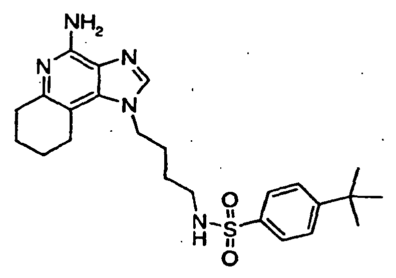 | 456.1 |
| 63 | 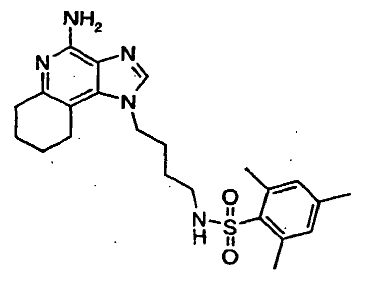 | 442 |
| 64 | 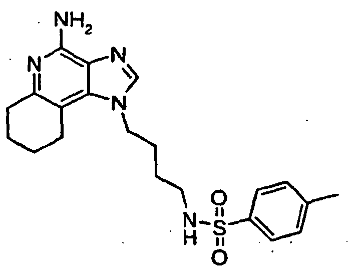 | 414 |
| 65 | 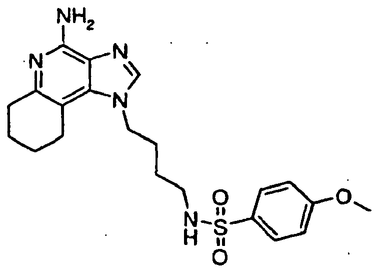 | 430 |
| 66 | 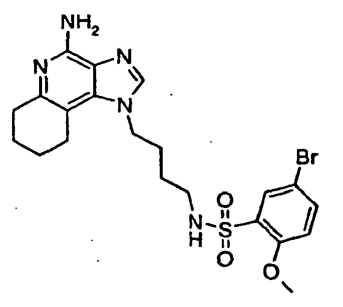 | 508.0 |
| 67 | 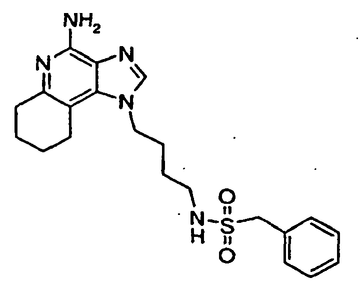 | 414.1 |
| 68 | 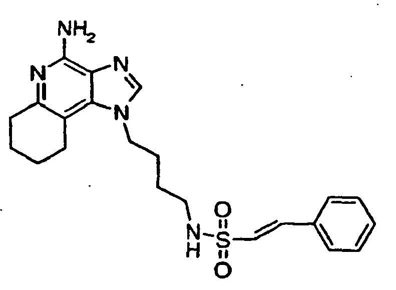 | 426.1 |
| 69 | 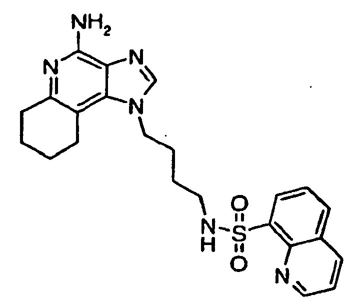 | 451.1 |
| 70 | 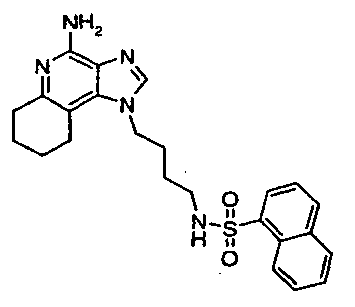 | 450.1 |
| 71 | 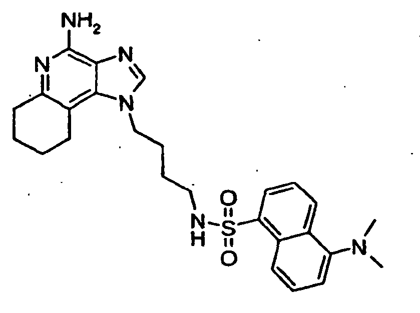 | 493.1 |

### Example 72

### N-(4-(4-Amino-6,7,8,9-tetrahydro-1H-imidazo[4,5-c]quinolin-1-yl)butyl] methanesulfonamide Trifluoroacetate

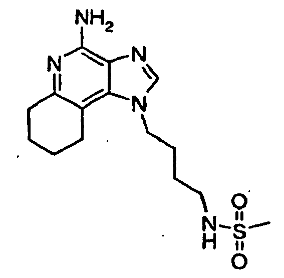

This compound was prepared using the method of Examples 54 - 71 above except that 1.1 eq of methanesulfonic anhydride was used in place of the sulfonyl chloride. (Observed Mass = 338.2)

### Examples 73 - 201

The compounds in the table below were prepared according the synthetic method of Reaction Scheme II above using the following general method.

The 1*H*-imidazo[4,5-*c*]quinolin-4-amine (50 mg) was placed in a 2 dram (7.4 mL) vial. Diisopropylethylamine (1.2 eq) in dichloromethane (~1 mL) was added. A solution containing the sulfonyl chloride (1.1 eq) in dichloromethane (~1 mL) was added. The vial was placed on a shaker for about 2-16 (usually 2) hours at ambient temperature. The reaction mixture was analyzed by LC/MS to confirm the formation of the desired product. The solvent was removed and the residue was purified by semi-preparative HPLC (Capcell Pak C 18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfonamide.

| Example # | Structure of the Free Base | APCI-MS m/e |
|---|---|---|
| 73 | 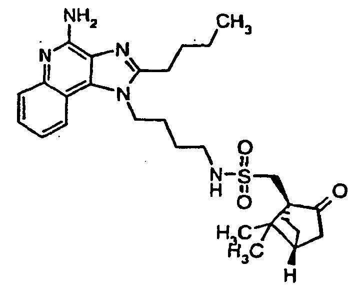 | 526.2 |
| 74 | 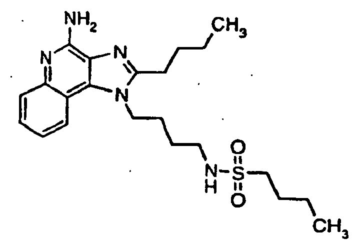 | 432.2 |
| 75 | 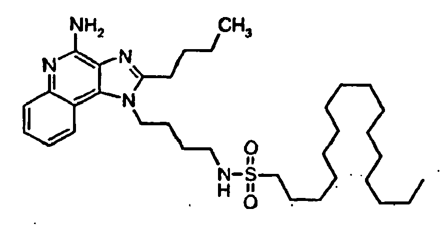 | 600.3 |
| 76 | 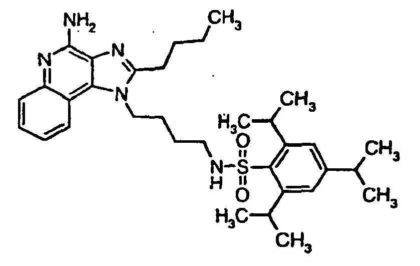 | 578.2 |
| 77 | 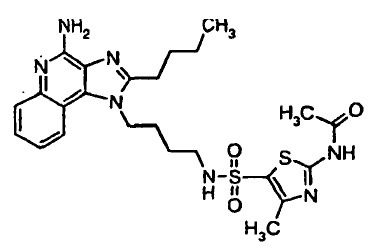 | 530.1 |
| 78 | 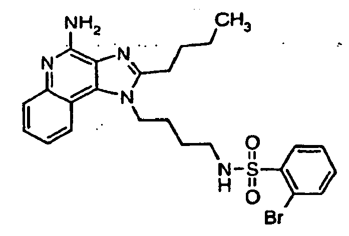 | 530, 532.0 |
| 79 | 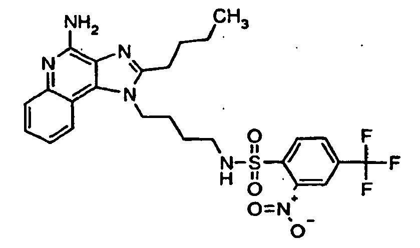 | 565.0 |
| 80 | 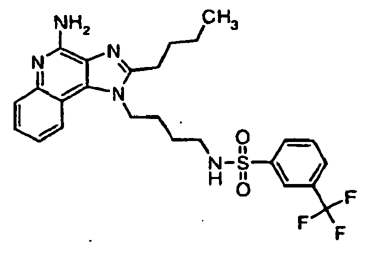 | 520.1 |
| 81 | 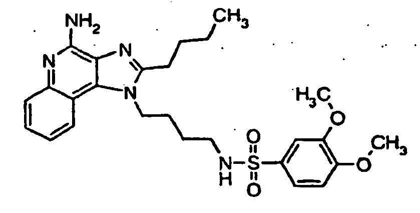 | 512.1 |
| 82 | 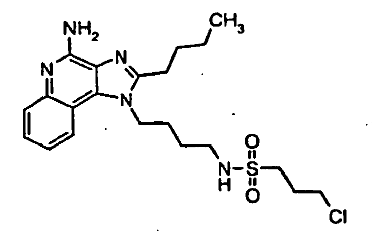 | 452.1 |
| 83 | 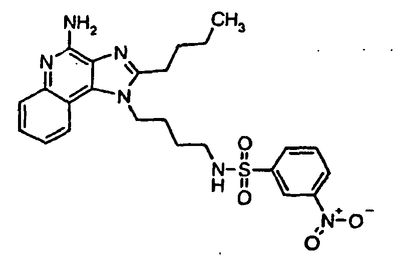 | 497.1 |
| 84 | 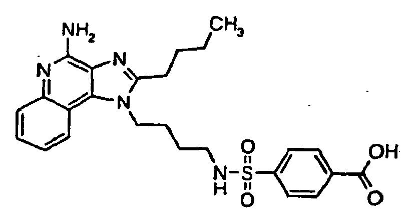 | 496.1 |
| 85 | 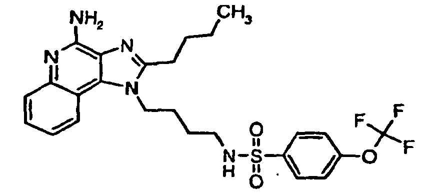 | 536.1 |
| 86 | 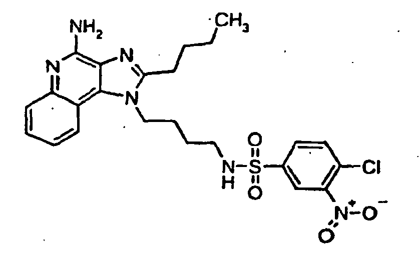 | 531.0, 533.0 |
| 87 | 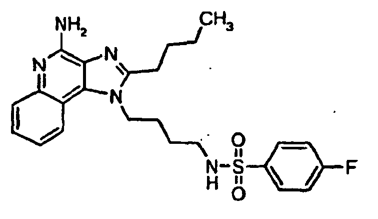 | 470.1 |
| 88 | 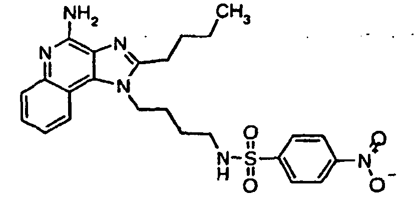 | 497.1 |
| 89 | 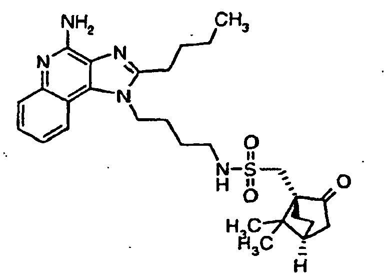 | 526.2 |
| 90 | 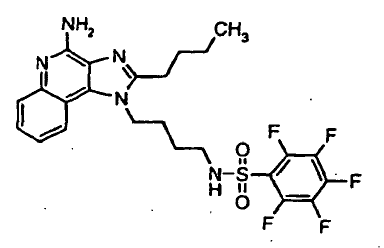 | 542.0 |
| 91 | 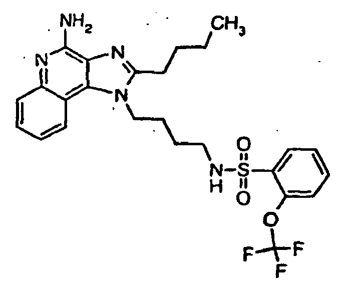 | 536. 1 |
| 92 | 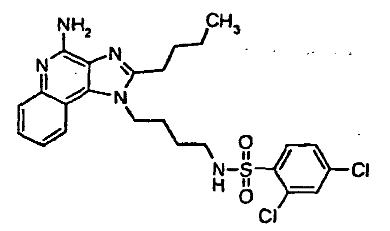 | 520.0, 522.0 |
| 93 | 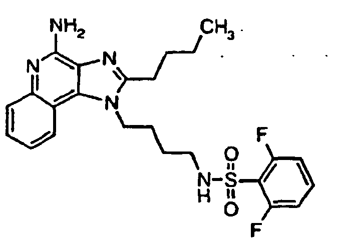 | 488.1 |
| 94 | 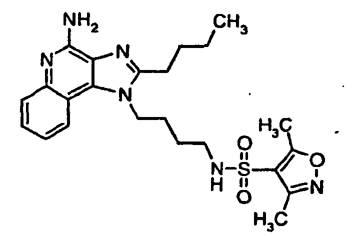 | 471.1 |
| 95 | 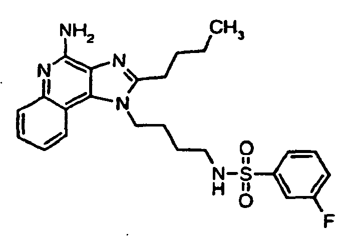 | 470.1 |
| 96 | 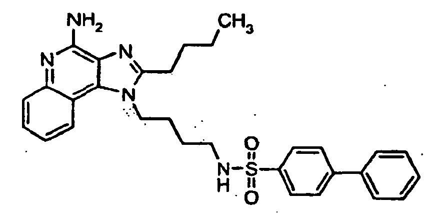 | 528.1 |
| 97 | 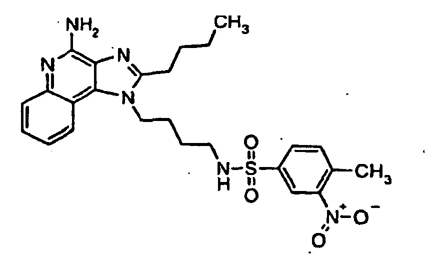 | 511.1 |
| 98 | 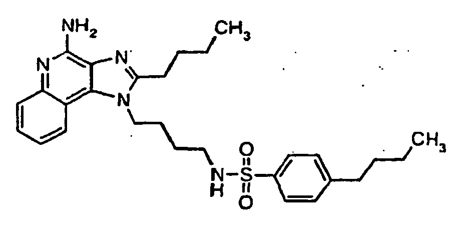 | 508.1 |
| 99 | 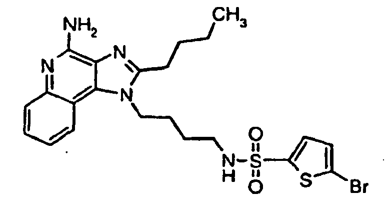 | 537.9 |
| 100 | 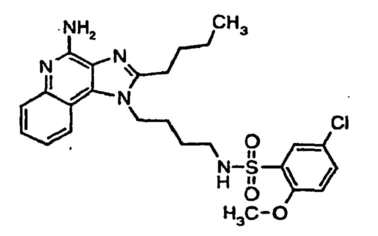 | 516.0, 518.0 |
| 101 | 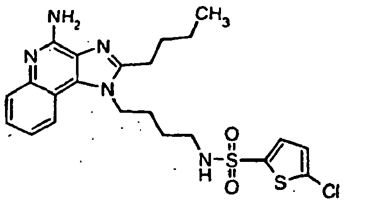 | 492.0, 494.0 |
| 102 | 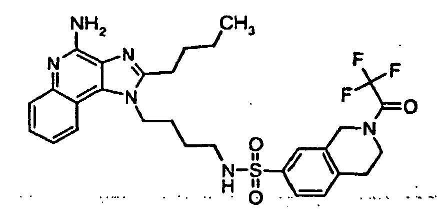 | 603.1 |
| 103 | 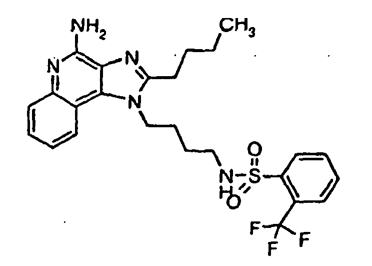 | 520.1 |
| 104 | 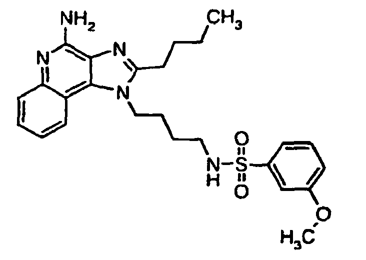 | 482.1 |
| 105 | 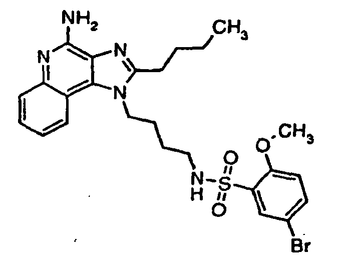 | 560.0, 562 |
| 106 | 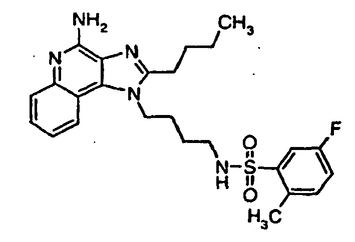 | 484.1 |
| 107 | 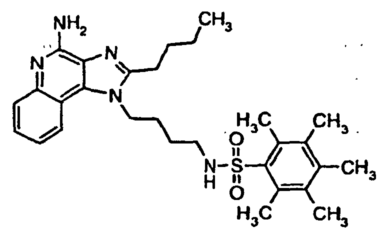 | 522.1 |
| 108 | 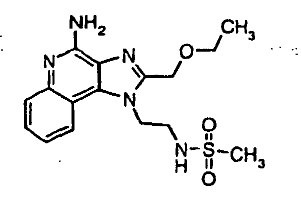 | 364.1 |
| 109 | 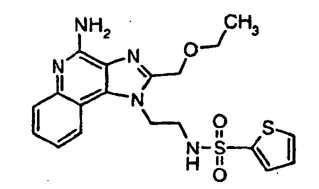 | 432.0 |
| 110 | 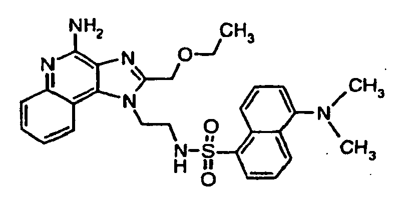 | 519.1 |
| 111 | 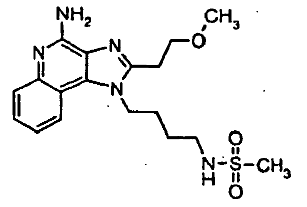 | 392.2 |
| 112 | 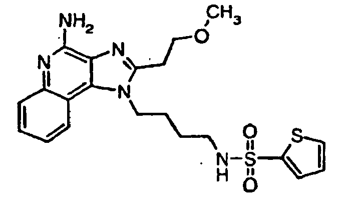 | 460.1 |
| 113 | 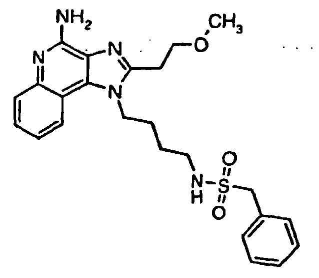 | 468.2 |
| 114 | 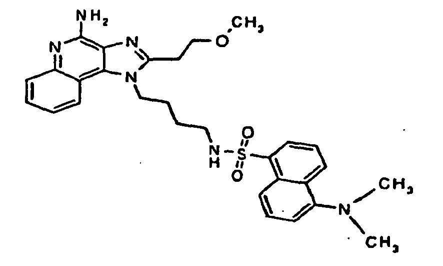 | 547.3 |
| 115 | 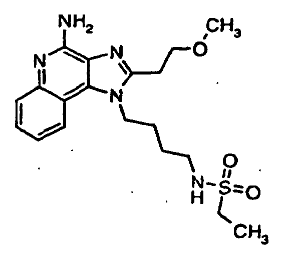 | 406.1 |
| 116 | 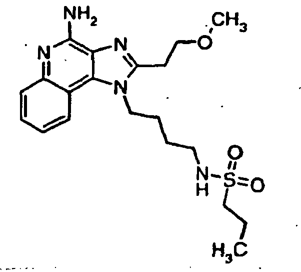 | 420.1 |
| 117 | 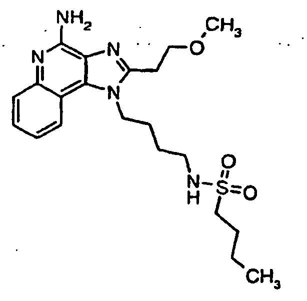 | 434.1 |
| 118 | 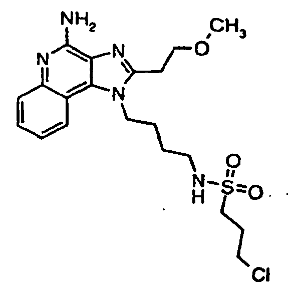 | 454.1 |
| 119 | 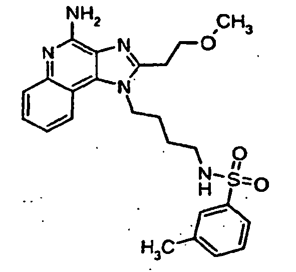 | 468.1 |
| 120 | 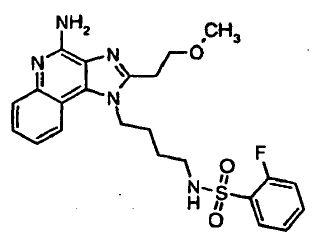 | 472.1 |
| 121 | 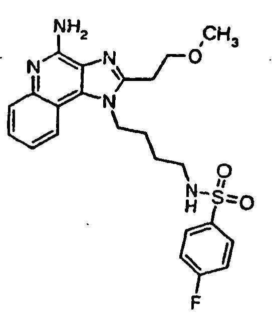 | 472.1 |
| 122 | 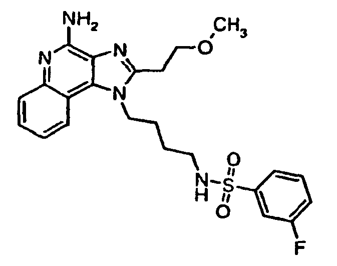 | 472.1 |
| 123 | 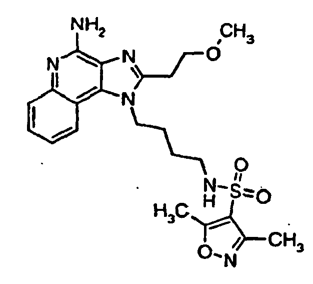 | 473.1 |
| 124 | 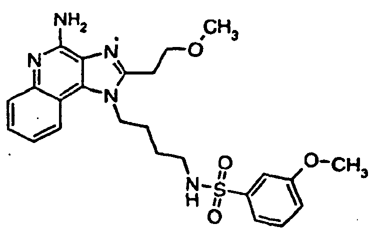 | 484.1 |
| 125 | 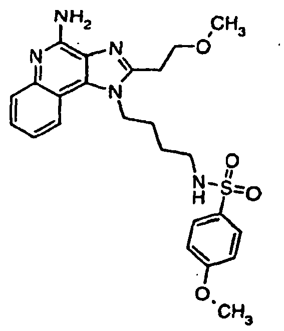 | 484.1 |
| 126 | 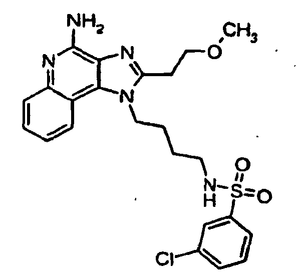 | 488.1 |
| 127 | 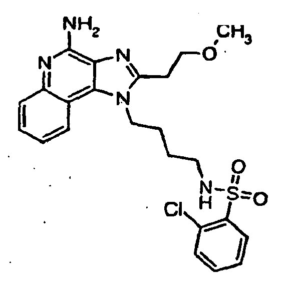 | 488.1 |
| 128 | 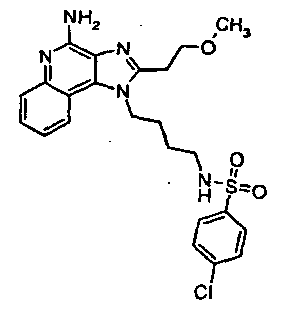 | 488.0 |
| 129 | 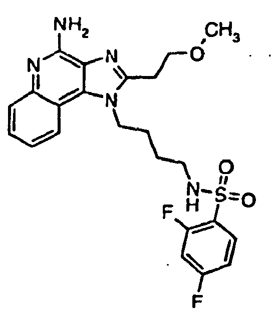 | 490.1 |
| 130 | 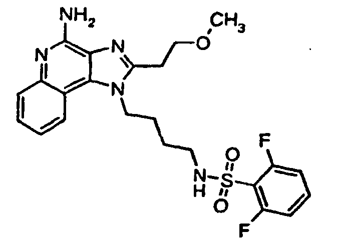 | 490.1 |
| 131 | 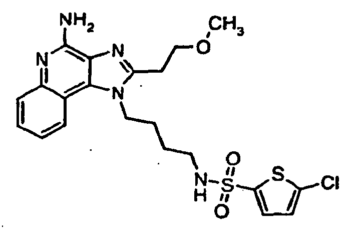 | 494.0 |
| 132 | 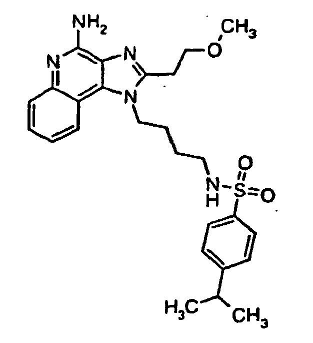 | 496.2 |
| 133 | 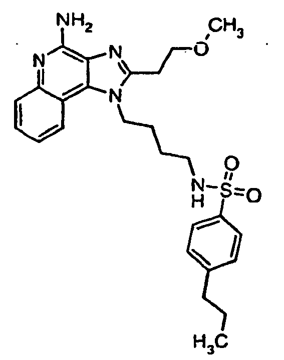 | 496.1 |
| 134 | 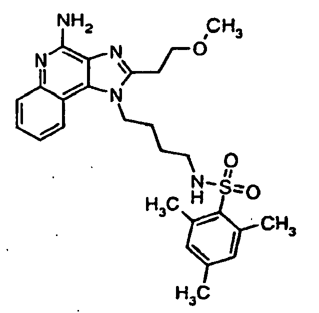 | 496.2 |
| 135 | 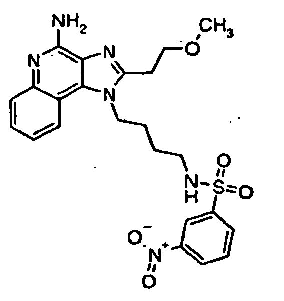 | 499.1 |
| 136 | 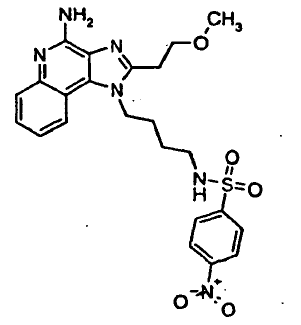 | 499.1 |
| 137 | 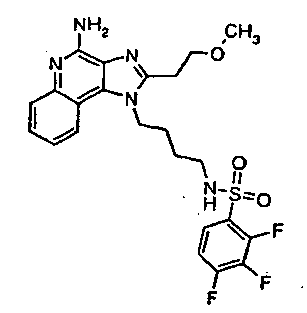 | 508.1 |
| 138 | 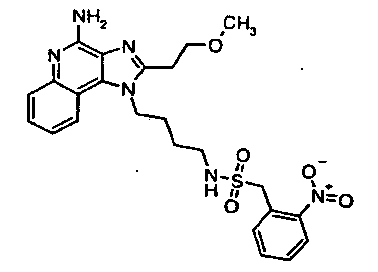 | 513.1 |
| 139 | 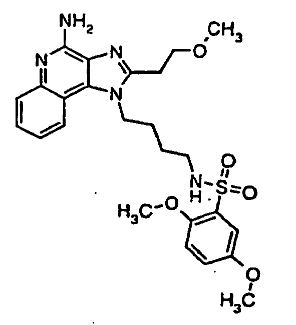 | 514.1 |
| 140 | 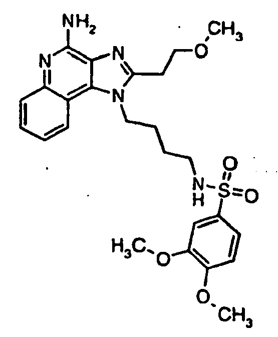 | 514.1 |
| 141 | 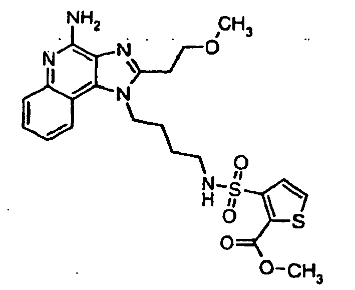 | 518.0 |
| 142 | 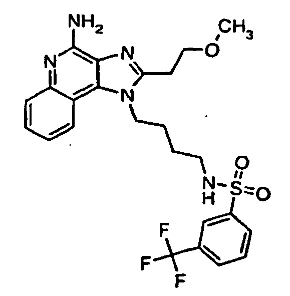 | 522.1 |
| 143 | 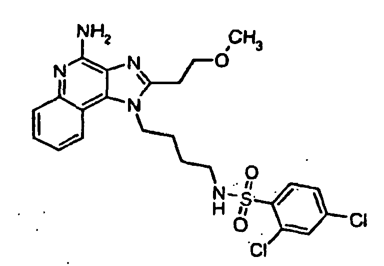 | 522.0, 524.0 |
| 144 | 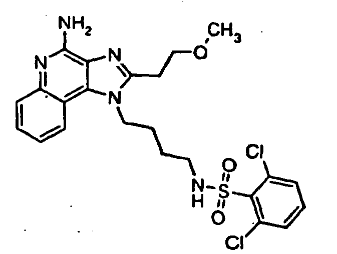 | 522.0, 524.0 |
| 145 | 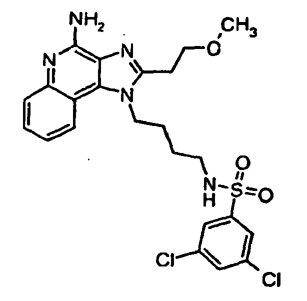 | 522.0, 524.0 |
| 146 | 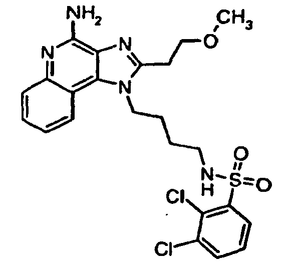 | 522.0, 524.0 |
| 147 | 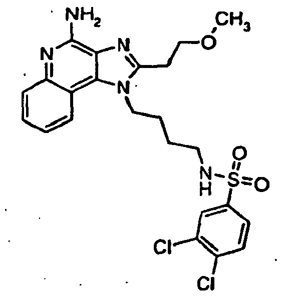 | 522.0, 524.0 |
| 148 | 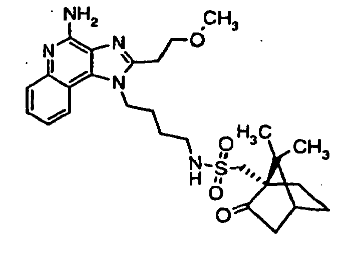 | 528.2 |
| 149 | 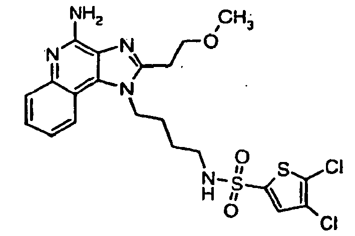 | 528.0, 530.0 |
| 150 | 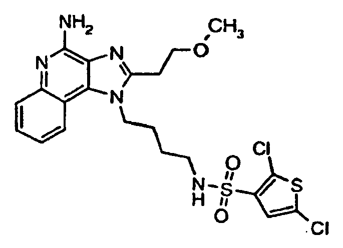 | 528.0, 530.0 |
| 151 | 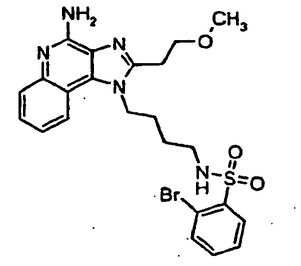 | 532, 534.0 |
| 152 | 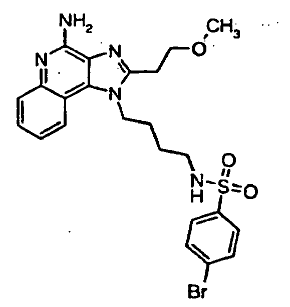 | 532, 534.0 |
| 153 | 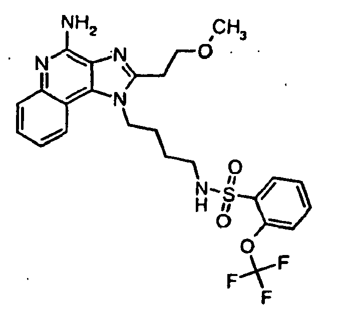 | 538.1 |
| 154 | 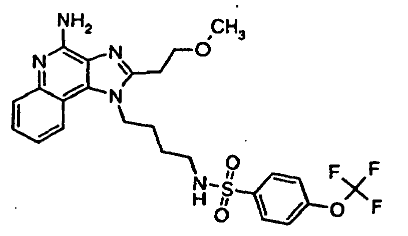 | 538.1 |
| 155 | 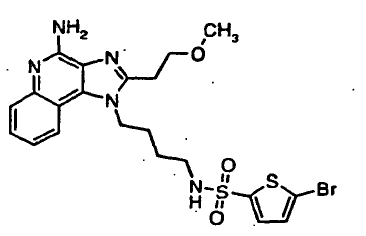 | 538, 540.0 |
| 156 | 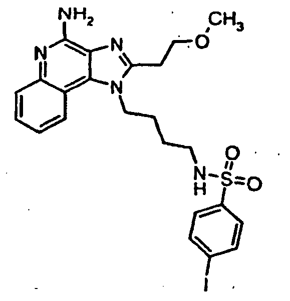 | 580.0 |
| 157 | 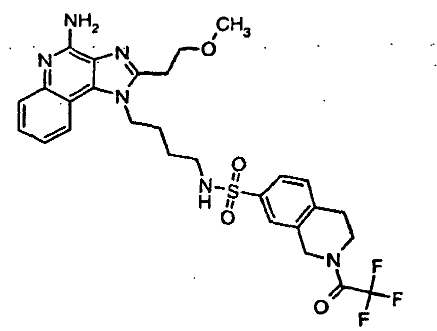 | 605.1 |
| 158 | 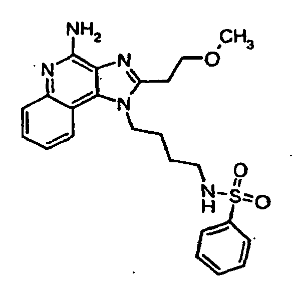 | 454.2 |
| 159 | 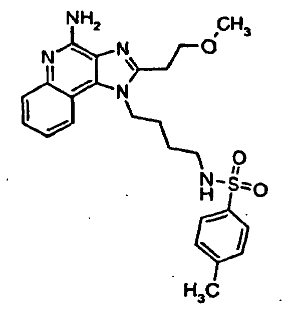 | 468.2 |
| 160 | 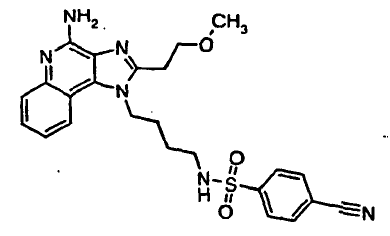 | 479.2 |
| 161 | 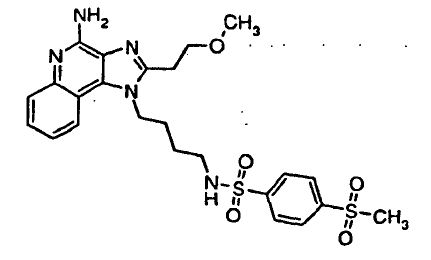 | 532.2 |
| 162 | 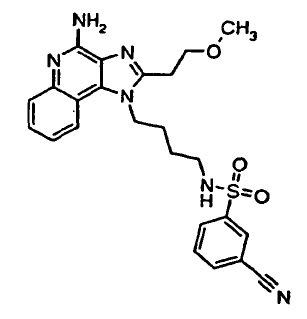 | 479.1 |
| 163 | 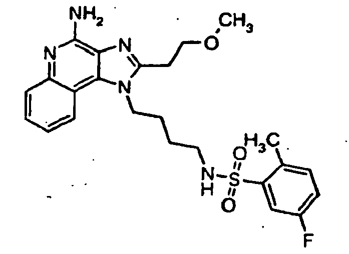 | 486.1 |
| 164 | 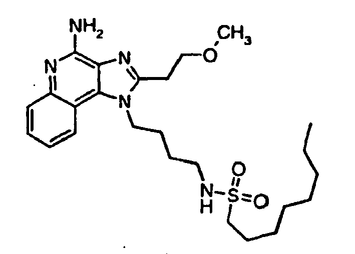 | 490.2 |
| 165 | 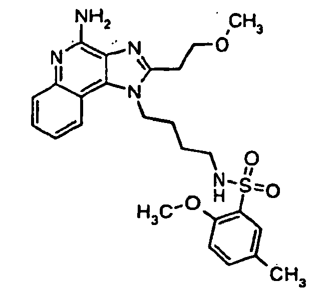 | 498.1 |
| 166 | 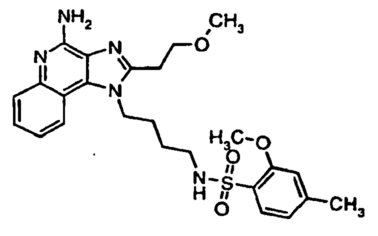 | 498.1 |
| 167 | 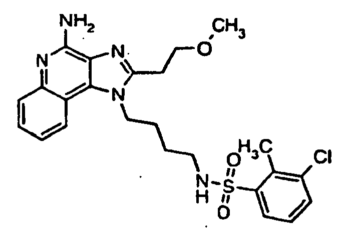 | 502.1 |
| 168 | 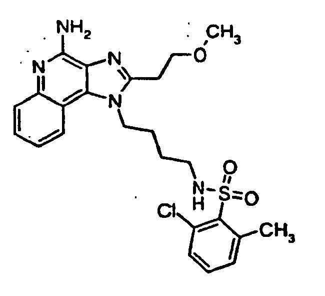 | 502.1 |
| 169 | 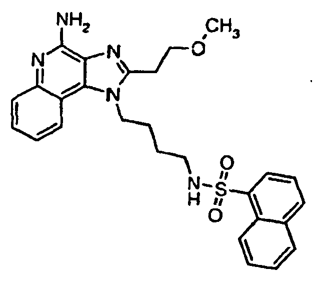 | 504.2 |
| 170 | 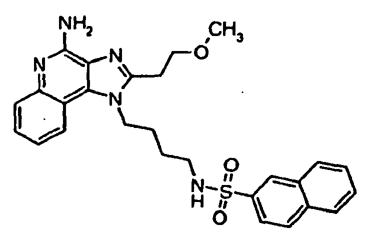 | 504.1 |
| 171 | 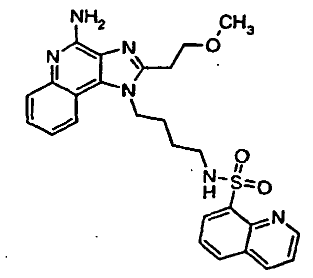 | 505.2 |
| 172 | 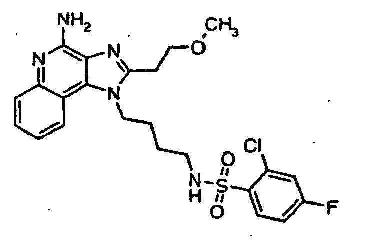 | 506.1 |
| 173 | 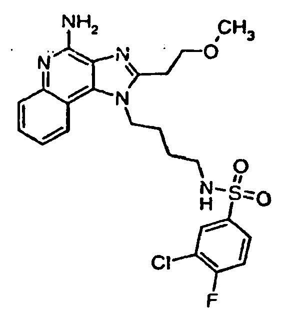 | 506.2 |
| 174 | 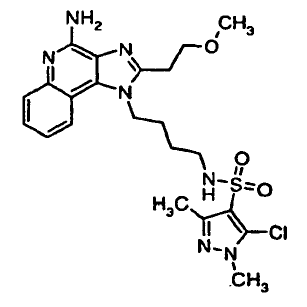 | 506.2 |
| 175 | 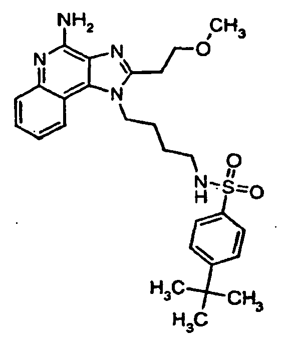 | 510.3 |
| 176 | 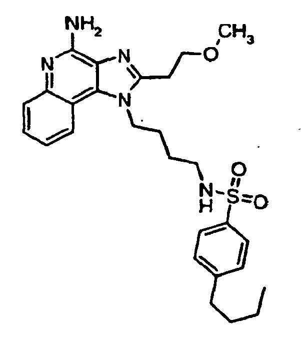 | 510.2 |
| 177 | 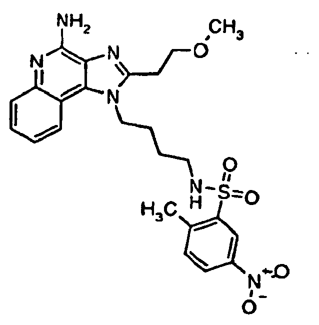 | 513.2 |
| 178 | 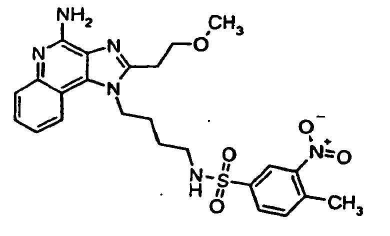 | 513.2 |
| 179 | 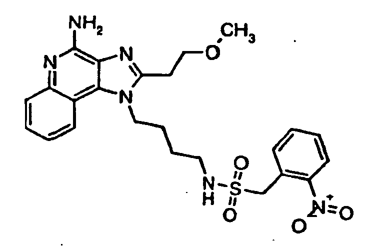 | 513.2 |
| 180 | 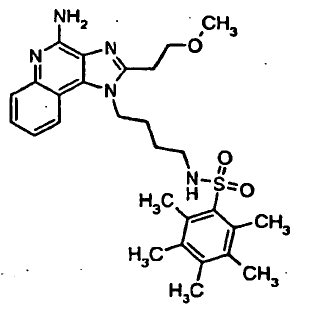 | 524.2 |
| 181 | 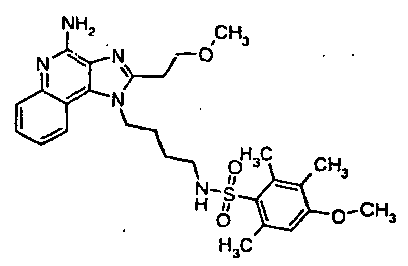 | 526.2 |
| 182 | 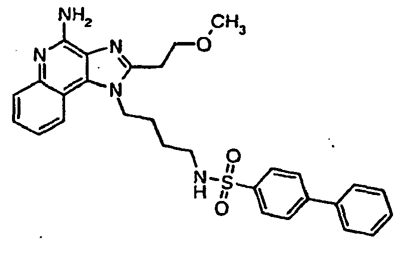 | 530.2 |
| 183 | 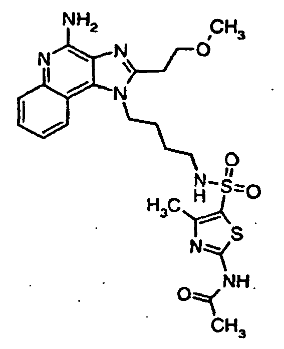 | 532.2 |
| 184 | 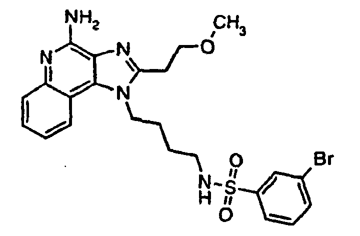 | 534.1 |
| 185 | 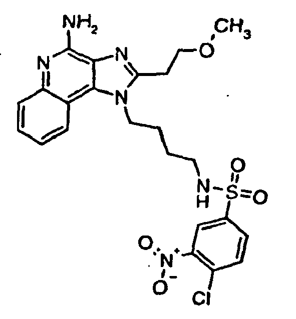 | 533.1 |
| 186 | 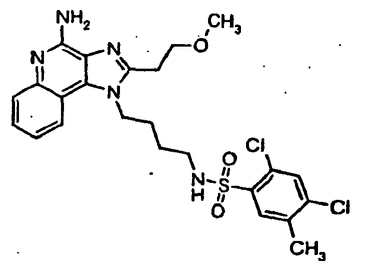 | 536.1, 538.1 |
| 187 | 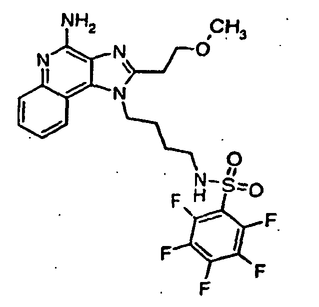 | 544.1 |
| 188 | 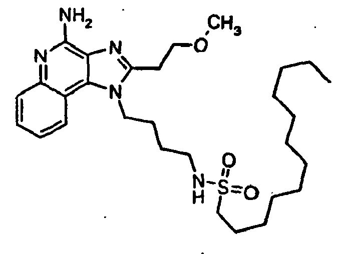 | 546.3 |
| 189 | 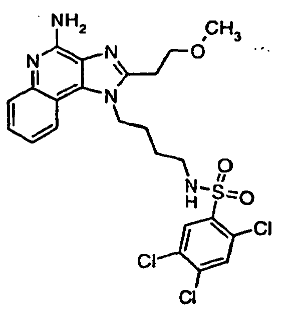 | 556, 558.1 |
| 190 | 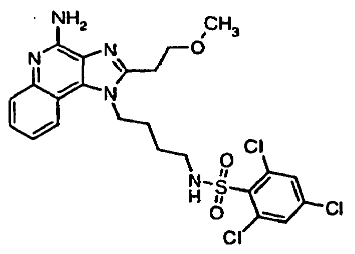 | 556, 558.1 |
| 191 | 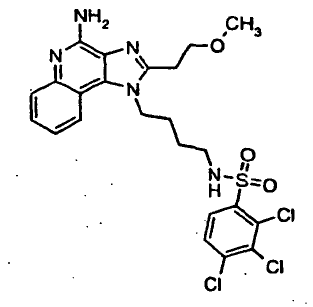 | 556, 558.1 |
| 192 | 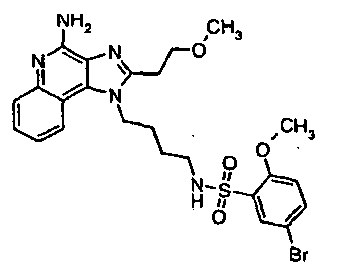 | 562, 564.1 |
| 193 | 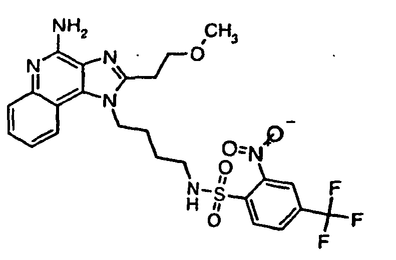 | 567.2 |
| 194 | 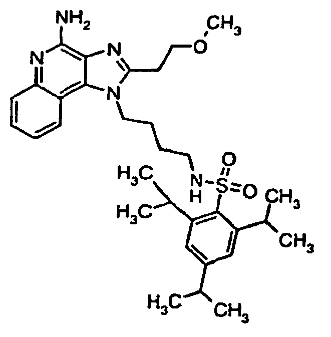 | 580.3 |
| 195 | 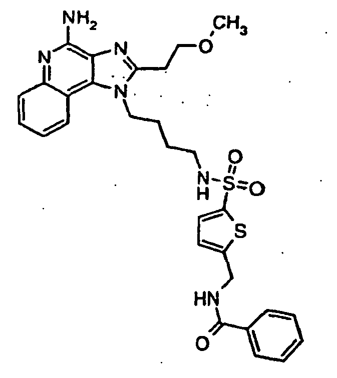 | 593.2 |
| 196 | 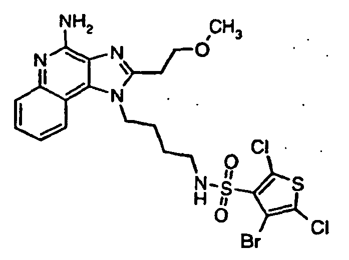 | 606.0, 608.0, 609.9 |
| 197 | 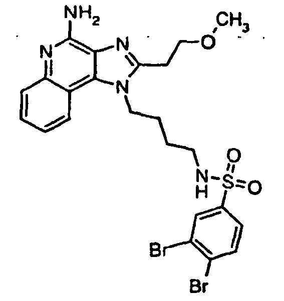 | 610.0, 612.0, 614.0 |
| 198 | 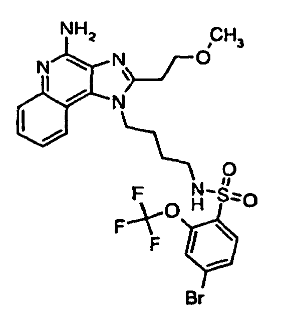 | 616, 618.1 |
| 199 | 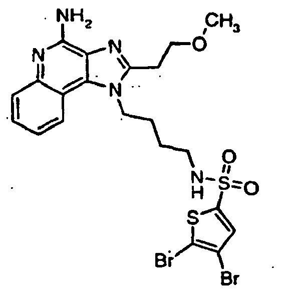 | 616.0, 617.9, 620.0 |
| 200 | 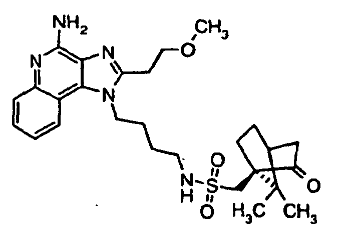 | 528.3 |
| 201 | 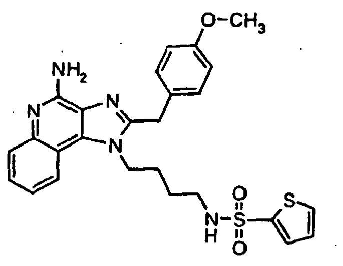 | 522.2 |

### Examples 202 - 213

The examples in the table below were prepared according to the synthetic method of Reaction Scheme VI above.

### Part A

The tetrahydroquinoline amine starting materials were prepared as follows. A catalytic amount of platinum (IV) oxide was added to a solution of 1-(4-aminobutyl)-2-butyl-1*H*-imidazo[4,5-*c*]quinolin-4-amine (2.2 g, 7.06 mmol) in trifluoroacetic acid (200 mL). The reaction mixture was hydrogenated at 50 psi (3.44 X 10⁵ Pa) on a Parr apparatus for 6 days.. The reaction mixture was filtered to remove the catalyst and the filtrate was concentrated under vacuum. The residue was combined with 1 N hydrochloric acid (100 mL) and heated on a steam bath for 2 hours. The mixture was cooled, made basic with ammonium hydroxide and then extracted with dichloromethane. The extract was concentrated under vacuum to provide of 1-(4-aminobutyl)-2-butyl-6,7,8,9-tetrahydro -1*H*-imidazo[4,5-*c*]quinolin-4-amine as a solid, m.p. 63-67°C

A catalytic amount of platinum (IV) oxide was added to a solution of 1-(4-aminobutyl)-2-methoxyethyl-1*H*-imidazo[4,5-*c*]quinolin-4-amine (7.7 g, 24.5 mmol) in trifluoroacetic acid (250 mL). The reaction mixture was hydrogenated at 50 psi (3.44 X 10⁵ Pa) on a Parr apparatus. The progress of the reaction was monitored by LC/MS. Additional catalyst was added 7, 11, and 17 days after the start of the reaction. After 25 days the reaction was complete. The reaction mixture was filtered through a layer of Celite® filter aid to remove the catalyst and the filtrate was concentrated under vacuum. The residue was combined with 1 N hydrochloric acid (100 mL) and stirred overnight. The mixture was made basic (pH = 11) with ammonium hydroxide and then extracted with dichloromethane (3 X 300 mL). The extracts were combined and concentrated under vacuum to provide 3.5 g of 1-(4-aminobutyl)-6,7,8,9-tetrahydro-2-methoxyethyl-1*H-*imidazo[4,5-c]quinolin-4-amine as a solid.

### Part B

The tetrahydroimidazoquinoline amines from Part A were reacted with the appropriate sulfonyl chloride using the method of Examples 73 - 201 above to provide the desired sulfonamide.

| Example # | Structure of the Free Base | APCI-MS m/e |
|---|---|---|
| 202 | 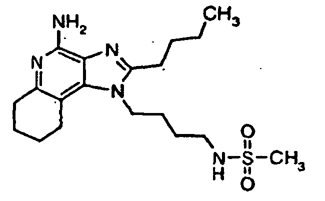 | 394.20 |
| 203 | 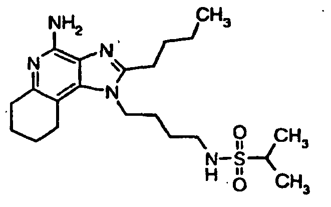 | 422.1 |
| 204 | 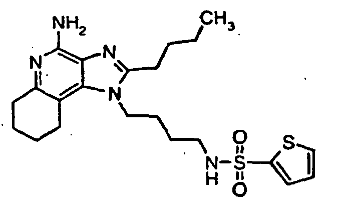 | 462.1 |
| 205 | 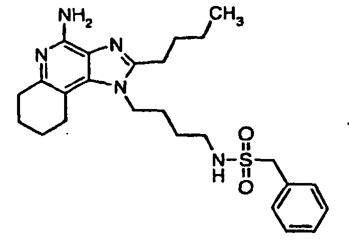 | 470.1 |
| 206 | 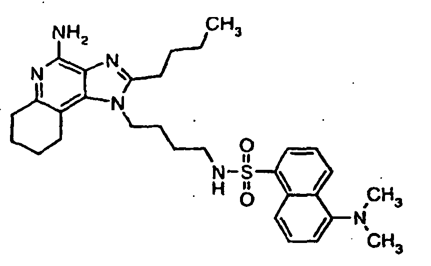 | 549.2 |
| 207 | 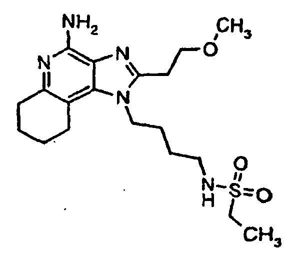 | 410.2 |
| 208 | 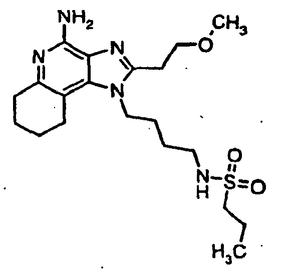 | 424.2 |
| 209 | 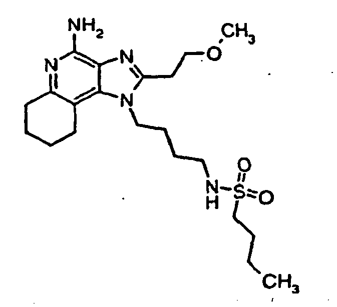 | 438.2 |
| 210 | 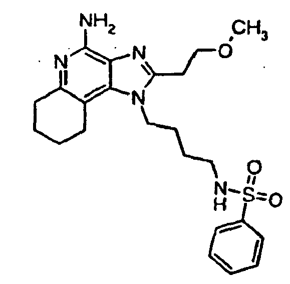 | 458.1 |
| 211 | 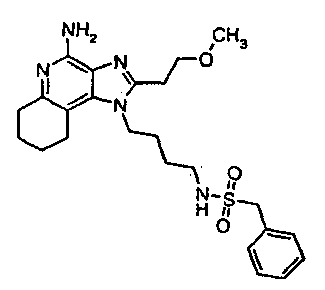 | 472.2 |
| 212 | 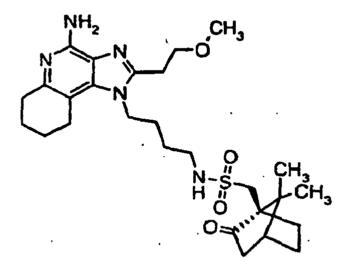 | 532.2 |
| 213 | 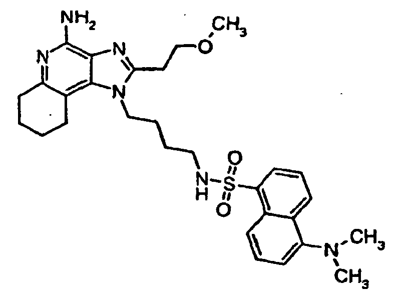 | 551.2 |

### Example 214

### N-[4-(4-Amino-6,7,8,9-tetrahydro-2-(2-methoxyethyl)-1H-imidazo[4,5-c]quinolin-1-yl)butyl]methanesulfonamide Trifluoroacetat

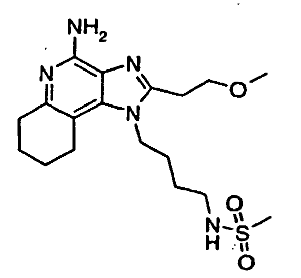

This compound was prepare using the method of Examples 202 - 213 above except that methanesulfonic anhydride was used in place of the sulfonyl chloride.

### Example 215

### N-[4-(4-Amino-2-(2-methoxyethyl)-1H-imidazo[4,5-c]quinolin-1-yl)butyl]-N-methyl-3,5-dimethylisooxazolo-4-sulfonamide Trifluoroacetate

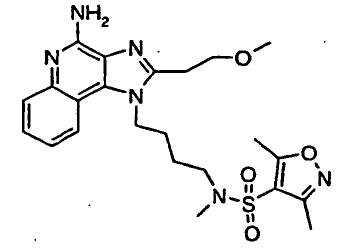

### Part A

Using the general method of Example DC001, 1-(4-aminobutyl)-2-(2-methoxyethyl)-1*H*-imidazo[4,5-*c*]quinolin-4-amine was reacted with 3,5-dimethyloxazole-4-sulfonyl chloride to provide N-[4-(4-amino -2-(2-methoxyethyl)-1*H*-imidazo[4,5-*c*]quinolin-1-yl)butyl] -3,5-dimethylisooxazolo-4-sulfonamide trifluoroacetate.

### Part B

Sodium hydride (5.8 mg) was added to a solution of the material from Part A (25.4 mg) in dimethylformamide. Iodomethane (3.2 µL) was added and the reaction mixture was shaken at ambient temperature for 2 hours.. The reaction mixture was analyzed by LC/MS to confirm the formation of the desired product. The solvent was removed and the residue was purified by semi-preparative HPLC (Capcell Pak C18 column, 35 mm x 20 mm, 5 micron particle size, 20 mL/min, gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized. The lyophilized material was purified a second time by semi-preparative HPLC using the same conditions except that the gradient elution from 5-95% B was run for 60 minutes instead of 10 minutes. The semi-prep HPLC fractions were analyzed by LC-APCI/MS and the appropriate fractions were combined and lyophilized to provide the trifluoroacetate salt of the desired amide.

### Example 216

### N-[4-(4-Amino-2-(2-methoxyethyl)-1H-imidazo[4,5-c]quinolin-1-yl)butyl]-N-methyltrifluoromethanesulfonamide Trifluoroacetate

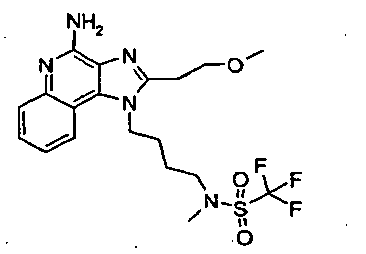

This compound was prepared using the general method of Example 215 above, except that trifluoromethanesulfonic anhydride was used in place of the sulfonyl chloride in Part A.

### Examples 217 - 221

The examples in the table below were prepare using the following general method. The 1*H*-imidazo[4,5-c]quinolin-4-amine or the 6,7,8,9-tetrahydro-1*H*-imidazo[4,5-clquinolin-4-amine (50 mg) was placed in a 2 dram (7.4 mL) vial. Dichloromethane (2 mL) and diisopropylethylamine (1.2 eq) were added. Dimethylsulfamoyl chloride (1.1 eq) was added. The vial was placed on a shaker for about 2 - 4 hours at ambient temperature. The reaction mixture was analyzed by LC/MS to confirm the formation of the desired product. The solvent was removed and the residue was purified by semi-preparative HPLC (Capcell Pak C18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfamide.

| Example # | Structure of the Free Base | I APCI-MS m/e |
|---|---|---|
| 217 | 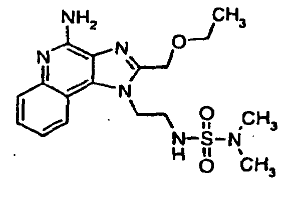 | 393.1 |
| 218 | 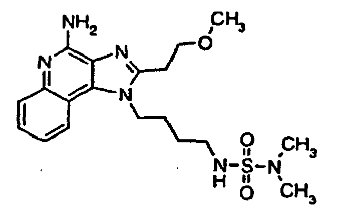 | 421.2 |
| 219 | 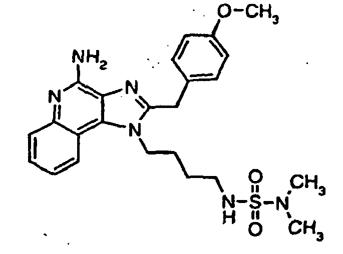 | 483.3 |
| 220 | 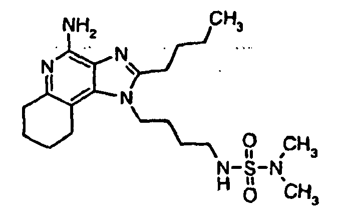 | 423.2 |
| 221 | 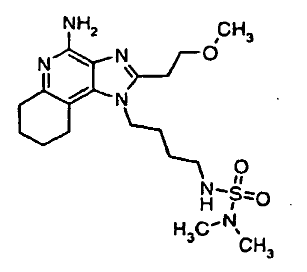 | 425.1 |

### Examples 222 - 228

The examples in the table below were prepared according to the synthetic method shown in Reaction Scheme V above.

1-(4-Aminobutyl)-2-(2-methoxyethyl)-1*H*-imidazo[4,5-c]quinolin-4-amine (50 mg) was placed in a 2 dram (7.4 mL) vial. 4-(Dimethylamino)pyridine (19 mg, 1.0 eq) and dichloromethane (800 µL) were added. The vial was sealed and cooled to -78°C in a dry ice/acetone bath. Sulfuryl chloride (186 µL of 1 M in dichloromethane) was added. The vial was put on a shaker for about 30 minutes and then cooled back down to -78°C. A separate vial was charged with the amine of formula R₄R₅NH (2.0 eq), triethylamine (2.0 eq) and dichloromethane (1 mL) and cooled to -78°C. The amine/triethylamine solution was added to the first vial. The vial was placed on a shaker at ambient temperature for about 1 hour. The reaction mixture was analyzed by LC/MS to confirm the formation of the desired product. The solvent was removed and the residue was purified by semi-preparative HPLC (Capcell Pak C 18 column, 35 mm x 20 mm, 5 micron particle size, 20 mL/min., gradient elution from 5-95% B in 10 min., hold at 95% B for 2 min., where A=0.1 % trifluoroacetic acid/water and B=0.1 % trifluoroacetic acid/acetonitrile, peak detection at 254 nm for triggering fraction collection). The semi-prep HPLC fractions were analyzed by LC-APCI/MS and the appropriate fractions were combined and lyophilized to provide the trifluoroacetate salt of the desired sulfamide.

| Example # | Structure of the Free Base | APCI-MS m/e |
|---|---|---|
| 222 | 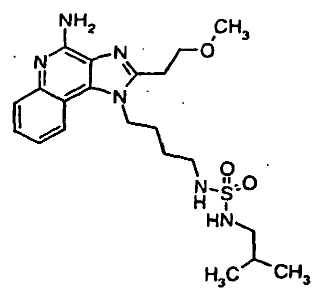 | 449.2 |
| 223 | 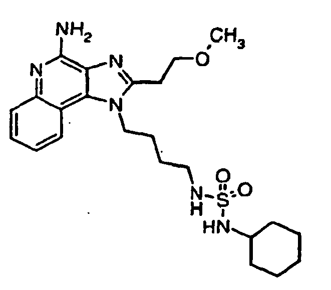 | 475.3 |
| 224 | 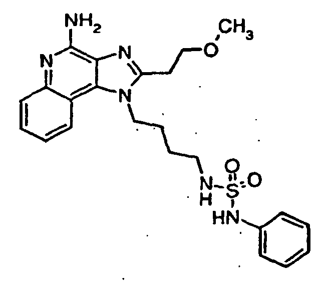 | 469.1 |
| 225 | 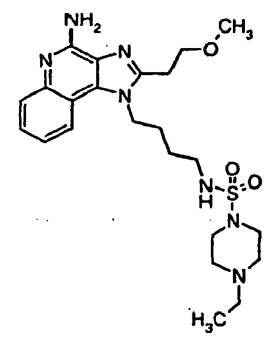 | 490.2 |
| 226 | 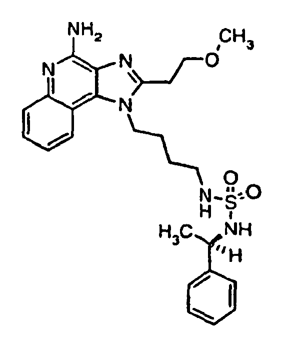 | 497.1 |
| 227 | 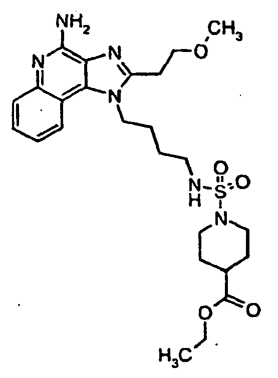 | 533.2 |
| 228 | 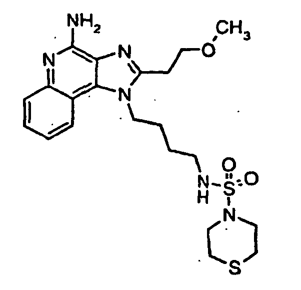 | 479.1 |

### Examples 229 - 231

The examples in the table below were prepared using the method of Examples 222 - 228 above except that the amine of formula R₄R₅NH was reacted with the sulfuryl chloride to provide the sulfamoyl chloride intermediate which was then reacted with 2.0 eq of 1-(4-aminobutyl)-2-(2-methoxyethyl)-1*H*-imidazo[4,5-c]quinolin-4-amine.

| Example # | Structure of the Free Base | APCI-MS m/e |
|---|---|---|
| 229 | 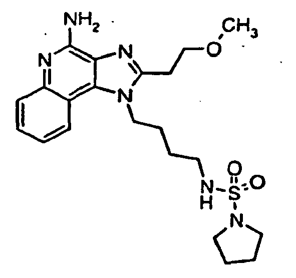 | 447.1 |
| 230 | 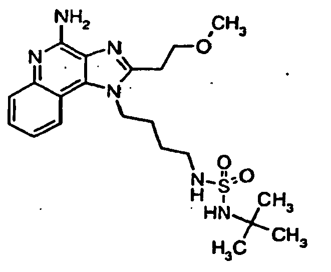 | 449.2 |
| 231 | 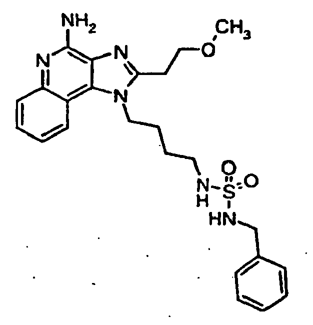 | 483.2 |

### CYTOKINE INDUCTION IN HUMAN CELLS

An in vitro human blood cell system was used to assess cytokine induction by compounds of the invention. Activity is based on the measurement of interferon and tumor necrosis factor (α) (IFN and TNF, respectively) secreted into culture media as described by Testerman et. al. In "Cytokine Induction by the Immunomodulators Imiquimod and S-27609", Journal of Leukocyte Biology, 58, 365-372 (September, 1995).

### Blood Cell Preparation for Culture

Whole blood is collected by venipuncture into EDTA vacutainer tubes from healthy human donors. Peripheral blood mononuclear cells (PBMCs) are separated from whole blood by density gradient centrifugation using Histopaque®-1077 (Sigma Chemicals, St. Louis, MO). The PBMCs are suspended at 3-4 x 10⁶ cells/mL in RPMI 1640 medium containing 10 % fetal bovine serum, 2 mM L-glutamine and 1 % penicillin/streptomycin solution (RPMI complete). The PBMC suspension is added to 48 well flat bottom sterile tissue culture plates (Costar, Cambridge, MA or Becton Dickinson Labware, Lincoln Park, NJ) containing an equal volume of RPMI complete media containing test compound.

### Compound Preparation

The compounds are solubilized in dimethyl sulfoxide (DMSO). The DMSO concentration should not exceed a final concentration of 1 % for addition to the culture wells.

### Incubation

The solution of test compound is added at 60 µM to the first well containing RPMI complete and serial (three fold or ten fold) dilutions are made. The PBMC suspension is then added to the wells in an equal volume, bringing the test compound concentrations to the desired range. The final concentration of PBMC suspension is 1.5-2 X 10⁶ cells/mL. The plates are covered with sterile plastic lids, mixed gently and then incubated for 18 to 24 hours at 37°C in a 5% carbon dioxide atmosphere.

### Separation

Following incubation the plates are centrifuged for 5-10 minutes at 1000 rpm (~200 x g) at 4°C. The cell culture supernatant is removed with a sterile polypropylene pipet and transferred to sterile polypropylene tubes. Samples are maintained at -30 to - 70°C until analysis. The samples are analyzed for interferon (α) and tumor necrosis factor (α) by ELISA

### Interferon (α) and Tumor Necrosis Factor (α) Analysis by ELISA

Interferon (α) concentration is determined by ELISA using a Human Multi-Species kit from PBL Biomedical Laboratories, New Brunswick, NJ.

Tumor necrosis factor (α) (TNF)concentration is determined using ELISA kits available from Genzyme, Cambridge, MA; R&D Systems, Minneapolis, MN; or Pharmingen, San Diego, CA.

The table below lists the lowest concentration found to induce interferon and the lowest concentration found to induce tumor necrosis factor for each compound. A "**" indicates that no induction was seen at any of the tested concentrations (0.12, 0.37, 1.11, 3.33. 10 and 30 µM). A "***" indicates that no induction was seen at any of the tested concentrations (0.0001, 0.001, 0.01, 0.1, 1 and 10 µM).

| Cytokine Induction in Human Cells | | |
|---|---|---|
| Example Number | Lowest Effective Concentration (µM) | |
| | Interferon | Tumor Necrosis Factor |
| 1 | 0.12 | 3.33 |
| 2 | ** | ** |
| 3 | 0.01 | ** |
| 6 | 0.00017 | 1.11 |
| 7 | 0.01 | ** |
| 9 | 0.04 | ** |
| 11 | 0.01 | 1.11 |
| 13 | 10 | ** |
| 17 | 1.11 | 3.33 |
| 18 | 3.33 | ** |
| 19 | 0.12 | 3.33 |
| 20 | 0.12 | 3.33 |
| 21 | 1.11 | 30 |
| 22 | 0.37 | ** |
| 23 | 0.12 | 10 |
| 24 | 0.12 | 30 |
| 25 | 3.33 | ** |
| 26 | 10 | ** |
| 27 | 1-11 | 30 |
| 28 | 1.11 | 30 |
| 29 | 0.37 | 10 |
| 30 | 1.11 | ** |
| 31 | 1.11 | ** |
| 32 | 1.11 | ** |
| 33 | 1.11 | 10 |
| 34 | 0.04 | 0.37 |
| 35 | 1.11 | 10 |
| 36 | 0.0015 | 3.33 |
| 37 | 0.01 | 1.11 |
| 38 | 0.0015 | 0.37 |
| 40 | 0.0015 | 3.33 |
| 41 | 0.01 | ** |
| 42 | 0.01 | ** |
| 43 | 0.04 | ** |
| 44 | 0.0015 | 1.11 |
| 45 | 0.37 | ** |
| 46 | 0.37 | ** |
| 47 | 0.37 | ** |
| 48 | 0.37 | 10 |
| 50 | 0.12 | ** |
| 51 | 0.0415 | 0.37 |
| 52 | 0.12 | 10 |
| 53 | 0.01 | 3.33 |
| 54 | 10 | ** |
| 55 | 3.33 | ** |
| 56 | ** | ** |
| 57 | 3.33 | ** |
| 58 | 3.33 | ** |
| 59 | 3.33 | ** |
| 60 | ** | ** |
| 61 | 3.33 | ** |
| 62 | ** | ** |
| 63 | ** | ** |
| 64 | 3.33 | ** |
| 65 | 3.33 | ** |
| 66 | ** | 30 |
| 67 | 10 | ** |
| 68 | 10 | ** |
| 69 | 10 | ** |
| 70 | ** | ** |
| 71 | ** | 30 |
| 72 | 3.33 | ** |
| 73 | 0.001 | 0.1 |
| 74 | 0.001 | 0.01 |
| 75 | *** | *** |
| 76 | *** | *** |
| 77 | 0.001 | 1 |
| 78 | 0.001 | 0.1 |
| 79 | 0.01 | 1 |
| 80 | 1 | 10 |
| 81 | 0.001 | 1 |
| 82 | 0.001 | 1 |
| 83 | 0.001 | 1 |
| 84 | 1 | 10 |
| 85 | 1 | *** |
| 86 | 0.01 | 1 |
| 87 | 0.001 | |
| 88 | 0.01 | 1 |
| 89 | 0.001 | 1 |
| 90 | 0.01 | 1 |
| 91 | 0.01 | 1 |
| 92 | 0.1 | 10 |
| 93 | 0.001 | 0.1 |
| 94 | 0.001 | 1 |
| 95 | 0.001 | 1 |
| 96 | 1 | *** |
| 97 | 0.1 | 10 |
| 98 | 1 | *** |
| 99 | 0.1 | 10 |
| 100 | 0.01 | 10 |
| 101 | 0.01 | 10 |
| 102 | 0.001 | 10 |
| 103 | 0.1 | 10 |
| 104 | 0.01 | *** |
| 105 | 1 | 10 |
| 106 | 1 | 1 |
| 107 | 1 | *** |
| 108 | 0.1 | 10 |
| 109 | 1 | 10 |
| 110 | 10 | *** |
| 111 | 0.001 | 10 |
| 112 | 0.0001 | *** |
| 113 | 0.0001 | *** |
| 114 | 0.01 | *** |
| 116 | 0.001 | 1 |
| 117 | 0.0001 | 1 |
| 120 | 0.0001 | 1 |
| 121 | 0.0001 | 10 |
| 122 | 0.0001 | |
| 123 | 0.0001 | 10 |
| 127 | 0.0001 | 10 |
| 128 | 0.0001 | |
| 131 | 0.0001 | 1 |
| 138 | 0.0001 | 10 |
| 148 | 0.0001 | 1 |
| 152 | 0.0001 | 10 |
| 154 | 0.001 | 10 |
| 158 | 0.0001 | 1 |
| 159 | 0.0001 | 0.1 |
| 160 | 0.001 | 1 |
| 161 | 0.01 | 10 |
| 184 | 0.0001 | 1 |
| 200 | 0.01 | 0.1 |
| 202 | 0.0001 | 1 |
| 203 | 0.0001 | 1 |
| 204 | 0.0001 | 1 |
| 205 | 0.0001 | 1 |
| 206 | 1 | *** |
| 207 | 0.001 | 1 |
| 208 | 0.0001 | 1 |
| 209 | 0.0001 | 0.1 |
| 210 | 0.0001 | 1 |
| 211 | 0.0001 | 1 |
| 212 | 0.0001 | 0.01 |
| 213 | 0.0001 | 1 |
| 214 | 0.01 | 10 |
| 215 | 0.01 | 1 |
| 217 | 1 | *** |
| 218 | 0.0001 | 1 |
| 220 | 0.0001 1 | 1 |
| 221 | 0.000 | 1 |
| 224 | 0.0001 | 10 |
| 226 | 0.0001 | 0.1 |
| 227 | 0.001 | *** |
| 229 | 0.0001 . | 0.1 |
| 230 | 0.0001 | 1 |
| 231 | 0.0001 | 1 |

## Claims

1. A compound of the formula (I): 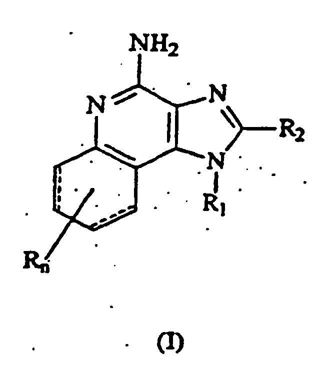 wherein
**R₁** is -alkyl-NR₃- SO₂ -X-R₄ or -alkenyl-NR₃- SO₂ -X-R₄;
**X** is a bond or -NR₅-;
**R₄** is aryl, heteroaryl, heterocyclyl, alkyl or alkenyl, each of which may be unsubstituted or substituted by one or more substituents selected from the group consisting of:
-alkyl;
-alkenyl;
-aryl;
-heteroaryl;
-heterocyclyl;
-substituted cycloalkyl;
-substituted aryl;
-substituted heteroaryl;
-substituted heterocyclyl;
-O-alkyl;
-O-(alkyl)₀₋₁-aryl;
-O-(alkyl)₀₋₁-substituted aryl;
-O-(alkyl)₀₋₁-heteroaryl;
-O-(alkyl)₀₋₁-substituted heteroaryl;
-O-(alkyl)₀₋₁-heterocyclyl;
-O-(alkyl)₀₋₁-substituted heterocyclyl;
-COOH;
-CO-O-alkyl;
-CO-alkyl;
-S(O)₀₋₂-alkyl;
-S(O)₀₋₂-(alkyl)₀₋₁aryl;
-S(O)₀₋₂-(alkyl)₀₋₁-substituted aryl;
-S(O)₀₋₂-(alkyl)₀₋₁heteroaryl:
-S(O)₀₋₂-(alkyl)₀₋₁-substituted heteroaryl;
-S(O)₀₋₂-(alkyl)₀₋₁heterocyclyl;
-S(O)₀₋₂-(alkyl)₀₋₁-substituted heterocyclyl;
-(alkyl)₀₋₁-NR₃R₃;
-(alkyl)₀₋₁-NR₃-CO-O-alkyl;
-(alkyl)₀₋₁-NR₃-CO-alkyl;
-(alkyl)₀₋₁-NR₃-CO-aryl;
-(alkyl)₀₋₁-NR₃-CO-substituted aryl;
-(alkyl)₀₋₁-NR₃-CO-heteroaryl;
-(alkyl)₀₋₁-NR₃-CO-substituted heteroaryl;
-N₃;
-halogen;
-haloalkyl;
-haloalkoxy;
-CO-haloalkyl;
-CO-haloalkoxy;
-NO₂;
-CN;
-OH;
-SH; and in the case of alkyl, alkenyl, or heterocyclyl, oxo;
**R₂** is selected from the group consisting of:
-hydrogen;
-alkyl;
-alkenyl;
-aryl;
-substituted aryl;
-heteroaryl;
-substituted heteroaryl;
-alkyl-O-alkyl;
-alkyl-O-alkenyl; and
-alkyl or alkenyl substituted by one or more substituents selected from the group consisting of:
-OH;
-halogen;
-N(R₃)₂;
-CO-N(R₃)₂;
-CO-C₁₋₁₀ alkyl;
-CO-O-_{C1-10} alkyl;
-N₃;
-aryl;
-substituted aryl;
-heteroaryl;
-substituted heteroaryl;
-heterocyclyl;
-substituted heterocyclyl;
-CO-aryl;
-CO-(substituted aryl);
-CO-heteroaryl; and
-CO-(substituted heteroaryl);
each **R₃** is independently selected from the group consisting of hydrogen and C₁₋₁₀ alkyl;
**R₅** is selected from the group consisting of hydrogen and C₁₋₁₀ alkyl, or **R₄** and **R₅** can combine to form a 3 to 7 membered heterocyclic or substituted heterocyclic ring;
n is 0 to 4 and each R present is independently selected from the group consisting of C₁₋₁₀ alkyl, C₁₋₁₀ alkoxy, halogen and trifluoromethyl, and wherein the terms "substituted cycloalkyl", "substituted aryl", "substituted heteroaryl" and "substituted heterocyclyl" indicate that the rings or ring systems in question are further substituted by one or more substituents independently selected from the group consisting of alkyl, alkoxy, alkylthio, hydroxy, halogen, haloalkyl, haloalkylcarbonyl, haloalkoxy (*e.g*., trifluoromethoxy), nitro, alkylcarbonyl, alkenylcarbonyl, arylcarbonyl, heteroarylcarbonyl, aryl, arylalkyl heteroaryl, heteroarylalkyl, heterocyclyl, heterocycloalkyl, nitrile, alkoxycarbonyl, alkanoyloxy, alkanoylthio, and in the case of cycloalkyl and heterocyclyl, oxo,
or a pharmaceutically acceptable salt thereof.

2. A compound of claim 1 wherein X is a bond.

3. A compound of claim 2 wherein R₁ is -(CH₂)₂₋₄- NR₃- SO₂ -R₄.

4. A compound of claim 2 wherein R₄ is selected from the group consisting of alkyl, aryl and heteroaryl that may be unsubstituted or substituted by one or more substituents selected from the group consisting of:
-alkyl;
-alkenyl;
-aryl;
-heteroaryl;
-heterocyclyl;
-substituted aryl;
-substituted heteroaryl;
-substituted heterocyclyl;
-O-alkyl;
-O-(alkyl)₀₋₁aryl;
-O-(alkyl)₀₋₁-substituted aryl;
-O-(alkyl)₀₋₁-heteroaryl;
-O-(alkyl)₀₋₁-substituted heteroaryl;
-O-(alkyl)₀₋₁-heterocyclyl;
-O-(alkyl)₀₋₁-subsututed heterocyclyl;
-COOH;
-CO-O-alkyl;
-CO-alkyl;
-S(O)₀₋₂ -alkyl;
-S(O)₀₋₂-(alkyl)₀₋₁-aryl;
-S(O)₀₋₂-(alkyl)₀₋₁-subtituted aryl;
-S(O)₀₋₂-(alkyl)₀₋₁-heteroaryl;
-S(O)₀₋₂-(alkyl)₀₋₁substituted heteroaryl:
-S(O)₀₋₂-(alkyl)₀₋₁-heterocyclyl;
-S(O)₀₋₂-(alkyl)₀₋₁-substituted heterocyclyl;
-(alkyl)₀₋₁-NR₃R₃;
-(alkyl)₀₋₁-NR₃-CO-O-alkyl;
-(alkyl)₀₋₁-NR₃-CO-alkyl;
-(alkyl)₀₋₁-NR₃-CO-aryl;
-(alkyl)₀₋₁-NR₃-CO-substituted aryl;
-(alkyl)₀₋₁NR₃-CO-heteroaryl;
-(alkyl)₀₋₁-NR₃-CO-substituted heteroaryl;
-N₃;
-halogen;
-haloalkyl;
-haloalkoxy;
-CO-haloalkoxy;
-NO₂;
-CN;
-OH;
-SH; and in the case of alkyl, oxo, and wherein the terms "substituted aryl", "substituted heteroaryl" and "substituted heterocyclyl" are as defined in claim 1.

5. A compound of claim 2 wherein the dashed bonds are absent.

6. A compound of claim 1 wherein X is -NR₅-.

7. A compound of claim 2 or 6 wherein n is 0.

8. A compound of claim 6 wherein R₁ is -(CH₂)₂₋₄- NR₃- SO₂ -NR₅ -R₄.

9. A compound of claim 6 wherein R₄ and R₅ join to form a 3 to 7 membered heterocyclic or substituted heterocyclic ring, wherein the term "substituted heterocyclyl" is defined as in claim 1

10. A compound of claim 9 wherein R₄ and R₅ join to form a substituted or unsubstituted pyrrolidine, morpholine, thiomorpholine, piperidine, or piperazine ring.

11. A compound of claim 2, 6 or 10 wherein R₂ is selected from the group consisting of hydrogen. C₁₋₄alkyl, and C₁₋₄alkyl-O-C₁₋₄alkyl.

12. compound of claim 6 wherein R₄ and R₅ are alkyl.

13. A compound of claim 2, 6, 9 or 12 wherein R₂ is selected from the group consisting of hydrogen; alkyl; akyl-O-alkyl; (alkyl)₀₋₁ aryl, (alkyl)₀₋₁-(substittited aryl); (alkyl)₀₋₁-heteroaryl; and (alkyl)₀₋₁-(substituted heteroaryl), and wherein the terms "substituted aryl" and "substituted heteroaryl" are as defined in claim 1.

14. A compound of claim 2, 6, 10 or 12 wherein R₃ is hydrogen.

15. A compound selected from the group consisting of:
N²-[2-(4-amino-2-butyl-1*H*-imidazo[4,5-*c*]quinolin-1-yl)ethyl]-2-thiophenesulfonamide;
N¹-[2-(4-amino-2-butyl-1*H*-imidazo[4,5-*c*]quinolin-1-yl)ethyl]-1-benzenesulfonamide;
N⁸-[2-(4-amino-2-butyl-1*H*-imidazo[4,5-*c*]quinolin-1-yl)ethyl]-8-quinolinesulfonamide.
N¹-[2-(4-amino-2-butyl-1*H*-imidazo[4,5-*c*]quinolin-1-yl)ethyl]-5-(dimethylamino)-1-naphthalenesulfonamide;
N-[4-(4-amino-2-butyl-1*H*-imidazo[4,5-*c*]quinolin-1-yl)butyl]methanesulfonamide;
N¹-[4-(4-amino-2-butyl-1*H*-imidazo[4,5-*c*]quinolin-1-yl)butyl]-1-benzenesulfonamide;
N⁸-[4-(4-amino-2-butyl-1*H*-imidazo[4,5-*c*]quinolin-1-yl)butyl]-8-quinolinesulfonamide;
N²-[4-(4-amino-2-butyl-1*H*-imidazo[4,5-*c*]quinolin-1-yl)butyl]-2-thiophenesulfonamide;
N²-[4-(4-amino-6,7,8,9-tetrahydro-1*H*-imidazo[4,5-*c*]quinolin-1-yl)butyl]-2-thiophenesulfonamide;
N¹-[4[-(4-amino-6,7,8,9-tetrabydro-1*H*-imidazo[4,5-*c*]quinolin-1-yl)butyl]-1-benzenesulfonamide;
N⁸-[4-(4-amino-6,7,8.9-tetrahydro-1*H-*imidazo[4,5-*c*]quinolin-1-yl)butyl]-8-quinolinesulfonamide;
N¹-[4-(4-amino-6,7,8,9-tetrahydro-1*H*-imidazo[4,5-*c*]quinolin-1-yl)butyl]-5-(dimethylamino)-1-naphthalenesulfonamide;
N¹-[4-(4-amino-2-butyl-1*H*-imidazo[4,5-*c*]quinolin-1-yl)butyl]-4-fluoro-1-benzenesulfonamide;
N¹-[4-(4-amino-2-butyl-1*H*-imidazo[4,5-*c*]quinolin-1-yl)butyl]-3-fluoro-1-benzenesulfonamide;
N-{2-[4amino-2-(ethoxymethyl)-1*H*-imidazo[4,5-*c*]quinolin-1-yl]ethyl}methanesulfonamide;
N²-{2-[4-amino-2-(ethoxymethyl)-1*H*-imidazo[4,5-*c*]quinolin-1-yl]ethyl}-2-thiophenesulfonamide;
N¹-{2-[4-amino-2-(ethoxymethyl-1*H*-imidazo[quinolin-1-yl]ethyl}-5-(dimethylamino)-1-naphthalenesulfonamide;
N-{4-[4-amino-2-(2-methoxyethyl)-1*H*-imidazo[4,5-*c*]quinolin-1-yl]butyl}methanesulfonamide;
N²-(4-[4-amino-2-(2-methoxyethyl)-1*H*-imidazo[4,5-*c*]quinolin-1-yl]butyl}-2-thiophenesulfonamide
N¹-{4-[4-amino-2-(2-methoxyethyly-1*H*-imidazo[4,5-*c*]quinolin-1-yl]butyl}-5-(dimethylamino)-1-naphthalenesulfonamide;
N'-(4-[4-amino-2-(2-methoxyethyl)-1*H*-imidazo[4,5-*c*]quinolin-1-yl]butyl}-4-fluoro-1-benzenesulfonamide;
N¹-{4-[4-amino-2-(2-methoxyethyl)-1*H*-imidazo[4,5-*c*]quinolin-1-yl]butyl}-3-fluoro-1-benzenesulfonamide;
N¹-{4-[4-amino-2-(2-methoxyethyl)-1*H*-imidazo[4,5-*c*]quinolin-1-yl]butyl}-1-benzenesulfonamide;
N⁸-{4-[4-amino-2-(2-methoxyethyl)-1*H*-imidazo[4,5-*c*]quinolin-1-yl]butyl}-8-quinolinesulfonamide;
N²-[4-[4-amino-2-(4-methoxybenzyl}-1*H*-imidazo[4,5-*c*]quinolin-1-yl]butyl}-2-thiophenesulfonamide;
N-[4-(4-amino-2-butyl-6,7,8,9-tetrahydro-1*H*-imidazo[4,5-*c*]quinolin-1-yl)butyl]methanesulfonamide;
N²-[4-(4-amino-2-butyl-6,7,8,9-tetrahydr6-1*H*-imidazo[4,5-*c*]quinolin-1-yl)butyl]-2-thiophenesulfonamide;
N¹-[4-(4-amino-1-butyl-6,7,8,9-tetrahydro-1*H*-imidazo[4,5-*c*]quinolin-1-yl)butyl]-5-(dimethylamino)-1-naphthalenesulfonamide.
N¹{-4-[4-amino-2-(2-methoxyethyl)-6,7,8,9-tetrahydro-1*H*-imidazo[4,5-*c*]quinolin-1-yl]butyl}-1-benzenesulfonamide:
N¹-{4-[4-amino-2-(2-methoxyethyl)-6,7,8,9-tetrahydro-1*H*-imidazo[4,5-*c*]quinolin-1-yl]butyl}-5-(dimethylamino)-1-naphthalenesulfonamide;
N'-{2-[4-amino-2-(ethoxymethyl)-1*H-*imidazo[4,5-*c*]quinolin-1-yl]ethyl}-*N,N-*dimethylsulfamide;
N'-{4-[4-amino-2-(2-methoxyethyl)-1*H-*imidazo[4,5-*c*]quinolin-1-yl]butyl}*-N,N-*dimethylsulfamide;
N'-{4-[4-amino-2-(4-methoxybenzyl)-1*H*-imidazo[4,5-*c*]quinolin-1-yl]butyl}-*N,N-*dimethylsulfamide;
N'-[4-(4-amino-2-butyl-6,7,8,9-tetrahydro-1*H*-imidazo[4,5-*c*]quinolin-1-yl)butyl]-*N,N*-dimethylsulfamide;
*N*-{4-[4-amino-2-(2-methoxyethyl)-6,7,8,9-tetrahydro-1*H*-imidazo[4,5-*c*]quinolin-1-yl]butyl}-*N,N*-dimethylsulfamide;
N⁴-{4-[4-amino-2-(2-methoxyethyl)-1*H*-imidazo[4,5-*c*]quinolin-1-yl]butyl}-4-thiomorpholinesulfonamide;
N¹-{4-[4-amino-2-(2-methoxyethyl)-1*H*-imidazo[4,5-*c*]quinolin-1-yl]butyl}-1-pyrrolidinesulfonamide;
N¹-[4-(4-amino-2-butyl-6,7,8,9-tetrahydro-1*H*-imidazo[4,5-*c*]quinolin-1-yl)butyl]-4-fluoro-1-benzenesulfonamide
N-[4-(4-Amino-2-(2-methoxyethyl)-6,7,8,9-tetrahydro-1*H*-imidazo[4,5-*c*]quinolin-1-yl)butyl]methanesulfonamide; and
N-{4-[4-amino-2-(2-methoxyethyl)-1*H*-imidazo[4,5-*c*]quinolin-1-yl]butyl}phenylmethanesulfonamide.

16. A compound selected from the group consisting of:
N¹-[4-(4-Amino-2-butyl-1*H*-imidazo[4,5-*c*]quinolin-1-yl)butyl]-5-(dimethylamino)-1-naphthalenesulfonamide;
N¹-[4-(4-Amino-1*H*-imidazo[4,5-*c*]quinolin-1-yl)butyl]-5-(dimethylamino)-1-naphthalenesulfonamide;
N²-[4-(4-Amino-2-buryl-1*H-*imidazo[4,5-*c*]quinolin-1-yl)butyl]-2-thiophenesulfonamide;
N-[4-(4-Amino-2-butyl-1*H*-imidazo[4,5-*c*]quinolin-1-yl)butyl]-phenylmethanesulfonamide;
N¹-[4-(4-Amino-2-butyl-1*H*-imidazo[4,5-*c*]quinolin-1-yl)butyl]-1-benzenesulfonamide;
N-[4-(4-Amino-2-butyl-1*H*-imidazo[4,5-*c*]quinolin-1-yl)butyl] Methanesulfonamide;
N'-[4-(4-Amino-2-butyl-1*H*-imidazo[4,5-*c*]quinolin-1-yl)butyl]-3-nitro-1-benzenesulfonamide;
N¹-[4-(4-Amino-2-butyl-1*H*-imidazo[4,5-*c*]quinolin-1-yl)butyl]-3-amino-1-benzenesulfonamide;
N¹-[4-(4-Amino-2-butyl-1*H*-imidazo[4,5-*c*]quinolin-1-yl)butyl]-4-nitro-1-benzenesulfonamide;
N¹-[4-(4-Amino-2-buryl-1*H*-imidazo[4,5-*c*]quinolin-1-yl)butyl]-4-amino-1-benzenesulfonamide;
N⁵-[4-(4-Amino-2-butyl-1*H*-imidazo[4,5-*c*]quinolin-1-yl)butyl]-5-isoquinolinesulfonamide
N-[4-(4-Amino-2-(4-methoxybenzyl)-1*H*-imidazo[4,5-*c*]quinolin-1-yl]butyl]-methanesulfonamide;
N¹-[4-(4-Amino-1*H*-imidazo[4,5-*c*]quinolin-1-y)butyl]-1-butanesulfonamide;
N¹-{4-[4-Amino-2-(2-methoxyethyl)-6,7,8,9-tetrahydro-1*H*-imidazo[4,5-*c*]quinolin-1-yl]butyl}-4-fluoro-1-benzenesulfonamide;
N¹-[4-(4-Amino-2-phenyl-1*H*-imidazo[4,5-*c*]quinolin-1-yl)butyl]-4-fluoro-1-benzenesulfonamide; and
N-[4-(4-Amino-2-phenyl-1*H*-imidazo[4,5-*c*]quinolin-1-yl)butyl] methanesulfonamide.

17. A pharmaceutical composition comprising a therapeutically effective amount of a compound of claim 1, 2 or 6 and a pharmaceutically acceptable carrier.

18. A compound according to claim 1, 2 or 6 for use in inducing cytokine biosynthesis.

19. A compound according to claim 1, 2 or 6 for use in the treatment of a viral disease.

20. A compound according to claim 1, 2 or 6 for use in the treatment of a neoplastic disease.

21. Use of a compound according to claim 1, 2 or 6 for the manufacture of a medicament for inducing cytokine biosynthesis.

22. Use of a compound according to claim 1, 2 or 6 for the treatment of a viral disease.

23. Use of a compound according to claim 1, 2 or 6 for the treatment of a neoplastic disease.

## Patentansprüche

1. Verbindung der Formel (I): 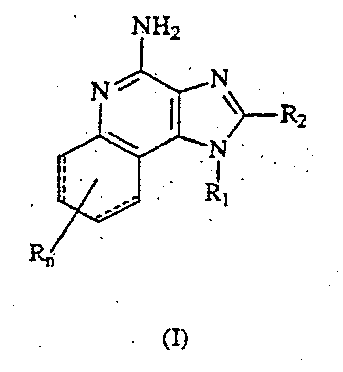 wobei
R₁ für -Alkyl-NR₃-SO₂-X-R₄ oder -Alkenyl-NR₃-SO₂-X-R₄ - steht;
X für eine Bindung oder -NR₅- steht;
R₄ für Aryl, Heteroaryl, Heterocyclyl, Alkyl oder Alkenyl steht, wobei jede dieser Gruppen unsubstituiert oder durch einen oder mehrere Substituenten aus der Gruppe bestehend aus:
-Alkyl;
-Alkenyl;
-Aryl;
-Heteroaryl;
-Heterocyclyl;
-substituiertem Cycloalkyl;
-substituiertem Aryl;
-substituiertem Heteroaryl;
-substituiertem Heterocyclyl;
-O-Alkyl;
-O-(Alkyl)₀₋₁-aryl;
-O-(Alkyl)₀₋₁-subst. aryl;
-O-(Alkyl)₀₋₁-heteroaryl;
-O-(Alkyl)₀₋₁-subst. heteroaryl;
-O-(Alkyl)₀₋₁-heterocyclyl;
-O-(Alkyl)₀₋₁-subst. heterocyclyl;
-COOH;
-CO-O-Alkyl;
-CO-Alkyl;
-8(O)₀₋₂-Alkyl;
-S(O)₀₋₂-(Alkyl)₀₋₁-aryl;
-S(O)₀₋₂-(Alkyl)₀₋₁-subst. aryl;
-S(O)₀₋₂-(Alkyl)₀₋₁-heteroaryl;
-S(O)₀₋₂-(Alkyl)₀₋₁-subst. heteroaryl;
-S(O)₀₋₂-(Alkyl)₀₋₁-heterocyclyl;
-S(O)₀₋₂-(Alkyl)₀₋₁-subst. heterocyclyl;
- (Alkyl)₀₋₁-NR₃R₃;
- (Alkyl)₀₋₁-NR₃-CO-O-alkyl;
- (Alkyl)₀₋₁-NR₃-CO-alkyl ;
- (Alkyl)₀₋₁-NR₃-CO-aryl;
- (Alkyl)₀₋₁-NR₃-CO-subst. aryl;
- (Alkyl)₀₋₁-NR₃-CO-heteroaryl ;
-(Alkyl)₀₋₁-NR₃-CO-subst. heteroaryl;
-N₃;
-Halogen;
-Halogenalkyl;
-Halogenalkoxy;
-CO-Halogenalkyl;
-CO-Halogenalkoxy;
-NO₂ ;
-CN;
-OH;
-SH; und, im Fall von Alkyl, Alkenyl oder Heterocyclyl, Oxo
substituiert sein kann;
R₂ aus der Gruppe bestehend aus
-Wasserstoff;
-Alkyl;
-Alkenyl;
-Aryl;
-substituiertem Aryl;
-Heteroaryl;
-substituiertem Heteroaryl;
-Alkyl-O-alkyl;
-Alkyl-O-alkenyl und
₋Alkyl oder -Alkenyl, das durch einen oder mehrere Substituenten aus der Gruppe bestehend aus
-OH;
-Halogen;
-N(R₃)2;
-CO-N(R₃)₂;
-CO-C₁₋₁₀-Alkyl;
-CO-O-C₁₋₁₀-Alkyl;
-N3 ;
-Aryl;
-substituiertem Aryl;
-Heteroaryl;
-substituiertem Heteroaryl;
-Heterocyclyl;
-substituiertem Heterocyclyl;
-CO-Aryl;
-CO-(subst. Aryl);
-CO-Heteroaryl und
-CO-(subst. Heteroaryl)
substituiert ist,
ausgewählt ist;
R₃ jeweils unabhängig voneinander aus der Gruppe bestehend aus Wasserstoff und C₁₋₁₀-Alkyl ausgewählt ist;
R₅ aus der Gruppe bestehend aus Wasserstoff und C₁-₁₀-Alkyl ausgewählt ist; oder R₄ und R₅ gemeinsam einen 3- bis 7-gliedrigen heterocyclischen oder substituierten heterocyclischen Ring bilden können,
n für 0 bis 4 steht und jedes vorhandene R unabhängig voneinander aus der Gruppe bestehend aus C₁₋₁₀-Alkyl, C₁₋₁₀-Alkoxy, Halogen und Trifluormethyl ausgewählt ist, und wobei die Begriffe "substituiertes Cycloalkyl", "substituiertes Aryl", "substituiertes Heteroaryl" und "substituiertes Heterocyclyl" anzeigen, daß die betreffenden Ringe oder Ringsysteme ferner durch einen oder mehrere Substituenten substituiert sind, unabhängig voneinander ausgewählt aus der Gruppe bestehend aus Alkyl, Alkoxy, Alkylthio, Hydroxy, Halogen, Halogenalkyl, Halogenalkylcarbonyl, Halogenalkoxy (z.B. Trifluormethoxy), Nitro, Alkylcarbonyl, Alkenylcarbonyl, Arylcarbonyl, Heteroarylcarbonyl, Aryl; Arylalkyl, Heteroaryl, Heteroarylalkyl, Heterocyclyl, Heterocycloalkyl, Nitril, Alkoxycarbonyl, Alkanoyloxy, Alkanoylthio, und, im Fall von Cycloalkyl und Heterocyclyl, Oxo;
oder ein pharmazeutisch verträgliches Salz davon.

2. Verbindung nach Anspruch 1, wobei X für eine Bindung steht.

3. Verbindung nach Anspruch 2, wobei R₁ für -(CH₂)₂₋₄-NR₃-SO₂-R₄ steht.

4. Verbindung nach Anspruch 2, wobei R₄ aus der Gruppe bestehend aus Alkyl, Aryl und Heteroaryl ausgewählt ist, das unsubstituiert oder durch einen oder mehrere Substituenten aus der Gruppe bestehend aus:
-Alkyl;
-Alkenyl;
-Aryl;
-Heteroaryl;
-Heterocyclyl;
-substituiertem Aryl;
-substituiertem Heteroaryl;
-substituiertem Heterocyclyl;
-O-Alkyl;
-O-(Alkyl)₀₋₁-aryl;
-O-(Alkyl)₀₋₁-subst. aryl;
-O-(Alkyl)₀₋₁-heteroaryl;
-O-(Alkyl)₀₋₁-subst. heteroaryl;
-O-(Alkyl)₀₋₁-heterocyclyl;
-O-(Alkyl)₀₋₁-subst. heterocyclyl;
-COOH;
-CO-O-Alkyl;
-CO-Alkyl;
-S(O)₀₋₂-Alkyl;
-S(O)₀₋₂-(Alkyl)₀₋₁-aryl;
-S(O)₀₋₂-(Alkyl)₀₋₁-subst. aryl;
-S(O)₀₋₂-(Alkyl)₀₋₁-heteroaryl;
-S(O)₀₋₂-(Alkyl)₀₋₁-subst heteroaryl;
-S(O)₀₋₂-(Alkyl)₀₋₁-heterocyclyl;
-S(O)₀₋₁-(Alkyl)₀₋₁-subst. heterocyclyl;
- (Alkyl)₀₋₁-NR₃R₃;
- (Alkyl)₀₋₁-NR₃-CO-O-alkyl;
- (Alkyl)₀₋₁-NR₃-CO-alkyl;
- (Alkyl)₀₋₁NR₃-CO-aryl;
- (Alkyl)₀₋₁-NR₃-CO-subst. aryl;
- (Alkyl)₀₋₁-NR₃-CO-heteroaryl;
- (Alkyl)₀₋₁-NR₃-CO-subst. heteroaryl;
-N₃ ;
-Halogen;
-Halogenalkyl;
-Halogenalkoxy;
-CO-Halogenalkoxy;
-NO₂;
-CN;
-OH;
-SH; und, im Fall von Alkyl, Oxo
substituiert sein kann, und wobei die Begriffe "substituiertes Aryl", "substituiertes Heteroaryl" und "substituiertes Heterocyclyl" wie in Anspruch 1 definiert sind.

5. Verbindung nach Anspruch 2, wobei die gestrichelten Bindungen fehlen.

6. Verbindung nach Anspruch 1, wobei X für -NR₅- steht.

7. Verbindung nach Anspruch 2 oder 6, wobei n für 0 steht.

8. Verbindung nach Anspruch 6, wobei R₁ für-(CH₂)₂₋₄-NR₃-SO₂-NR₅-R₄ steht.

9. Verbindung nach Anspruch 6, wobei R₄ und R₅ gemeinsam einen 3- bis 7-gliedrigen heterocyclischen oder substituierten heterocyclischen Ring bilden, wobei der Begriff "substituiertes Heterocyclyl" wie in Anspruch 1 definiert ist.

10. Verbindung nach Anspruch 9, wobei R₄ und R₅ gemeinsam einen substituierten oder unsubstituierten Pyrrolidin-, Morpholin-, Thiomorpholin-, Piperidin- oder Piperazinring bilden.

11. Verbindung nach Anspruch 2, 6 oder 10, wobei R₂ aus der Gruppe bestehend aus Wasserstoff, C₁₋₄-Alkyl und C₁₋₄-Alkyl-O-C₁₋₄-alkyl ausgewählt ist.

12. Verbindung nach Anspruch 6, wobei R₄ und R₅ für Alkyl stehen.

13. Verbindung nach Anspruch 2, 6, 9 oder 12, wobei R₂ aus der Gruppe bestehend aus Wasserstoff; Alkyl; Alkyl-O-alkyl; (Alkyl)₀₋₁-aryl, (Alkyl)₀₋₁-(subst. aryl); (Alkyl)₀₋₁-heteroaryl und (Alkyl)₀₋₁-(subst. heteroaryl) ausgewählt ist und wobei die Begriffe "substituiertes Aryl" und "substituiertes Heteroaryl" wie in Anspruch 1 definiert sind.

14. Verbindung nach Anspruch 2, 6, 10 oder 12, wobei R₃ für Wasserstoff steht.

15. Verbindung aus der Gruppe bestehend aus:
N²-[2-(4-Amino-2-butyl-1*H*-imidazo[4,5*-c*]chinolin-1-yl)ethyl]-2-thiophensulfonamid;
N¹-[2-(4-Amino-2-butyl-1*H*-imidazo[4,5*-c*]chinolin-1-yl)ethyl]-1-benzolsulfonamid;
N⁸-[2-(4-Amino-2-butyl-1*H*-imidazo[4,5*-c*]chinolin-1-yl)ethyl]-8*-*chinolinsulfonamid;
N¹-[2-(4-Amino-2-butyl-1*H*-imidazo[4,5*-c*]chinolin-1-yl)ethyl]-5-(dimethylamino)-1-naphthalinsulfonamid;
N-[4-(4-Amino-2-butyl-1*H*-imidazo[4,5*²*]chinolin-1-yl)butyl]methansulfonamid;
N¹-[4-(4-Amino-2-butyl-1*H*-imidazo[4,5*-c*]chinolin-1-yl)butyl]-1-benzolsulfonamid;
N⁸-[4-(4-Amino-2-butyl-1*H*-imidazo[4,5*-c*]chinolin-1-yl)butyl]-8-chinolinsulfonamid;
N²-[4-(4-Amino-2-butyl-1*H*-imidazo[4,5*-c*]chinolin-1-yl)butyl]-2-thiophensulfonamid;
N²-[4-(4-Amino-6,7,8,9-tetrahydro-1*H*-imidazo[4,5-*c*]chinolin-1-yl)butyl]-2-thiophensulfonamid;
N¹-[4-(4-Amino-6,7,8,9-tetrahydro-1*H*-imidazo[4,5-*c*]chinolin-1-yl)butyl]-1-benzolsulfonamid;
N⁸-[4-(4-Amino-6,7,8,9-tetrahydro-1*H*-imidazo[4,5-*c*]chinolin-1-yl)butyl]-8-chinolinsulfonamid;
N¹-[4-(4-Amino-6,7,8,9-tetrahydro-1*H*-imidazo[4,5-*c*]chinolin-1-yl)butyl]-5-(dimethylamino)-1-naphthalinsulfonamid;
N¹-[4-(4-Amino-2-butyl-1*H*-imidazo[4,5*-c*]chinolin-1-yl)butyl]-4-fluor-1-benzolsulfonamid;
N¹-[4-(4-Amino-2-butyl-1*H*-imidazo[4,5*-c*]chinolin-1-yl)butyl]-3-fluor-1-benzolsulfonamid;
N-{2-[4-Amino-2-(ethoxymethyl)-1*H*-imidazo[4,5*-c*]-chinolin-1-yl]ethyl}methansulfonamid;
N²-{2-[4-Amino-2-(ethoxymethyl)-1*H*-imidazo[4,5-*c*]chinolin-1-yl]ethyl}-2-thiophensulfonamid;
N¹-{2-[4-Amino-2-(ethoxymethyl)-1*H*-imidazo[4,5-*c*]chinolin-1-yl]ethyl}-5-(dimethylamino)-1-naphthalinsulfonamid;
N-{4-[4-Amino-2-(2-methoxyethyl)-1*H*-imidazo[4,5-*c*]chinolin-1-yl]butyl}methansulfonamid;
N²-{4-[4-Amino-2-(2-methoxyethyl)-1*H*-imidazo[4,5-*c*]chinolin-1-yl]butyl}-2-thiophensulfonamid;
N¹-{4-[4-Amino-2-(2-methoxyethyl)-1*H*-imidazo[4,5-*c*]chinolin-1-yl]butyl}-5-(dimethylamino)-1-naphthalinsulfonamid;
N¹-{4-[4-Amino-2-(2-methoxyethyl)-1*H*-imidazo[4,5-*c*]chinolin-1-yl]butyl}-4-fluor-1-benzolsulfonamid;
N¹-{4-[4-Amino-2-(2-methoxyethyl)-1*H*-imidazo[4,5-*c*]chinolin-1-yl]butyl}-3-fluor-1-benzolsulfonamid;
N¹-{4-[4-Amino-2-(2-methoxyethyl)-1*H*-imidazo[4,5-*c*]chinolin-1-yl]butyl}-1-benzolsulfonamid;
N⁸-{4-[4-Amino-2-(2-methoxyethyl)-1*H*-imidazo[4,5-*c*]chinolin-1-yl]butyl}-8-chinolinsulfonamid;
N²-{4-[4-Amino-2-(4-methoxybenzyl)-1*H*-imidazo[4,5-*c*]chinolin-1-yl]butyl}-2-thiophensulfonamid;
N-[4-(4-Amino-2-butyl-6,7,8,9-tetrahydro-1*H-*imidazo[4,5*-c*]chinolin-1-yl)butyl]methansulfonamid;
N²-[4-(4-Amino-2-butyl-6,7,8,9-tetrahydro-1*H-*imidazo[4,5*-c*]chinolin-1-yl)butyl]-2-thiophensulfonamid;
N¹-[4-(4-Amino-2-butyl-6,7,8,9-tetrahydro-1*H-*imidazo[4,5*-c*]chinolin-1-yl)butyl]-5-(dimethylamino)-1-naphthalinsulfonamid;
N¹-{4-[4-Amino-2-(2-methoxyethyl)-6,7,8,9-tetrahydro-1*H*-imidazo[4,5*-c*]chinolin-1-yl]butyl}-1-benzolsulfonamid;
N¹-{4-[4-Amino-2-(2-methoxyethyl)-6,7,8,9-tetrahydro-1*H*-imidazo[4,5*-c*]chinolin-1-yl]butyl}-5-(dimethylamino)-1-naphthalinsulfonamid;
N'-{2-[4-Amino-2-(ethoxymethyl)-1*H*-imidazo[4,5-*c*]chinolin-1-yl]ethyl}-*N,N*-dimethylsulfamid;
N'-{4-[4-Amino-2-(2-methoxyethyl)-1*H-*imidazo[4,5-*c*]chinolin-1-yl]butyl}-*N,N*-dimethylsulfamid;
N'-{4-[4-Amino-2-(4-methoxybenzyl)-1*H-*imidazo[4,5-*c*]chinolin-1-yl]butyl}-*N,N*-dimethylsulfamid;
N'-[4-(4-Amino-2-butyl-6,7,8,9-tetrahydro-1*H-*imidazo[4,5*-c*]chinolin-1-yl)butyl]-*N,N*-dimethylsulfamid;
N'-{4-[4-Amino-2-(2-methoxyethyl)-6,7,8,9-tetrahydro-1*H-*imidazo[4,5*-c*]chinolin-1-yl]butyl}-*N,N*-dimethylsulfamid;
N⁴-{4-[4-Amino-2-(2-methoxyethyl)-1*H-*imidazo[4,5-*c*]chinolin-1-yl]butyl}-4-thiomorpholinsulfonamid;
N¹-{4-[4-Amino-2-(2-methoxyethyl)-1*H-*imidazo[4,5-*c*]chinolin-1-yl]butyl}-1-pyrrolidinsulfonamid;
N¹-[4-(4-Amino-2-butyl-6,7,8,9-tetrahydro-1*H-*imidazo[4,5*-c*]chinolin-1-yl)butyl]-4-fluor-1-benzolsulfonamid;
N-[4-(4-Amino-2-(2-methoxyethyl)-6,7,8,9-tetrahydro-1*H-*imidazo[4,5*-c*]chinolin-1-yl)butyl]methansulfonamid und
N-{4-[4-Amino-2-(2-methoxyethyl)-1*H-*imidazo[4,5-*c*]chinolin-1-yl]butyl}phenylmethansulfonamid.

16. Verbindung aus der Gruppe bestehend aus:
N1-[4-(4-Amino-2-butyl-1*H-*imidazo[4,5*-c*]chinolin-1-yl)butyl]-5-(dimethylamino)-1-naphthalinsulfonamid;
N¹-[4-(4-Amino-1*H-*imidazo[4,5*-c*]chinolin-1-yl)-butyl]-5-(dimethylamino)-1-naphthalinsulfonamid;
N²-[4-(4-Amino-2-butyl-1*H-*imidazo[4,5*-c*]chinolin-1-yl)butyl]-2-thiophensulfonamid;
N-[4-(4-Amino-2-butyl-1*H-*imidazo[4,5*-c*]chinolin-1-yl)butyl]phenylmethansulfonamid;
N¹-[4-(4-Amino-2-butyl-1*H-*imidazo[4,5*-c*]chinolin-1-yl)butyl]-1-benzolsulfonamid;
N-[4-(4-Amino-2-butyl-1*H-*imidazo[4,5*-c*]chinolin-1-yl)butyl]methansulfonamid;
N¹-[4-(4-Amino-2-butyl-1*H-*imidazo[4,5*-c*]chinolin-1-yl)butyl]-3-nitro-1-benzolsulfonamid;
N¹-[4-(4-Amino-2-butyl-1*H-*imidazo[4,-5*-c*]chinolin-1-yl)butyl]-3-amino-1-benzolsulfonamid;
N¹-[4-(4-Amino-2-butyl-1*H-*imidazo[4,5*-c*]chinolin-1-yl)butyl]-4-nitro-1-benzolsulfonamid;
N¹-[4-(4-Amino-2-butyl-1*H-*imidazo[4,5*-c*]chinolin-1-yl)butyl]-4-amino-1-benzolsulfonamid;
N⁵-[4-(4-Amino-2-butyl-1*H-*imidazo[4,5*-c*]chinolin-1-yl)butyl]-5-isochinolinsulfonamid;
N-[4-(4-Amino-2-(4-methoxybenzyl)-1*H-*imidazo[4,5-*c*]chinolin-1-yl)butyl]methansulfonamid;
N¹-[4-(4-Amino-1*H-*imidazo[4,5*-c*]chinolin-1-yl)-butyl]-1-butansulfonamid;
N¹-{4-[4-Amino-2-(2-methoxyethyl)-6,7,8,9-tetrahydro-1*H-*imidazo[4,5*-c*]chinolin-1-yl]butyl}-4-fluor-1-benzolsulfonamid;
N¹-[4-(4-Amino-2-phenyl-1*H-*imidazo[4,5*-c*]chinolin-1-yl)butyl]-4-fluor-1-benzolsulfonamid und
N-[4-(4-Amino-2-phenyl-1*H-*imidazo[4,5*-c*]chinolin-1-yl)butyl]methansulfonamid.

17. Arzneimittel, umfassend eine therapeutisch wirksame Menge einer Verbindung nach Anspruch 1, 2 oder 6 und einen pharmazeutisch verträglichen Träger.

18. Verbindung nach Anspruch 1, 2 oder 6 zur Verwendung bei der Induktion der Cytokin-Biosynthese.

19. Verbindung nach Anspruch 1 , 2 oder 6 zur Verwendung bei der Behandlung einer Viruserkrankung.

20. Verbindung nach Anspruch 1, 2 oder 6 zur Verwendung bei der Behandlung einer neoplastischen Erkrankung.

21. Verwendung einer Verbindung nach Anspruch 1, 2 oder 6 zur Herstellung eines Medikaments zur Induktion der Cytokin-Biosynthese.

22. Verwendung einer Verbindung nach Anspruch 1, 2 oder 6 zur Behandlung einer Viruserkrankung.

23. Verwendung einer Verbindung nach Anspruch 1, 2 oder 6 zur Behandlung einer neoplastischen Erkrankung.

## Revendications

1. Composé de formule (I) : 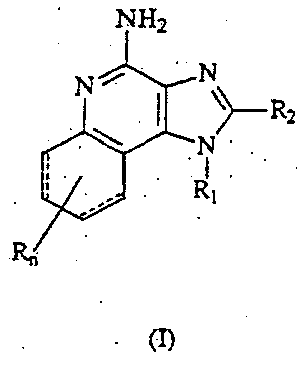 dans laquelle
R₁ est -alkyl-NR₃-SO₂-X-R₄ ou -alcényl-NR₃-SO₂-X-R₄ ;
X est une liaison ou -NR₅- ;
R₄ est aryle, hétéroaryle, hétérocyclyle, alkyle ou alcényle, dont chacun peut être non substitué ou substitué par un ou plusieurs substituants choisis dans le groupe constitué de :
-alkyle ;
-alcényle ;
-aryle ;
-hétéroaryle ;
-hétérocyclyle ;
-cycloalkyle substitué ;
-aryle substitué ;
-hétéroaryle substitué ;
-hétérocyclyle substitué ;
-O-alkyle;
-O-(alkyl)₀₋₁-aryle ;
-O-(alkyl)₀₋₁-aryle substitué ;
-O-(alkyl)₀₋₁-hétéroaryle ;
-O-(alkyl)₀₋₁-hétéroaryle substitué ;
-O-(alkyl)₀₋₁-hétérocyclyle ;
-O-(alkyl)₀₋₁-hétérocyclyle substitué ;
-COOH ;
-CO-O-alkyle ;
-CO-alkyle ;
-S(O)₀₋₂-alkyle ;
-S(O)₀₋₂-(alkyl)₀₋₁-aryle ;
-S(O)₀₋₂-(alkyl)₀₋₁-aryle substitué ;
-S(O)₀₋₂-(alkyl)₀₋₁-hétéroaryle ;
-S(O)₀₋₂-(alkyl)₀₋₁-hétéroaryle substitué ;
-S(O)₀₋₂-(alkyl)₀₋₁-hétérocyclyle ;
-S(O)₀₋₂-(alkyl)₀₋₁-hétérocyclyle substitué ;
-(alkyl)₀₋₁-NR₃R₃ ;
-(alkyl)₀₋₁-NR₃-CO-O-alkyle ;
-(alkyl)₀₋₁-NR₃-CO-alkyle ;
-(alkyl)₀₋₁-NR₃-CO-aryle ;
-(alkyl)₀₋₁-NR₃-CO-aryle substitué ;
-(alkyl)₀₋₁-NR₃-CO-hétéroaryle ;
-(alkyl)₀₋₁-NR₃-CO-hétéroaryle substitué ;
-N₃ ;
-halogène ;
-halogénoalkyle ;
-halogénoalcoxy ;
-CO-halogénoalkyle ;
-CO-halogénoalcoxy ;
-NO₂;
-CN ;
-OH ;
-SH ; et dans le cas d'alkyle, d'alcényle ou d'hétérocyclyle, oxo ;
R₂ est choisi dans le groupe constitué de :
-hydrogène ;
-alkyle ;
-alcényle ;
-aryle ;
-aryle substitué ;
-hétéroaryle ;
-hétéroaryle substitué ;
-alkyl-O-alkyle ;
-alkyl-O-alcényle ; et
-alkyle ou -alcényle substitué par un ou plusieurs substituants choisis dans le groupe constitué de :
-OH ;
-halogène ;
-N(R₃)₂ ;
-CO-N(R₃)₂ ;
-CO-C₁₋₁₀ alkyle ;
-CO-O-C₁₋₁₀ alkyle ;
-N₃ ;
-aryle ;
-aryle substitué ;
-hétéroaryle ;
-hétéroaryle substitué ;
-hétérocyclyle ;
-hétérocyclyle substitué ;
-CO-aryle ;
-CO-(aryle substitué) ;
-CO-hétéroaryle ; et
-CO-(hétéroaryle substitué) ;
chaque R₃ est choisi indépendamment dans le groupe constitué d'hydrogène et de C₁₋₁₀ alkyle ;
R₅ est choisi dans le groupe constitué d'hydrogène et de C₁₋₁₀ alkyle, ou R₄ et R₅ peuvent s' associer pour former un cycle hétérocyclique ou hétéro,cyclique substitué à 3 à 7 chaînons ;
n est 0 à 4 et chaque R présent est choisi indépendamment dans le groupe constitué de C₁₋₁₀ alkyle, C₁₋₁₀ alcoxy, halogène et trifluorométhyle,
et dans laquelle les termes « cycloalkyle substitué », « aryle substitué », « hétéroaryle substitué » et « hétérocyclyle substitué » indiquent que les cycles ou systèmes de cycles en question sont encore substitués par- un ou plusieurs substituants choisis indépendamment dans le groupe constitué d'alkyle, d'alcoxy, d'alkylthio, d'hydroxy, d'halogène, d'halogénoalkyle, d'halogénoalkylcarbonyle, d'halogénoalcoxy (par exemple trifluorométhoxy), de nitro, d'alkylcarbonyle, d'alcénylcarbonyle, d'arylcarbonyle, d'hétéroarylcarbonyle, d'aryle, d'arylalkyle, d'hétéroaryle, d'hétéroarylalkyle, d'hétérocyclyle, d'hétérocycloalkyle, de nitrile, d'alcoxycarbonyle, d'alcanoyloxy, d'alcanoylthio, et dans le cas de cycloalkyle et d'hétérocyclyle, oxo,
ou un sel pharmaceutiquement acceptable de celui-ci.

2. Composé selon la revendication 1, dans lequel X est une liaison.

3. Composé selon la revendication 2, dans lequel R₁ est -(CH₂)₂₋₄-NR₃-SO₂-R₄.

4. Composé selon la revendication 2, dans lequel R₄ est choisi dans le groupe constitué d'alkyle, d'aryle et d'hétéroaryle qui peuvent être non substitués ou substitués par un ou plusieurs substituants choisis dans le groupe constitué de :
-alkyle ;
-alcényle ;
-aryle ;
-hétéroaryle ;
-hétérocyclyle ;
-aryle substitué ;
-hétéroaryle substitué ;
-hétérocyclyle substitué ;
-O-alkyle ;
-O-(alkyl)₀₋₁-aryle ;
-O-(alkyl)₀₋₁-aryle substitué ;
-O-(alkyl)₀₋₁-hétéroaryle ;
-O-(alkyl)₀₋₁-hétéroaryle substitué ;
-O-(alkyl)₀₋₁-hétérocyclyle ;
-O-(alkyl)₀₋₁-hétérocyclyle substitué ;
-COOH ;
-CO-O-alkyle ;
-CO-alkyle ;
-S(O)₀₋₂-alkyle ;
-S(0)₀₋₂-(alkyl)₀₋₁-aryle ;
-S(O)₀₋₂-(alkyl)₀₋₁-aryle substitué ;
-S(O)₀₋₂-(alkyl)₀₋₁-hétéroaryle
-S(O)₀₋₂-(alkyl)₀₋₁-hétéroaryle substitué ;
-S(O)₀₋₂-(alkyl)₀₋₁-hétérocyclyle ;
-S(O)₀₋₂-(alkyl)₀₋₁-hétérocyclyle substitué ;
-(alkyl)₀₋₁-NR₃R₃ ;
-(alkyl)₀₋₁-NR₃-CO-O-alkyle ;
-(alkyl)₀₋₁-NR₃-CO-alkyle ;
-(alkyl)₀₋₁-NR₃-CO-aryle ;
-(alkyl)₀₋₁-NR₃-CO-aryle substitué ;
-(alkyl)₀₋₁-NR₃-CO-hétéroaryle ;
-(alkyl)₀₋₁-NR₃-CO-hétéroaryle substitué ;
-N₃ ;
-halogène ;
-halogénoalkyle ;
-halogénoalcoxy ;
-CO-halogénoalcoxy ;
-NO₂;
-CN ;
-OH ;
-SH ; et dans le cas d'alkyle, oxo, et dans lequel les termes « aryle substitué », « hétéroaryle substitué » et « hétérocyclyle substitué » sont tels que définis dans la revendication 1.

5. Composé selon la revendication 2, dans lequel les liaisons en pointillé sont absentes.

6. Composé selon la revendication 1, dans lequel X est -NR₅-.

7. Composé selon la revendication 2 ou 6, dans lequel n est 0.

8. Composé selon la revendication 6, dans lequel R₁ est - (CH₂)₂₋₄-NR₃-SO₂-NR₅-R₄.

9. Composé selon la revendication 6, dans lequel R₄ et R₅ se joignent pour former un cycle hétérocyclique ou hétérocyclique substitué à 3 à 7 chaînons, dans lequel le terme « hétérocyclyle substitué » est tel que défini dans la revendication 1.

10. Composé selon la revendication 9, dans lequel R₄ et R₅ se joignent pour former un cycle pyrrolidine, morpholine, thiomorpholine, pipéridine ou pipérazine substitué ou non substitué.

11. Composé selon la revendication 2, 6 ou 10, dans lequel R₂ est choisi dans le groupe constitué d' hydrogène, de C₁₋₄alkyle et de C₁₋₄alkyl-O-C₁₋₄alkyle.

12. Composé selon la revendication 6, dans lequel R₄ et R₅ sont alkyle.

13. Composé selon la revendication 2, 6, 9 ou 12, dans lequel R₂ est choisi dans le groupe constitué d'hydrogène ; d'alkyle ; ; d'alkyl-O-alkyle; de (alkyl)₀-₁aryle, de (alkyl)₀₋₁-(aryle substitué) ; de (alkyl)₀-hétéroaryle ; et de (alkyl)₀₋₁-(hétéroaryle substitué), et dans lequel les termes « aryle substitué » et « hétéroaryle substitué » sont tels que définis dans la revendication 1.

14. Composé selon la revendication 2, 6, 10 ou 12, dans lequel R₃ est hydrogène.

15. Composé choisi dans le groupe constitué de :
N²-[2-(4-amino-2-butyl-1*H-*imidazo[4,5*-c*]quinoléin-1-yl)éthyl]-2-thiophènesulfonamide ;
N¹-[2-(4-amino-2-butyl-1*H-*imidazo[4,5*-c*]quinoléin-1-yl) éthyl]-1-benzènesulfonamide ;
N⁸-[2-(4-amino-2-butyl-1*H-*imidazo[4,5*-c*]quinoléin-1-yl)éthyl]-8-quinoléinesulfonamide ;
N¹-[2-(4-amino-2-butyl-1*H-*imidazo[4,5*-c*]quinoléin-1-yl)éthyl]-5-(diméthylamino)-1-naphtalènesulfonamide ;
N-[4-(4-amino-2-butyl-1*H-*imidazo[4,5*-c*]quinoléin-1-yl)butyl]méthanesulfonamide ;
N¹-(4-(4-amino-2-butyl-1*H-*imidazo[4,5*-c*]quinoléin-1-yl)butyl]-1-benzènesulfonamide ;
N⁸-[4-(4-amino-2-butyl-1*H-*imidazo[4,5*-c*]quinoléin-1-yl)butyl]-8-quinoléinesulfonamide ;
N²-[4-(4-amino-2-butyl-1*H-*imidazo[4,5*-c*]quinoléin-1-yl)butyl]-2-thiophènesulfonamide ;
N²-[4-(4-amino-6,7,8,9-tétrahydro-1*H-*imidazo[4,5-*c*]quinoléin-1-yl)butyl]-2-thiophènesulfonamide ;
N¹-[4-(4-amino-6,7,8,9-tétrahydro-1*H-*imidazo[4,5-*c*]quinoléin-1-yl)butyl]-1-benzènesulfonamide ;
N⁸-[4-(4-amino-6,7,8,9-tétrahydro-1*H-*imidazo[4,5-*c*]quinoléin-1-yl)butyl]-8-quinoléinesulfonamide ;
N¹-[4-(4-amino-6,7,8,9-tétrahydro-1*H-*imidazo[4,5-*c*]quinoléin-1-yl)butyl]-5-(diméthylamino)-1-naphtalènesulfonamide ;
N¹-[4-(4-amino-2-butyl-1*H-*imidazo[4,5*-c*]quinoléin-1-yl)butyl]-4-fluoro-1-benzènesulfonamide ;
N¹-[4-(4-amino-2-butyl-1*H-*imidazo[4,5*-c*]quinoléin-1-yl)butyl]-3-fluoro-1-benzènesulfonamide ;
N-{2-[4-amino-2-(éthoxyméthyl)-1*H-*imidazo[4,5-*c*]quinoléin-1-yl]éthyl}méthanesulfonamide ;
N²-{2-[4-amino-2-(éthoxyméthyl)-1*H-*imidazo[4,5-*c*]quinoléin-1-yl]éthyl}-2-thiophènesulfonamide ;
N¹-{2-[4-amino-2-(éthoxyméthyl)-1*H-*imidazo[4,5-*c*]quinoléin-1-yl]éthyl}-5-(diméthylamino)-1-naphtalènesulfonamide ;
N-{4-[4-amino-2-(2-méthoxyéthyl)-1*H-*imidazo[4,5-*c*]quinoléin-1-yl]butyl}méthanesulfonamide ;
N²-{4-[4-amino-2-(2-méthoxyéthyl)-1*H-*imidazo[4,5-*c*]quinoléin-1-yl]butyl}-2-thiophènesulfonamide ;
N¹-{4-[4-amino-2-(2-méthoxyéthyl)-1*H-*imidazo[4,5-*c*]quinoléin-1-yl]butyl}-5-(diméthylamino)-1-naphtalènesulfonamide ;
N¹-{4-[4-amino-2-(2-méthoxyéthyl)-1*H-*imidazo[4,5-*c*]quinoléin-1-yl]butyl}-4-fluoro-1-benzènesulfonamide ;
N¹-{4-[4-amino-2-(2-méthoxyéthyl)-1*H-*imidazo[4,5-*c*]quinoléin-1-yl]butyl}-3-fluoro-1-benzènesulfonamide ;
N¹-{4-[4-amino-2-(2-méthoxyéthyl)-1*H-*imidazo[4,5-*c*]quinoléin-1-yl]butyl}-1-benzènesulfonamide ;
N⁸-{4-[4-amino-2-(2-méthoxyéthyl)-1*H-*imidazo[4,5-*c*]quinoléin-1-yl)butyl}-8-quinoléinesulfonamide ;
N²-{4-[4-amino-2-(4-méthoxybenzyl)-1*H-*imidazo[4,5-*c*]quinoléin-1-yl]butyl}-2-thiophènesulfonamide ;
N-[4-(4-amino-2-butyl-6,7,8,9-tétrahydro-1*H-*imidazo[4,5*-c*]quinoléin-1-yl)butyl]méthanesulfonamide ;
N²-[4-(4-amino-2-butyl-6,7,8,9-tétrahydro-1*H-*imidazo[4,5*-c*]quinoléin-1-yl)butyl]-2-thiophènesulfonamide ;
N¹-[4-(4-amino-2-butyl-6,7,8,9-tétrahydro-1*H-*imidazo[4,5*-c*]quinoléin-1-yl)butyl]-5-(diméthylamino)-1-naphtalènesulfonamide ;
N¹-(4-[4-amino-2-(2-méthoxyéthyl)-6,7,8,9-tétrahydro-1*H-*imidazo[4,5*-c*]quinoléin-1-yl]butyl}-1-benzènesulfonamide ;
N¹-{4-[4-amino-2-(2-méthoxyéthyl)-6,7,8,9-tétrahydro-1*H-*imidazo[4,5*-c*]quinoléin-1-yl]butyl}-5-(diméthylamino)-1-naphtalènesulfonamide ;
N'-{2-[4-amino-2-(éthoxyméthyl)-1*H-*imidazo[4,5-*c*]quinoléin-1-yl]éthyl}-*N,N*-diméthylsulfamide ;
N'-{4-[4-amino-2-(2-méthoxyéthyl)-1*H-*imidazo[4,5-*c*]quinoléin-1-yl]butyl}-*N,N*-diméthylsulfamide ;
N'-{4-[4-amino-2-(4-méthoxybenzyl)-1*H-*imidazo[4,5-*c*]quinoléin-1-yl]butyl}-*N,N*-diméthylsulfamide ;
N'-[4-(4-amino-2-butyl-6,7,8,9-tétrahydro-1*H-*imidazo[4,5*-c*]quinoléin-1-yl)butyl]-*N,N-*diméthylsulfamide ;
N'-{4-[4-amino-2-(2-méthoxyéthyl)-6,7,8,9-tétrahydro-1*H-*imidazo[4,5*-c*]quinoléin-1-yl]butyl}-*N,N-*diméthylsulfamide ;
N⁴-{4-[4-amino-2-(2-méthoxyéthyl)-1*H-*imidazo[4,5-*c*]quinoléin-1-yl]butyl}-4-thiomorpholinesulfonamide ;
N¹-{4-[4-amino-2-(2-méthoxyéthyl)-1*H-*imidazol[4,5-*c*]quinoléin-1-yl]butyl}-1-pyrrolidinesulfonamide ;
N¹-[4-(4-amino-2-butyl-6,7,8,9-tétrahydro-1*H-*imidazo[4,5*-c*]quinoléin-1-yl)butyl]-4-fluoro-1-benzènesulfonamide ;
N-[4-(4-amino-2-(2-méthoxyéthyl)-6,7,8,9-tétrahydro-1*H-*imidazo[4,5*-c*]quinoléin-1-yl)butyl]méthanesulfonamide ; et
N-{4-[4-amino-2-(2-méthoxyéthyl)-1*H-*imidazo[4,5-*c*]quinoléin-1-yl]butyl}phénylméthanesulfonamide.

16. Composé choisi dans le groupe constitué de :
N¹-[4-(4-amino-2-butyl-1*H-*imidazo[4,5*-c*]quinoléin-1-yl)butyl]-5-(diméthylamino)-1-naphtalènesulfonamide ;
N¹-[4-(4-amino-1*H-*imidazo[4,5*-c*]quinoléin-1-yl)butyl]-5-(diméthylamino)-1-naphtalènesulfonamide ;
N²-[4-(4-amino-2-butyl-1*H-*imidazo[4,5*-c*]quinoléin-1-yl)butyl]-2-thiophènesulfonamide ;
N-[4-(4-amino-2-butyl-1*H-*imidazo[4,5*-c*]quinoléin-1-yl)butyl]phénylméthanesulfonamide ;
N¹-[4-(4-amino-2-butyl-1*H-*imidazo[4,5*-c*]quinoléin-1-yl)butyl]-1-benzènesulfonamide ;
N-[4-(4-amino-2-butyl-1*H-*imidazo[4,5*-c*]quinoléin-1-yl)butyl]méthanesulfonamide ;
N¹-[4-(4-amino-2-butyl-1*H-*imidazo[4,5*-c*]quinoléin-1-yl)butyl]-3-nitro-1-benzènesulfonamide ;
N¹-[4-(4-amino-2-butyl-1*H-*imidazo[4,5*-c*]quinoléin-1-yl)butyl]-3-amino-1-benzènesulfonamide ;
N¹-[4-(4-amino-2-butyl-1*H-*imidazo[4,5*-c*]quinoléin-1-yl)butyl]-4-nitro-1-benzènesulfonamide ;
N¹-[4-(4-amino-2-butyl-1*H-*imidazo[4,5*-c*]quinoléin-1-yl)butyl]-4-amino-1-benzènesulfonamide ;
N⁵-[4-(4-amino-2-butyl-1*H-*imidazo[4,5*-c*]quinoléin-1-yl)butyl]-5-isoquinoléinesulfonamide ;
N-[4-(4-amino-2-(4-méthoxybenzyl)-1*H-*imidazo[4,5-*c*]quinoléin-1-yl)butyl]méthanesulfonamide ;
N¹-[4-(4-amino-1*H-*imidazo[4,5*-c*]quinoléin-1-yl) butyl]-1-butanesulfonamide ;
N¹-{4-[4-amino-2-(2-méthoxyéthyl)-6,7,8,9-tétrahydro-1*H-*imidazo[4,5*-c*]quinoléin-1-yl]butyl}-4-fluoro-1-benzènesulfonamide ;
N¹-[4-(4-amino-2-phényl-1*H-*imidazo[4,5*-c*]quinoléin-1-yl)butyl]-4-fluoro-1-benzènesulfonamide ; et
N-[4-(4-amino-2-phényl-1*H-*imidazo[4,5*-c*]quinoléin-1-yl)butyl]méthanesulfonamide.

17. Composition pharmaceutique comprenant une quantité thérapeutiquement efficace d'un composé selon la revendication 1, 2 ou 6, et un support pharmaceutiquement acceptable.

18. Composé selon la revendication 1, 2 ou 6, destiné à être utilisé pour induire la biosynthèse de cytokines.

19. Composé selon la revendication 1, 2 ou 6, destiné à être utilisé dans le traitement d'une maladie virale.

20. Composé selon la revendication 1, 2 ou 6, destiné à être utilisé dans le traitement d'une maladie néoplastique.

21. Utilisation d'un composé selon la revendication 1, 2 ou 6, pour la fabrication d'un médicament destiné à l'induction de la biosynthèse de cytokines.

22. Utilisation d'un composé selon la revendication 1, 2 ou 6, pour le traitement d'une maladie virale.

23. Utilisation d'un composé selon la revendication 1, 2 ou 6, pour le traitement d'une maladie néoplastique.
